# EUROPEAN PATENT APPLICATION

(11) **EP 4 714 971 A1**
(43) Date of publication of application: **25.03.2026**
(21) Application number: 24803022.3
(22) Date of filing: 08.05.2024
(51) Int. Cl.: C07K 16/28, A61K 47/68, A61P 35/00

(54) **ANTIBODY OR ANTIGEN-BINDING FRAGMENT THEREOF, ANTIBODY-DRUG CONJUGATE, AND USE THEREOF**

(30) Priority: 08.05.2023 CN 202310513958
(71) Applicant: Shanghai Hansoh Biomedical Co., Ltd., Shanghai 201203 (CN); Changzhou Hansoh Pharmaceutical Co., Ltd., Xinbei District Changzhou Jiangsu 213001 (CN)
(72) Inventor: CHEN, Simeng, Shanghai 201203 (CN); WANG, Leilei, Shanghai 201203 (CN); ZHANG, Wei, Shanghai 201203 (CN)
(74) Representative: EP&C
(86) International application number: PCT/CN2024/091754
(87) International publication number: WO 2024/230742

(57) **Abstract**

The present invention relates to an antibody or an antigen-binding fragment thereof, an antibody-drug conjugate thereof, and the use thereof. Specifically, provided in the present invention are an SEZ6 antibody, an antigen-binding fragment thereof, an SEZ6 antibody-drug conjugate, a nucleic acid molecule encoding the antibody, an antigen-binding fragment thereof, a vector and host cell containing the nucleic acid molecule, and a method for preparing an antibody-coupled toxin. Also provided are a pharmaceutical composition containing the antibody, the antigen-binding fragment thereof, or the antibody-coupled toxin, and the related pharmaceutical use.

## Description

### Technical Field

The present invention belongs to the field of antibodies, and in particular relates to antibodies that specifically bind to SEZ6 in mammals, particularly humans, a preparation method therefor, a conjugate thereof, and the use thereof; this drug is formed by linking the anti-SEZ6 antibody to an anti-neoplastic drug 9106 via a linker.

### Background Art

An antibody-drug conjugate (ADC) is designed to selectively deliver a cytotoxin into a tumour cell by conjugating the cytotoxin to an antibody that specifically binds to the surface of the cancer cell, followed by antigen-antibody-mediated internalization. When the toxin accumulates to a certain level, the purpose of killing tumour cells is achieved. As an ADC, it consists of three parts: an antibody, a linker, and a toxin. ADC drugs can effectively enhance the precise tumour targeting of toxins and improve the therapeutic window. Therefore, the selection of targets for ADCs is critical, requiring specific high expression in tumours and no expression in normal tissues.

Small cell lung cancer is a highly malignant tumour, with approximately 250000 new cases diagnosed globally each year, accounting for about 15% of lung cancers, and 70% of patients have metastasized at the time of diagnosis; tumorigenesis is closely associated with smoking and is accompanied by mutations in multiple cancer suppressor genes; and the median survival time of diagnosed patients is only 8 to 12 months, and very few patients survive beyond 5 years. The standard first-line therapy for small cell lung cancer is platinum-based agents plus etoposide. Patients have a high response rate to first-line chemotherapy, but after relapse and drug resistance, there is a lack of new effective treatment regimens. Second-line topotecan therapy has a low response rate and does not significantly improve survival time. The treatment of small cell lung cancer is in urgent need of new breakthroughs and progress (Nature Reviews Clinical Oncology volume 14, pages 549-561 (2017)).

Human SEZ6 (seizure-related protein 6, hereinafter referred to as hSEZ6) is a single-pass transmembrane protein, which is mainly expressed in brain tissue and barely expressed in the periphery. In the fields of neurological diseases, knockout of mouse SEZ6 promotes the formation of short and multiple synapses (Neuron 56, 621-639, November 21, 2007); SEZ6 is a substrate for BACE1, and soluble SEZ6 in cerebrospinal fluid can serve as a marker for Alzheimer (*Molecular Neurodegeneration 2016 11: 67*).

As a tumour-specific marker, SEZ6 is specifically highly expressed in neuroendocrine tumour tissues. Immunohistochemical analysis of clinical specimens shows that SEZ6 is highly expressed in small cell lung cancer, and its expression level is much higher than that in normal tissues. In 174 tumour tissue sections of patients with small cell lung cancer, approximately 70% of the tumours exhibited positive SEZ6 expression (published patent: US 2016287720 A1).

Based on the specific high expression of SEZ6 in tumours described above, AbbVie Pharmaceuticals developed an ADC against SEZ6, ABBV-011, with an indication for small cell lung cancer (published patent: WO 2013126810 A). Calicheamicin is used as the toxin, with a DAR value of 2 and a non-degradable linker. The ADC drug entered phase I clinical trial in 2019 (NCT 03639194).

In the present patent, SEZ6 antibodies with high affinity and high internalization activity are obtained by means of mouse immunization and camel immunization, and are conjugated with toxin compound 169106 (published patent: WO 2020063676 A) to obtain an innovative ADC drug. 169106 is an inhibitor of topoisomerase I (also described herein as 9106). Previous studies have shown that the antibody-coupled toxin has good drug druggability, as well as anti-tumour activity and safety *in vivo.*

### Summary of the Invention

The present disclosure relates to an anti-SEZ6 antibody or an antigen-binding fragment thereof, an ADC thereof, and the pharmaceutical use thereof. More specifically, provided are a monoclonal antibody or an antigen-binding fragment that binds to the amino acid sequence or three-dimensional structure of the ECD region of SEZ6, and an ADC drug in which the monoclonal antibody or the antigen-binding fragment is conjugated to a cytotoxic substance, an exatecan analog.

In some embodiments, the present disclosure provides an anti-SEZ6 antibody or an antigen-binding fragment thereof, comprising a heavy chain variable region (VH) or a heavy chain single domain (VHH), wherein the heavy chain variable region or the heavy chain single domain comprises: (1) heavy chain HCDR1, HCDR2, and HCDR3 as set forth in amino acid sequences of SEQ ID NOs: 37, 38, and 39; or (2) heavy chain HCDR1, HCDR2, and HCDR3 as set forth in amino acid sequences of SEQ ID NOs: 43, 44, and 45; or (3) heavy chain HCDR1, HCDR2, and HCDR3 as set forth in amino acid sequences of SEQ ID NOs: 49, 50, and 51; or (4) heavy chain HCDR1, HCDR2, and HCDR3 as set forth in amino acid sequences of SEQ ID NOs: 65, 66, and 67; or (5) heavy chain HCDR1, HCDR2, and HCDR3 as set forth in amino acid sequences of SEQ ID NOs: 68, 69, and 70.

In some embodiments, the present disclosure provides an anti-SEZ6 antibody or an antigen-binding fragment thereof, comprising a heavy chain variable region (VH) and a light chain variable region (VL), wherein the heavy chain variable region and the light chain variable region comprise: (1) heavy chain HCDR1, HCDR2, and HCDR3 as set forth in the amino acid sequences of SEQ ID NOs: 37, 38, and 39, and light chain LCDR1, LCDR2, and LCDR3 as set forth in the amino acid sequences of SEQ ID NOs: 40, 41, and 42; or (2) heavy chain HCDR1, HCDR2, and HCDR3 as set forth in the amino acid sequences of SEQ ID NOs: 43, 44, and 45, and light chain LCDR1, LCDR2, and LCDR3 as set forth in the amino acid sequences of SEQ ID NOs: 46, 47, and 48; or (3) heavy chain HCDR1, HCDR2, and HCDR3 as set forth in the amino acid sequences of SEQ ID NOs: 49, 50, and 51, and light chain LCDR1, LCDR2, and LCDR3 as set forth in the amino acid sequences of SEQ ID NOs: 52, 53, and 54.

In some embodiments, the present disclosure provides an anti-SEZ6 antibody or an antigen-binding fragment thereof, wherein the heavy chain single domain comprises an amino acid sequence as set forth in SEQ ID NO: 63, or a sequence having at least 85%, 90%, 95%, or 99% homology thereto; or an amino acid sequence as set forth in SEQ ID NO: 64, or a sequence having at least 85%, 90%, 95%, or 99% homology thereto.

In some embodiments, the present disclosure provides an anti-SEZ6 antibody or an antigen-binding fragment thereof, wherein the heavy chain single domain comprises the amino acid sequence as set forth in SEQ ID NO: 63 or the amino acid sequence as set forth in SEQ ID NO: 64.

In some embodiments, the present disclosure provides an anti-SEZ6 antibody or an antigen-binding fragment thereof, wherein the amino acid sequence of the heavy chain single domain is as set forth in SEQ ID NO: 63, or a sequence having at least 85%, 90%, 95%, or 99% homology thereto; or the amino acid sequence of the heavy chain single domain is as set forth in SEQ ID NO: 64, or is a sequence having at least 85%, 90%, 95%, or 99% homology thereto.

In some embodiments, the present disclosure provides an anti-SEZ6 antibody or an antigen-binding fragment thereof, wherein the amino acid sequence of the heavy chain single domain is as set forth in SEQ ID NO: 63 or SEQ ID NO: 64.

In some embodiments, the present disclosure provides an anti-SEZ6 antibody or an antigen-binding fragment thereof, comprising a heavy chain variable region and a light chain variable region, wherein the heavy chain variable region comprises an amino acid sequence as set forth in SEQ ID NO: 29, SEQ ID NO: 31, or SEQ ID NO: 35, or a sequence having at least 85%, 90%, 95%, or 99% homology thereto; the light chain variable region comprises an amino acid sequence as set forth in SEQ ID NO: 30, SEQ ID NO: 32, SEQ ID NO: 34, or SEQ ID NO: 36, or a sequence having at least 85%, 90%, 95%, or 99% homology thereto.

In some embodiments, the present disclosure provides an anti-SEZ6 antibody or an antigen-binding fragment thereof, comprising a heavy chain variable region and a light chain variable region, wherein the heavy chain variable region comprises an amino acid sequence as set forth in SEQ ID NOs: 29, SEQ ID NO: 31, or SEQ ID NO: 35; the light chain variable region comprises an amino acid sequence as set forth in SEQ ID NOs: 30, SEQ ID NO: 32, SEQ ID NO: 34, or SEQ ID NO: 36.

In some embodiments, the present disclosure provides an anti-SEZ6 antibody or an antigen-binding fragment thereof, comprising a heavy chain variable region and a light chain variable region, wherein the amino acid sequence of the heavy chain variable region is selected from SEQ ID NO: 29, SEQ ID NO: 31, or SEQ ID NO: 35, or is a sequence having at least 85%, 90%, 95%, or 99% homology thereto; the amino acid sequence of the light chain variable region is selected from SEQ ID NO: 30, SEQ ID NO: 32, SEQ ID NO: 34, or SEQ ID NO: 36, or is a sequence having at least 85%, 90%, 95%, or 99% homology thereto.

In some embodiments, the present disclosure provides an anti-SEZ6 antibody or an antigen-binding fragment thereof, comprising a heavy chain variable region and a light chain variable region, wherein the amino acid sequence of the heavy chain variable region is selected from SEQ ID NO: 29, SEQ ID NO: 31, or SEQ ID NO: 35; the amino acid sequence of the light chain variable region is selected from SEQ ID NO: 30, SEQ ID NO: 32, SEQ ID NO: 34, or SEQ ID NO: 36.

In some embodiments, the present disclosure provides an anti-SEZ6 antibody or an antigen-binding fragment thereof, comprising a heavy chain variable region and a light chain variable region, wherein (1) the heavy chain variable region comprises an amino acid sequence as set forth in SEQ ID NO: 29, or a sequence having at least 85%, 90%, 95%, or 99% homology thereto, and the light chain variable region comprises an amino acid sequence as set forth in SEQ ID NO: 30, or a sequence having at least 85%, 90%, 95%, or 99% homology thereto; or (2) the heavy chain variable region comprises an amino acid sequence as set forth in SEQ ID NO: 31, or a sequence having at least 85%, 90%, 95%, or 99% homology thereto, and the light chain variable region comprises an amino acid sequence as set forth in SEQ ID NO: 32, or a sequence having at least 85%, 90%, 95%, or 99% homology thereto; or (3) the heavy chain variable region comprises an amino acid sequence as set forth in SEQ ID NO: 31, or a sequence having at least 85%, 90%, 95%, or 99% homology thereto, and the light chain variable region comprises an amino acid sequence as set forth in SEQ ID NO: 34, or a sequence having at least 85%, 90%, 95%, or 99% homology thereto; or (4) the heavy chain variable region comprises an amino acid sequence as set forth in SEQ ID NO: 35, or a sequence having at least 85%, 90%, 95%, or 99% homology thereto, and the light chain variable region comprises an amino acid sequence as set forth in SEQ ID NO: 36, or a sequence having at least 85%, 90%, 95%, or 99% homology thereto.

In some embodiments, the present disclosure provides an anti-SEZ6 antibody or an antigen-binding fragment thereof, comprising a heavy chain variable region and a light chain variable region, wherein (1) the heavy chain variable region comprises the amino acid sequence as set forth in SEQ ID NO: 29, and the light chain variable region comprises the amino acid sequence as set forth in SEQ ID NO: 30; or (2) the heavy chain variable region comprises the amino acid sequence as set forth in SEQ ID NO: 31, and the light chain variable region comprises the amino acid sequence as set forth in SEQ ID NO: 32; or (3) the heavy chain variable region comprises the amino acid sequence as set forth in SEQ ID NO: 31, and the light chain variable region comprises the amino acid sequence as set forth in SEQ ID NO: 34; or (4) the heavy chain variable region comprises the amino acid sequence as set forth in SEQ ID NO: 35, and the light chain variable region comprises the amino acid sequence as set forth in SEQ ID NO: 36.

In some embodiments, the present disclosure provides an anti-SEZ6 antibody or an antigen-binding fragment thereof, comprising a heavy chain variable region and a light chain variable region, wherein the heavy chain variable region and the light chain variable region comprise: (1) the amino acid sequence of the heavy chain variable region is as set forth in SEQ ID NO: 29, or is a sequence having at least 85%, 90%, 95%, or 99% homology thereto, and the amino acid sequence of the light chain variable region is as set forth in SEQ ID NO: 30, or is a sequence having at least 85%, 90%, 95%, or 99% homology thereto; or (2) the amino acid sequence of the heavy chain variable region is as set forth in SEQ ID NO: 31, or is a sequence having at least 85%, 90%, 95%, or 99% homology thereto, and the amino acid sequence of the light chain variable region is as set forth in SEQ ID NO: 32, or is a sequence having at least 85%, 90%, 95%, or 99% homology thereto; or (3) the amino acid sequence of the heavy chain variable region is as set forth in SEQ ID NO: 31, or is a sequence having at least 85%, 90%, 95%, or 99% homology thereto, and the amino acid sequence of the light chain variable region is as set forth in SEQ ID NO: 34, or is a sequence having at least 85%, 90%, 95%, or 99% homology thereto; or (4) the amino acid sequence of the heavy chain variable region is as set forth in SEQ ID NO: 35, or is a sequence having at least 85%, 90%, 95%, or 99% homology thereto, and the amino acid sequence of the light chain variable region is as set forth in SEQ ID NO: 36, or is a sequence having at least 85%, 90%, 95%, or 99% homology thereto.

In some embodiments, the present disclosure provides an anti-SEZ6 antibody or an antigen-binding fragment thereof, comprising a heavy chain variable region and a light chain variable region, wherein the heavy chain variable region and the light chain variable region comprise: (1) the amino acid sequence of the heavy chain variable region is as set forth in SEQ ID NO: 29, and the amino acid sequence of the light chain variable region is as set forth in SEQ ID NO: 30; or (2) the amino acid sequence of the heavy chain variable region is as set forth in SEQ ID NO: 31, and the amino acid sequence of the light chain variable region is as set forth in SEQ ID NO: 32; or (3) the amino acid sequence of the heavy chain variable region is as set forth in SEQ ID NO: 31, and the amino acid sequence of the light chain variable region is as set forth in SEQ ID NO: 34; or (4) the amino acid sequence of the heavy chain variable region is as set forth in SEQ ID NO: 35, and the amino acid sequence of the light chain variable region is as set forth in SEQ ID NO: 36.

In some embodiments, the aforementioned anti-SEZ6 antibody or antigen-binding fragment thereof is selected from Fab, Fab', F(ab')2, a single-chain antibody (scFv), a single-domain antibody (sdAb), a dimerized V region (diabody), a disulfide-stabilized V region (dsFv), and an antigen-binding fragment of a peptide comprising a CDR.

In some embodiments, the aforementioned anti-SEZ6 antibody or antigen-binding fragment thereof comprises an antibody constant region, wherein the heavy chain constant region is derived from human IgG1, IgG2, IgG3, or IgG4, or has at least 95% sequence identity thereto, or is a variant thereof; and/or the light chain constant region is derived from a human antibody κ or λ chain, or has at least 95% sequence identity thereto, or is a variant thereof; preferably, the amino acid sequence of the heavy chain constant region is derived from human IgG1, or has at least 95% sequence identity thereto, or is a variant thereof; and/or the light chain constant region is derived from a human antibody κ chain, or has at least 95% sequence identity thereto, or is a variant thereof; more preferably, the amino acid sequence of the heavy chain constant region is as set forth in SEQ ID NO: 33, and/or the amino acid sequence of the light chain constant region is as set forth in SEQ ID NO: 59.

In some embodiments, the aforementioned anti-SEZ6 antibody or antigen-binding fragment thereof comprises a heavy chain and a light chain, wherein (1) the heavy chain comprises an amino acid sequence as set forth in SEQ ID NO: 55, or a sequence having at least 85%, 90%, 95%, or 99% homology thereto, and the light chain comprises an amino acid sequence as set forth in SEQ ID NO: 56, or a sequence having at least 85%, 90%, 95%, or 99% homology thereto; or (2) the heavy chain comprises an amino acid sequence as set forth in SEQ ID NO: 57, or a sequence having at least 85%, 90%, 95%, or 99% homology thereto, and the light chain comprises an amino acid sequence as set forth in SEQ ID NO: 58, or a sequence having at least 85%, 90%, 95%, or 99% homology thereto; or (3) the heavy chain comprises an amino acid sequence as set forth in SEQ ID NO: 57, or a sequence having at least 85%, 90%, 95%, or 99% homology thereto, and the light chain comprises an amino acid sequence as set forth in SEQ ID NO: 60, or a sequence having at least 85%, 90%, 95%, or 99% homology thereto; or (4) the heavy chain comprises an amino acid sequence as set forth in SEQ ID NO: 61, or a sequence having at least 85%, 90%, 95%, or 99% homology thereto, and the light chain comprises an amino acid sequence as set forth in SEQ ID NO: 62, or a sequence having at least 85%, 90%, 95%, or 99% homology thereto.

In some embodiments, the aforementioned anti-SEZ6 antibody or antigen-binding fragment thereof comprises a heavy chain and a light chain, wherein (1) the heavy chain comprises the amino acid sequence as set forth in SEQ ID NO: 55, and the light chain comprises the amino acid sequence as set forth in SEQ ID NO: 56; or (2) the heavy chain comprises the amino acid sequence as set forth in SEQ ID NO: 57, and the light chain comprises the amino acid sequence as set forth in SEQ ID NO: 58; or (3) the heavy chain comprises the amino acid sequence as set forth in SEQ ID NO: 57, and the light chain comprises the amino acid sequence as set forth in SEQ ID NO: 60; or (4) the heavy chain comprises the amino acid sequence as set forth in SEQ ID NO: 61, and the light chain comprises the amino acid sequence as set forth in SEQ ID NO: 62.

In some embodiments, the aforementioned anti-SEZ6 antibody or antigen-binding fragment thereof comprises a heavy chain and a light chain, wherein the heavy chain and the light chain comprise: (1) a heavy chain sequence as set forth in SEQ ID NO: 55 or a sequence having at least 85%, 90%, 95%, or 99% homology thereto, and a light chain sequence as set forth in SEQ ID NO: 56 or a sequence having at least 85%, 90%, 95%, or 99% homology thereto; or (2) a heavy chain sequence as set forth in SEQ ID NO: 57 or a sequence having at least 85%, 90%, 95%, or 99% homology thereto, and a light chain sequence as set forth in SEQ ID NO: 58 or a sequence having at least 85%, 90%, 95%, or 99% homology thereto; or (3) a heavy chain sequence as set forth in SEQ ID NO: 57 or a sequence having at least 85%, 90%, 95%, or 99% homology thereto, and a light chain sequence as set forth in SEQ ID NO: 60 or a sequence having at least 85%, 90%, 95%, or 99% homology thereto; or (4) a heavy chain sequence as set forth in SEQ ID NO: 61 or a sequence having at least 85%, 90%, 95%, or 99% homology thereto, and a light chain sequence as set forth in SEQ ID NO: 62 or a sequence having at least 85%, 90%, 95%, or 99% homology thereto.

In some embodiments, the aforementioned anti-SEZ6 antibody or antigen-binding fragment thereof comprises a heavy chain and a light chain, wherein the heavy chain and the light chain comprise: (1) the heavy chain sequence as set forth in SEQ ID NO: 55 and the light chain sequence as set forth in SEQ ID NO: 56; or (2) the heavy chain sequence as set forth in SEQ ID NO: 57 and the light chain sequence as set forth in SEQ ID NO: 58; or (3) the heavy chain sequence as set forth in SEQ ID NO: 57 and the light chain sequence as set forth in SEQ ID NO: 60; or (4) the heavy chain sequence as set forth in SEQ ID NO: 61 and the light chain sequence as set forth in SEQ ID NO: 62. Or

In some embodiments, the aforementioned anti-SEZ6 antibody or antigen-binding fragment thereof consists of a heavy chain, wherein (1) the heavy chain comprises an amino acid sequence as set forth in SEQ ID NO: 71, or a sequence having at least 85%, 90%, 95%, or 99% homology thereto; or (2) the heavy chain comprises an amino acid sequence as set forth in SEQ ID NO: 72, or a sequence having at least 85%, 90%, 95%, or 99% homology thereto.

In some embodiments, the aforementioned anti-SEZ6 antibody or antigen-binding fragment thereof consists of a heavy chain, wherein (1) the heavy chain comprises the amino acid sequence as set forth in SEQ ID NO: 71; or (2) the heavy chain comprises the amino acid sequence as set forth in SEQ ID NO: 72.

In some embodiments, the aforementioned anti-SEZ6 antibody or antigen-binding fragment thereof consists of a heavy chain, wherein the heavy chain comprises: (1) a heavy chain sequence as set forth in SEQ ID NO: 71 or a sequence having at least 85%, 90%, 95%, or 99% homology thereto; or (2) a heavy chain sequence as set forth in SEQ ID NO: 72 or a sequence having at least 85%, 90%, 95%, or 99% homology thereto.

In some embodiments, the aforementioned anti-SEZ6 antibody or antigen-binding fragment thereof consists of a heavy chain, wherein the heavy chain comprises: (1) the heavy chain sequence as set forth in SEQ ID NO: 71; or (2) the heavy chain sequence as set forth in SEQ ID NO: 72.

In another aspect, the present disclosure provides an isolated anti-SEZ6 antibody or an antigen-binding fragment thereof that competes with the antibody or the antigen-binding fragment thereof according to any one of the foregoing for binding to SEZ6.

In another aspect, the present disclosure provides an isolated nucleic acid encoding the anti-SEZ6 antibody or the antigen-binding fragment thereof according to any one of the foregoing.

In another aspect, the present disclosure provides a vector comprising the nucleic acid as described above.

In yet another aspect, the present disclosure provides a host cell comprising the vector as described above.

In one aspect, the present disclosure provides a method for preparing an anti-SEZ6 antibody or an antigen-binding fragment thereof, comprising culturing the aforementioned host cell under conditions suitable for expression of the anti-SEZ6 antibody or the antigen-binding fragment thereof.

In yet another aspect, the present disclosure provides an antibody-drug conjugate or a pharmaceutically acceptable salt or solvate thereof, or a tautomer, mesomer, racemate, enantiomer, or diastereomer thereof, or a mixture thereof, comprising the anti-SEZ6 antibody or the antigen-binding fragment thereof according to any one of the foregoing.

In some embodiments, the drug in the aforementioned antibody-drug conjugate or pharmaceutically acceptable salt or solvate thereof, or tautomer, mesomer, racemate, enantiomer, or diastereomer thereof, or mixture thereof, is selected from a cytotoxic agent, a radiolabel, a fluorophore, a chromophore, an imaging agent, an immunomodulator, an angiogenesis inhibitor, a cell proliferation inhibitor, a pro-apoptotic agent, a cytolytic enzyme, and any combination thereof; preferably, the drug is selected from: a DNA damaging agent, a topoisomerase inhibitor, a microtubule inhibitor, a protein degrader, a STING agonist, and any combination thereof.

In some embodiments, the aforementioned antibody-drug conjugate or pharmaceutically acceptable salt or solvate thereof, or tautomer, mesomer, racemate, enantiomer, or diastereomer thereof, or mixture thereof, has a structure as shown in general formula (I):

**Pc-(L-Y-Dr)ₙ** **(I)**

wherein Dr is selected from: camptothecin, auristatin, maytansinoid, pyrrolobenzodiazepine (PBD), calicheamicin, duocarmycin, daunorubicin, doxorubicin, calicheamicin, anthramycin, neomycin, amatoxin, hemiasterlin, eribulin, tubulysin, and analogs or derivatives thereof. Preferably, Dr is selected from: exatecan, MMAE, MMAF, MMAD, SN-38, DM1, DM4, and pyrrolobenzodiazepine (PBD) dimer;
n is 1 to 10, preferably, n is 2 to 8, more preferably, n is 3 to 5 or 4 to 8, n is a decimal or an integer, further preferably, n is 4 to 6, n is a decimal or an integer, further preferably, n is 5 to 6, n is a decimal or an integer, still further preferably, n is 2, 2.5, 3, 3.5, 4, 4.5, 5, 5.5, 6, 6.5, 7, 7.5, or 8;
L and Y are linker units; Pc is the aforementioned anti-SEZ6 antibody or antigen-binding fragment thereof.

In some embodiments, the aforementioned antibody-drug conjugate or pharmaceutically acceptable salt or solvate thereof, or tautomer, mesomer, racemate, enantiomer, or diastereomer thereof, or mixture thereof, has a structure as shown in general formula (Pc-L-Y-Dr), general formula (II), general formula (III), or general formula (IV): 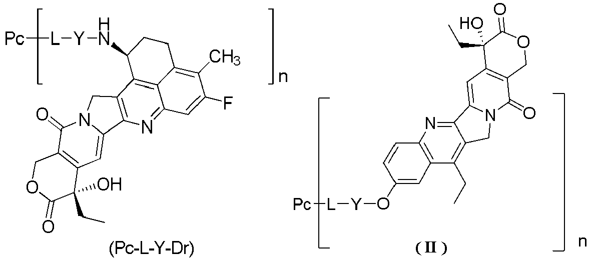 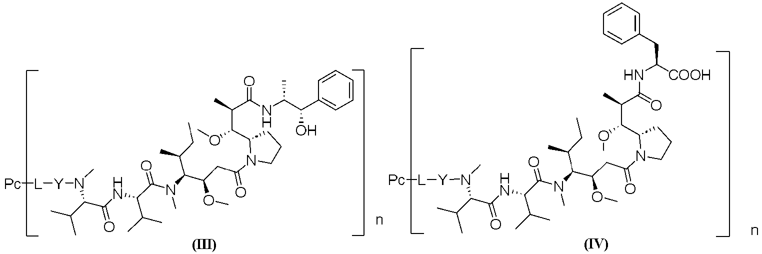 wherein Y is selected from -O-(CR^{a}R^{b})ₘ-CR¹R²-C(O)-, -O-CR¹R²-(CR^{a}R^{b})ₘ-, -O-CR¹R²-, -NH-(CR^{a}R^{b})ₘ-CR¹R²-C(O)-, or -S-(CR^{a}R^{b})ₘ-CR¹R²-C(O)-; R^{a} and R^{b} are identical or different, and are each independently selected from a hydrogen atom, a deuterium atom, halogen, alkyl, haloalkyl, deuterated alkyl, alkoxy, hydroxyl, amino, cyano, nitro, hydroxyalkyl, cycloalkyl, or heterocyclyl; or R^{a} and R^{b} together with the carbon atom to which they are attached form cycloalkyl or heterocyclyl; R¹ is selected from halogen, haloalkyl, deuterated alkyl, cycloalkyl, cycloalkylalkyl, alkoxyalkyl, heterocyclyl, aryl, or heteroaryl; R² is selected from a hydrogen atom, halogen, haloalkyl, deuterated alkyl, cycloalkyl, cycloalkylalkyl, alkoxyalkyl, heterocyclyl, aryl, or heteroaryl; or R¹ and R² together with the carbon atom to which they are attached form cycloalkyl or heterocyclyl; or R^{a} and R² together with the carbon atom to which they are attached form cycloalkyl or heterocyclyl; m is an integer from 0 to 4; n is 1 to 10, preferably, n is 2 to 8, more preferably, n is 3 to 5 or 4 to 8, n is a decimal or an integer, further preferably, n is 4 to 6, n is a decimal or an integer, further preferably, n is 5 to 6, n is a decimal or an integer, still further preferably, n is 2, 2.5, 3, 3.5, 4, 4.5, 5, 5.5, 6, 6.5, 7, 7.5, or 8; L is a linker unit; Pc is the anti-SEZ6 antibody or the antigen-binding fragment thereof according to any one of the foregoing.

In some embodiments, in the aforementioned antibody-drug conjugate or pharmaceutically acceptable salt or solvate thereof, or tautomer, mesomer, racemate, enantiomer, or diastereomer thereof, or mixture thereof, n is 2 to 8, preferably, 3 to 5 or 4 to 8, n is a decimal or an integer, more preferably, 4 to 6, n is a decimal or an integer, further preferably, 5 to 6, n is a decimal or an integer, and still further preferably, n is 2, 2.5, 3, 3.5, 4, 4.5, 5, 5.5, 6, 6.5, 7, 7.5, or 8.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, Y is -O-(CR^{a}R^{b})ₘ-CR¹R²-C(O)-; R^{a} and R^{b} are identical or different, and are each independently selected from a hydrogen atom, a deuterium atom, halogen, or alkyl; R¹ is haloalkyl or C₃₋₆ cycloalkyl; R² is selected from a hydrogen atom, haloalkyl, or C₃₋₆ cycloalkyl; or R¹ and R² together with the carbon atom to which they are attached form C₃₋₆ cycloalkyl; m is 0 or 1.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, Y is selected from: 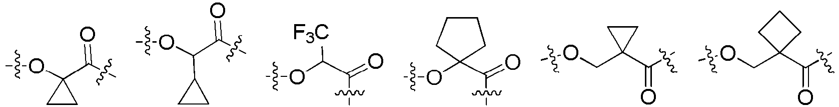 and

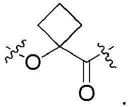

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, the O-terminus of Y is linked to the linker unit L.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, the linker unit -L- is -L¹-L²-L³-L⁴-,
L¹ is 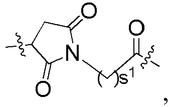 and s¹ is an integer from 2 to 8; L² is a chemical bond; L³ is a tetrapeptide residue; L⁴ is -NR⁵(CR⁶R⁷)t-, R⁵, R⁶, or R⁷ is identical or different, and is each independently a hydrogen atom or alkyl, and t is 1 or 2.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, the linker unit -L- is -L¹-L²-L³-L⁴-,
L¹ is 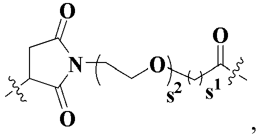 and s¹ and s² are independently integers selected from 0 to 8; preferably, s¹ is 2, and s² is an integer from 2 to 8; more preferably, s¹ is 2, and s² is 2, 3, 4, 5, or 6; L² is a chemical bond; L³ is a tetrapeptide residue; L⁴ is -NR⁵(CR⁶R⁷)t-, R⁵, R⁶, or R⁷ is identical or different, and is each independently a hydrogen atom or alkyl, and t is 1 or 2.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, in the linker unit -L-, the L¹ terminus is linked to Pc, and the L⁴ terminus is linked to Y.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, the tetrapeptide residue of L³ is an amino acid residue formed by two or more amino acids selected from phenylalanine, glycine, valine, lysine, citrulline, serine, glutamic acid, or aspartic acid; preferably, the tetrapeptide residue is GGFG.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, the -L-Y- has the following structure:

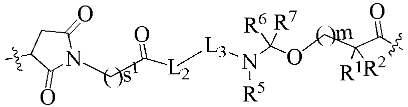

L² is a chemical bond; L³ is a tetrapeptide residue of GGFG; R¹ is haloalkyl or C₃₋₆ cycloalkyl; R² is selected from a hydrogen atom, haloalkyl, or C₃₋₆ cycloalkyl; or R¹ and R² together with the carbon atom to which they are attached form C₃₋₆ cycloalkyl; R⁵, R⁶, or R⁷ is identical or different, and is each independently a hydrogen atom or alkyl; s¹ is an integer from 2 to 8; m is an integer from 0 to 4.

In some embodiments, in the antibody-drug conjugate or the pharmaceutically acceptable salt or solvate thereof, or the tautomer, mesomer, racemate, enantiomer, or diastereomer thereof, or the mixture thereof according to any one of the foregoing, -L-Y- is selected from: 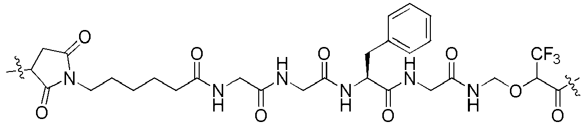 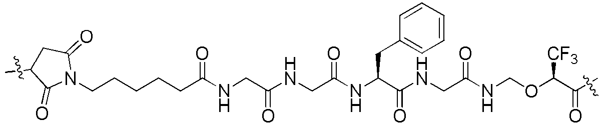 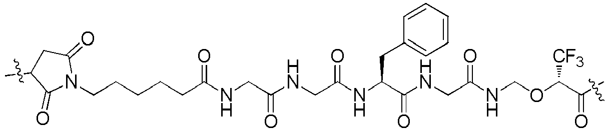 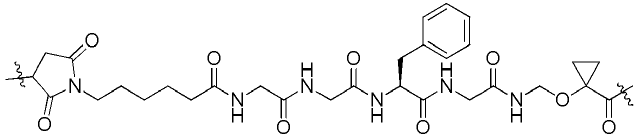 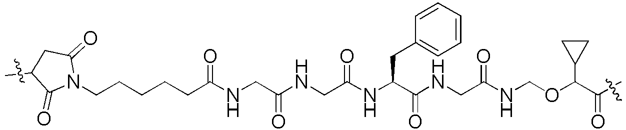 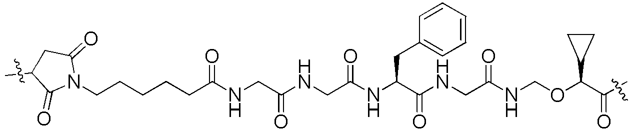 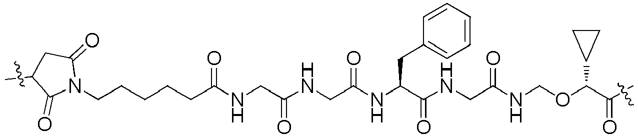 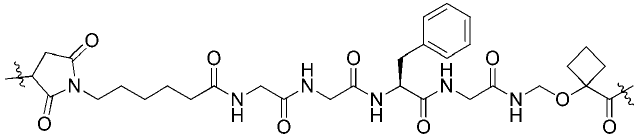 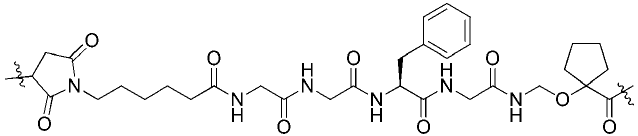 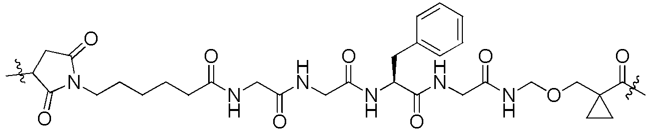 and

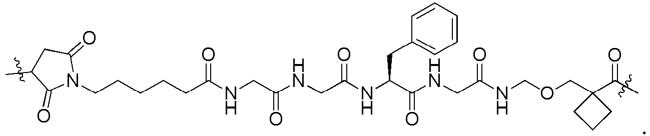

In some embodiments, the antibody-drug conjugate or the pharmaceutically acceptable salt or solvate thereof, or the tautomer, mesomer, racemate, enantiomer, or diastereomer thereof, or the mixture thereof according to any one of the foregoing, is an antibody-drug conjugate as shown in general formula (Pc-Lₐ-Y-Dr) or a pharmaceutically acceptable salt or solvate thereof, or a tautomer, mesomer, racemate, enantiomer, or diastereomer thereof, or a mixture thereof: 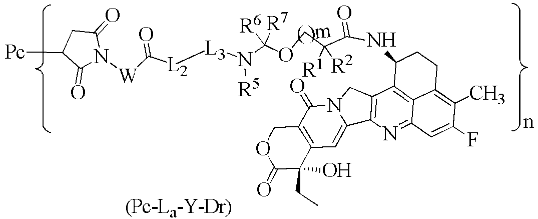 wherein W is selected from C₁₋₈ alkyl, C₁₋₈ alkyl-cycloalkyl, or linear heteroalkyl of 1 to 8 atoms, and the heteroalkyl comprises 1 to 3 heteroatoms selected from N, O, or S, wherein the C₁₋₈ alkyl, cycloalkyl, and linear heteroalkyl are each independently optionally further substituted with one or more substituents selected from halogen, hydroxyl, cyano, amino, alkyl, chloroalkyl, deuterated alkyl, alkoxy, and cycloalkyl; L² is selected from -NR⁴(CH₂CH₂O)ₚ₁CH₂CH₂C(O)-, - NR⁴(CH₂CH₂O)ₚ₁CH₂C(O)-, -S(CH₂)ₚ₁C(O)-, or a chemical bond, and p¹ is an integer from 1 to 20; L³ is a peptide residue consisting of 2 to 7 amino acids, wherein the amino acid can be substituted or unsubstituted, and when the amino acid is substituted, the substitution may occur at any available point of attachment, and the substituent is one or more independently selected from halogen, hydroxyl, cyano, amino, alkyl, chloroalkyl, deuterated alkyl, alkoxy, and cycloalkyl; R¹ is selected from halogen, haloalkyl, deuterated alkyl, cycloalkyl, heterocyclyl, aryl, or heteroaryl; R² is selected from a hydrogen atom, halogen, haloalkyl, deuterated alkyl, cycloalkyl, heterocyclyl, aryl, or heteroaryl; or R¹ and R² together with the carbon atom to which they are attached form cycloalkyl or heterocyclyl; R⁴ and R⁵ are identical or different, and are each independently selected from a hydrogen atom, alkyl, haloalkyl, deuterated alkyl, and hydroxyalkyl; R⁶ and R⁷ are identical or different, and are each independently selected from a hydrogen atom, halogen, alkyl, haloalkyl, deuterated alkyl, and hydroxyalkyl; m is an integer from 0 to 4; n is 1 to 10, preferably, n is 2 to 8, more preferably, n is 3 to 5 or 4 to 8, n is a decimal or an integer, further preferably, n is 4 to 6, n is a decimal or an integer, further preferably, n is 5 to 6, n is a decimal or an integer, still further preferably, n is 2, 2.5, 3, 3.5, 4, 4.5, 5, 5.5, 6, 6.5, 7, 7.5, or 8; Pc is the anti-SEZ6 antibody or the antigen-binding fragment thereof according to any one of the foregoing.

In some embodiments, the antibody-drug conjugate as shown in the aforementioned general formula (Pc-L-Y-Dr) or the pharmaceutically acceptable salt or solvate thereof, or the tautomer, mesomer, racemate, enantiomer, or diastereomer thereof, or the mixture thereof, is an antibody-drug conjugate as shown in general formula (Pc-L_{b}-Y-Dr) or a pharmaceutically acceptable salt or solvate thereof, or a tautomer, mesomer, racemate, enantiomer, or diastereomer thereof, or a mixture thereof: 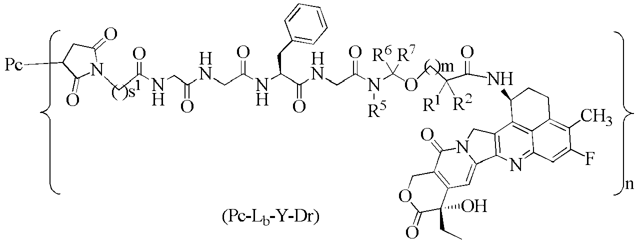 wherein s¹ is an integer from 2 to 8; R¹, R², R⁵-R⁷, m, and n are as defined in general formula (Pc-La-Y-Dr).

In some embodiments, in the aforementioned antibody-drug conjugate or pharmaceutically acceptable salt or solvate thereof, or tautomer, mesomer, racemate, enantiomer, or diastereomer thereof, or mixture thereof, the antibody-drug conjugate is selected from: 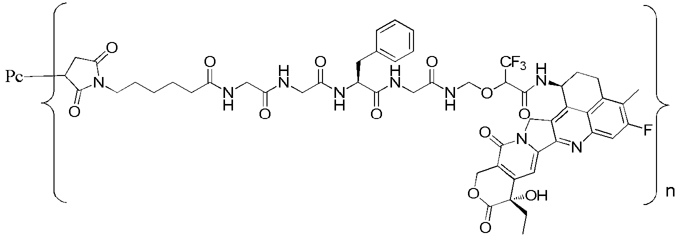 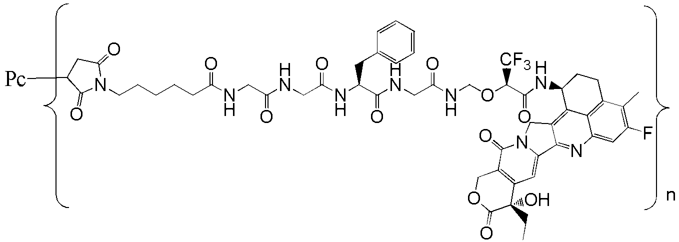 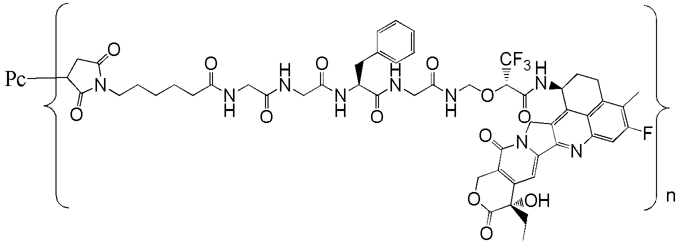 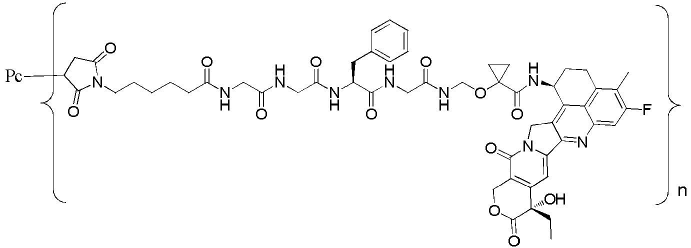 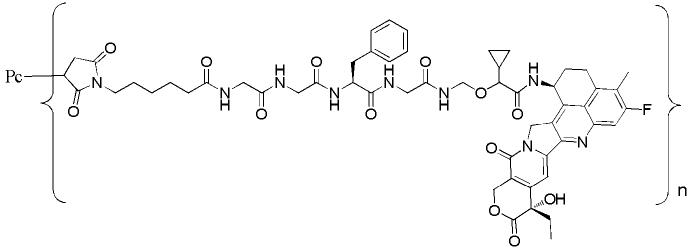 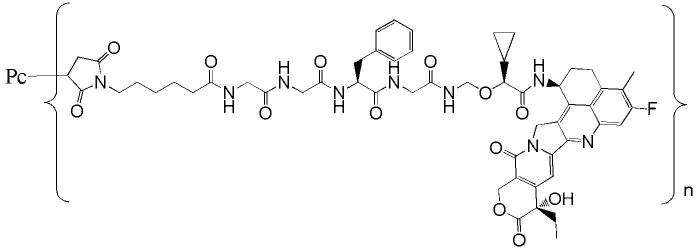 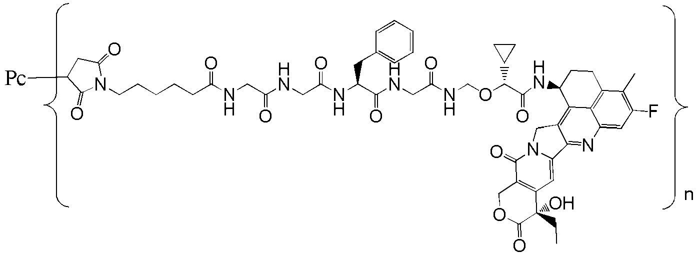 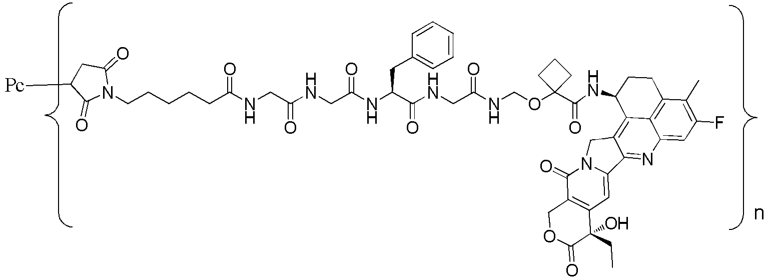 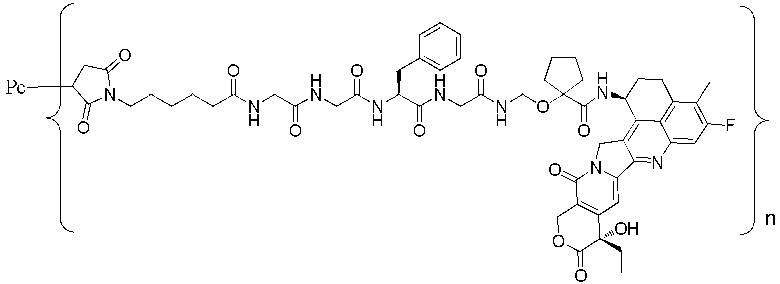 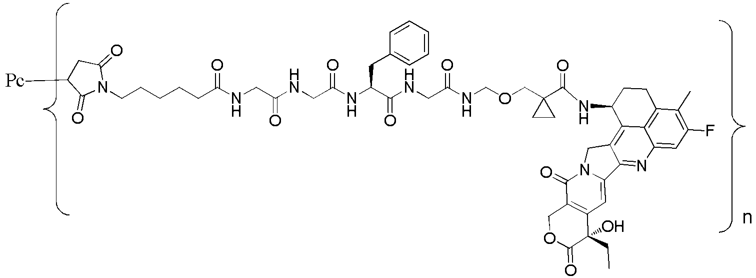 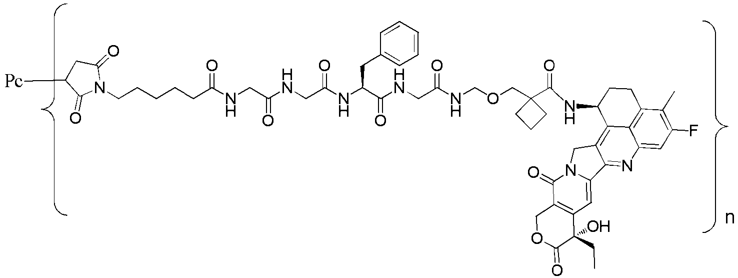 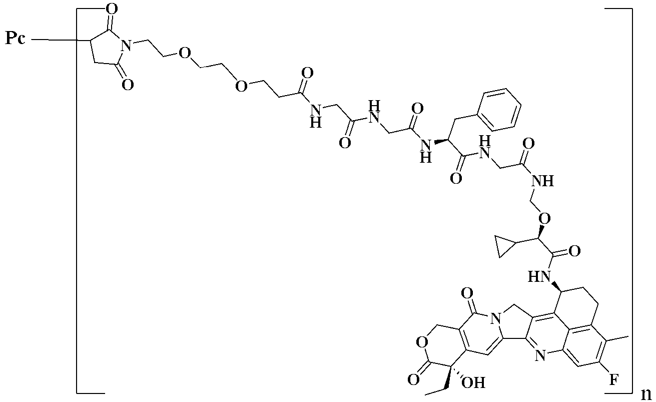 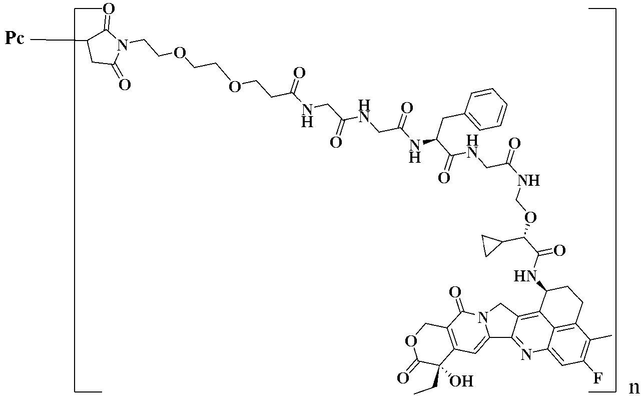 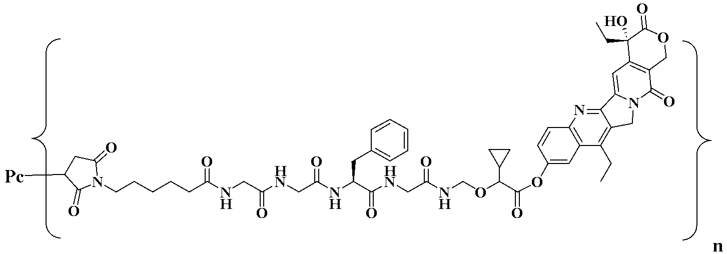 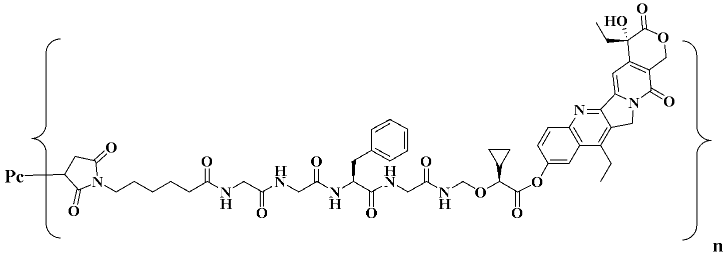 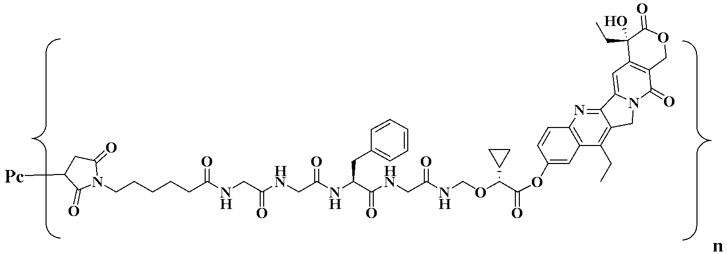 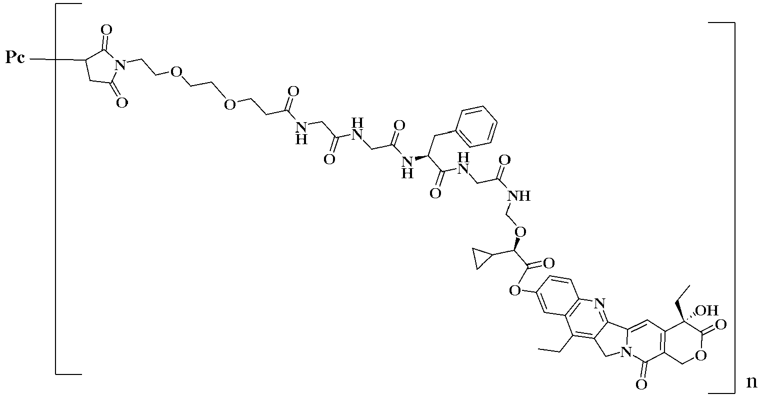 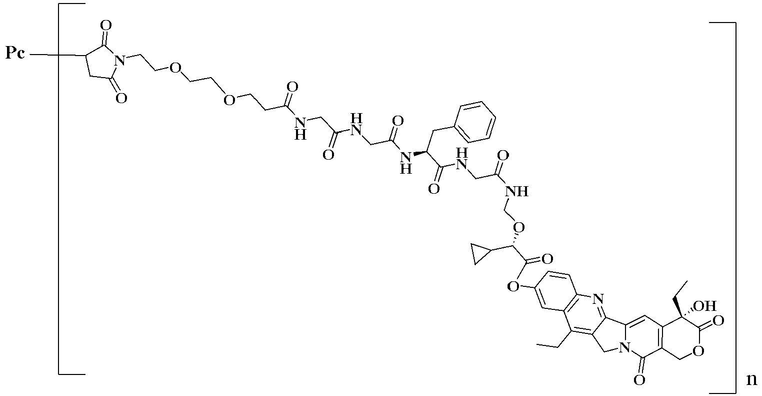 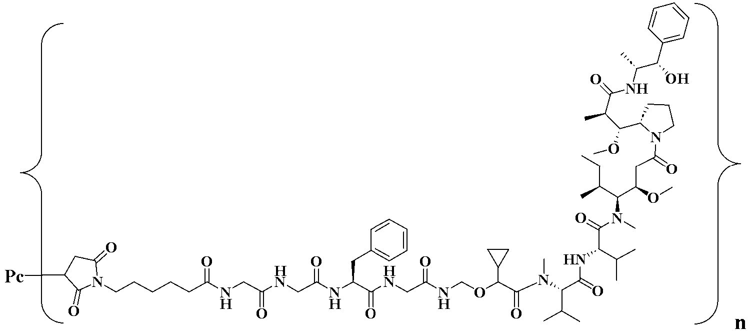 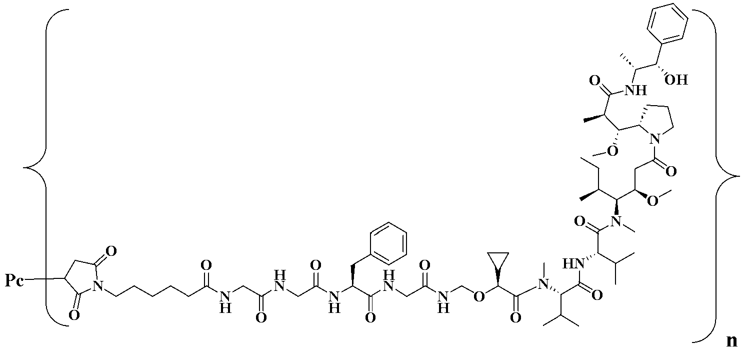 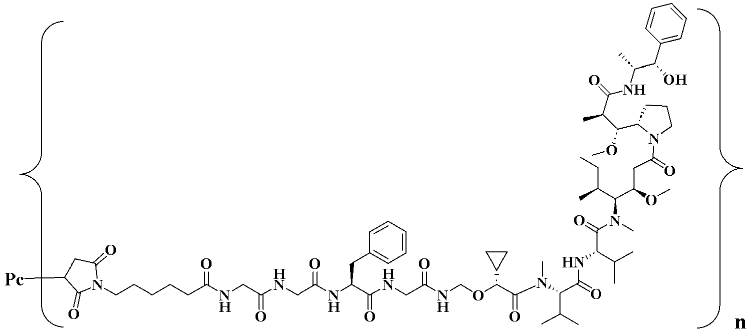 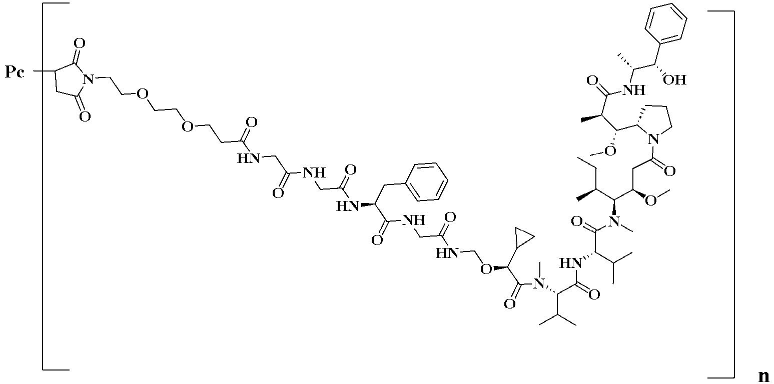 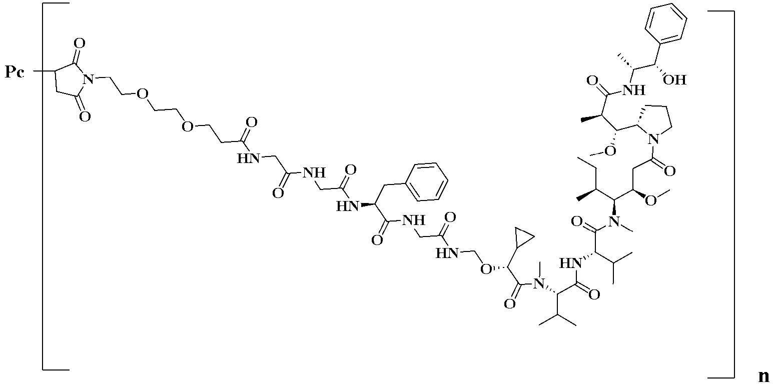 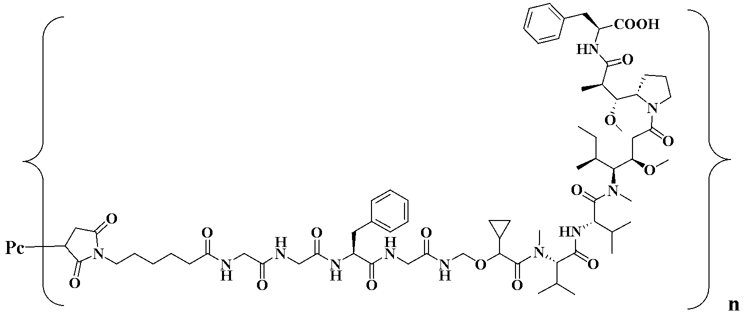 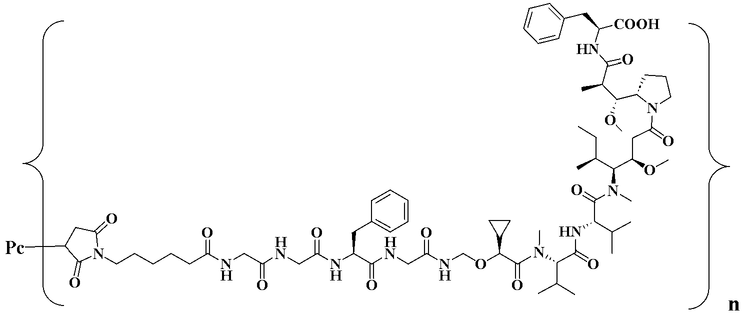 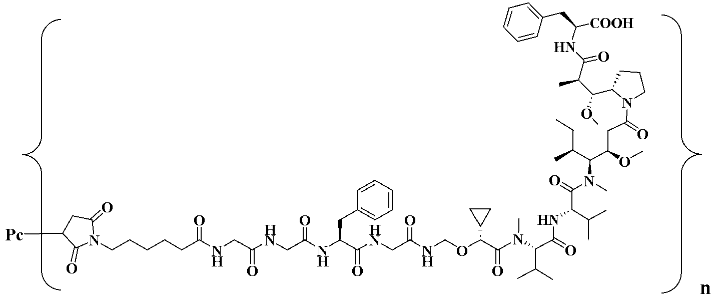 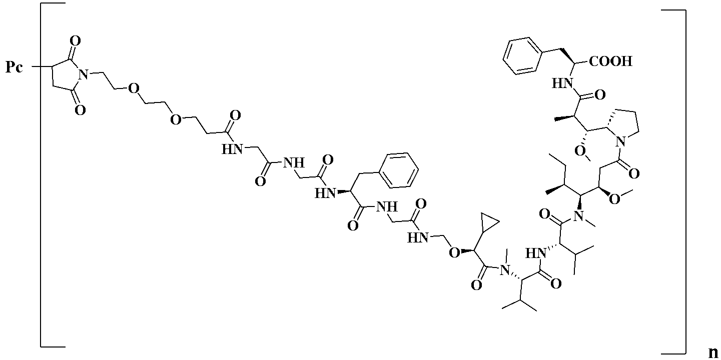 and 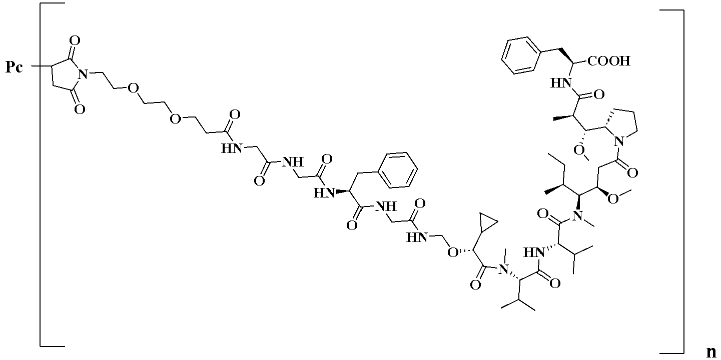
n is 1 to 10, preferably, n is 2 to 8, more preferably, n is 3 to 5 or 4 to 8, n is a decimal or an integer, further preferably, n is 4 to 6, n is a decimal or an integer, further preferably, n is 5 to 6, n is a decimal or an integer, still further preferably, n is 2, 2.5, 3, 3.5, 4, 4.5, 5, 5.5, 6, 6.5, 7, 7.5, or 8;
Pc is the aforementioned anti-SEZ6 antibody or antigen-binding fragment thereof.

In some embodiments, in the aforementioned antibody-drug conjugate or pharmaceutically acceptable salt or solvate thereof, or tautomer, mesomer, racemate, enantiomer, or diastereomer thereof, or mixture thereof, the antibody-drug conjugate is selected from: 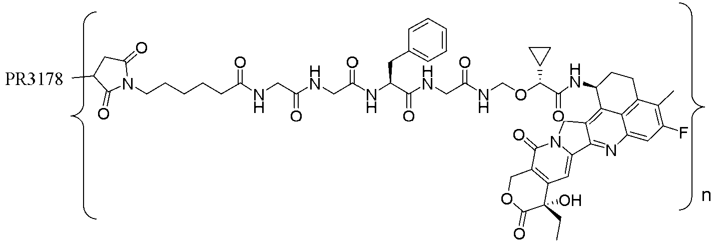 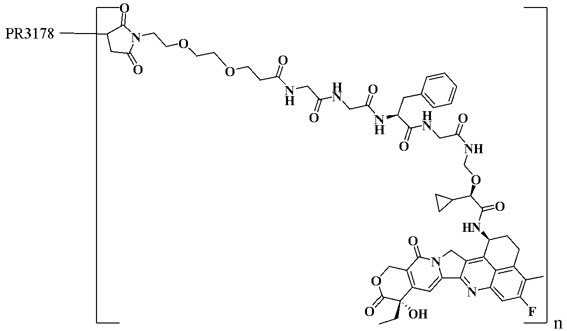 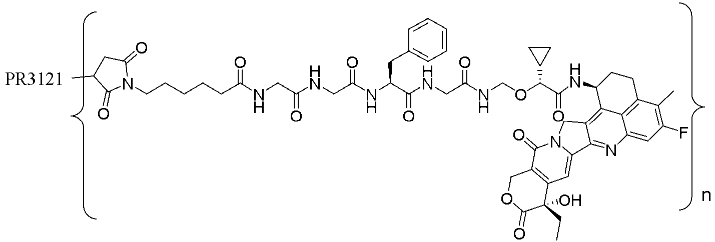 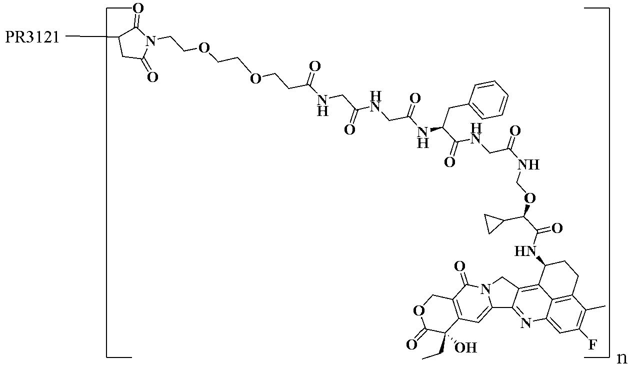 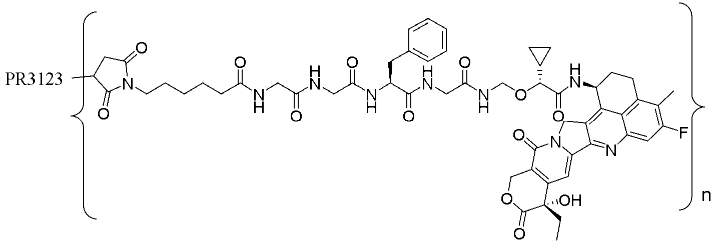 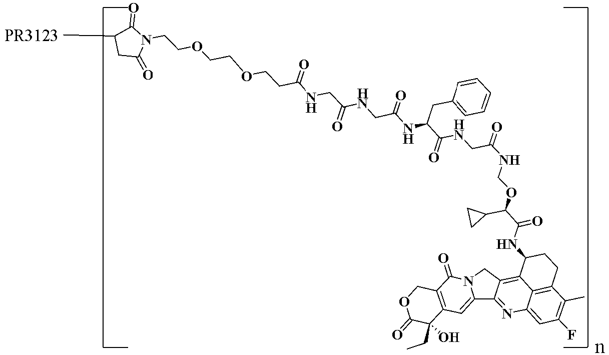 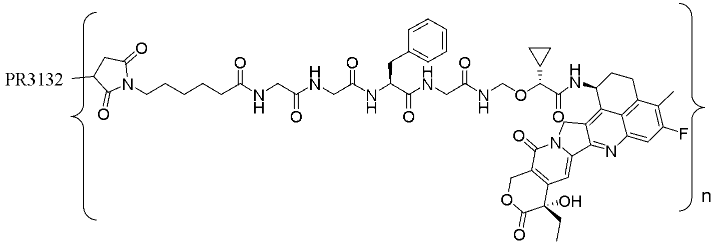 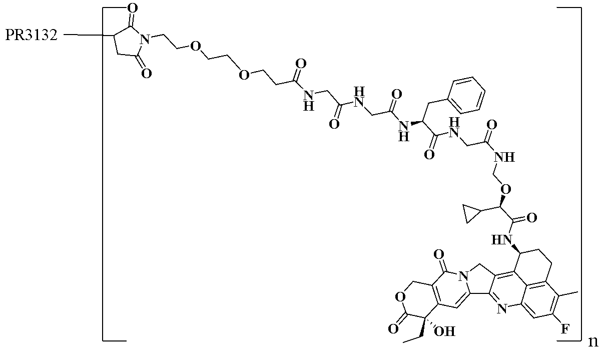 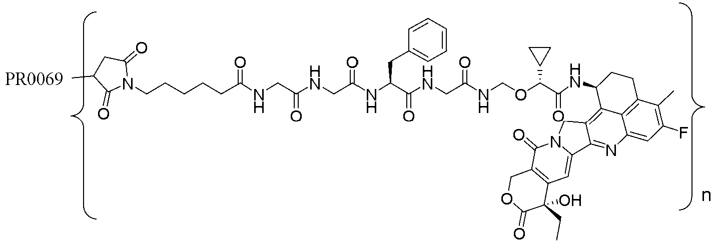 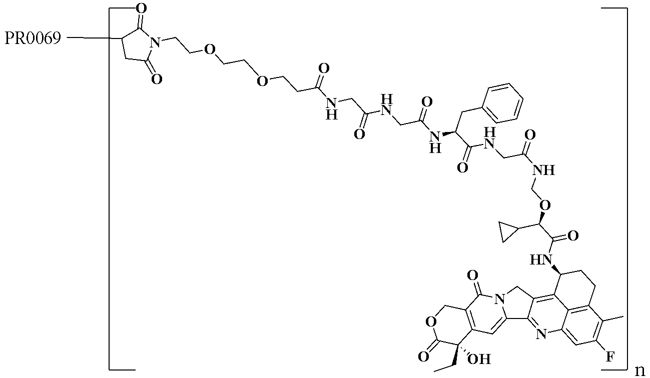 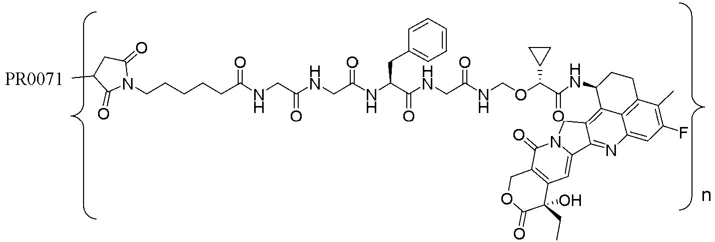 and 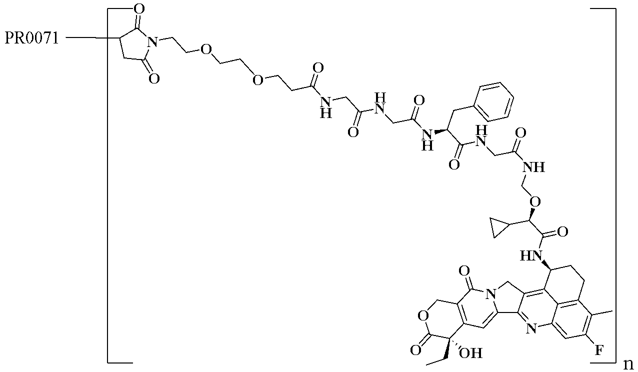 wherein n is 1 to 10, preferably, n is 2 to 8, more preferably, n is 3 to 5 or 4 to 8, n is a decimal or an integer, further preferably, n is 4 to 6, n is a decimal or an integer, further preferably, n is 5 to 6, n is a decimal or an integer, still further preferably, n is 2, 2.5, 3, 3.5, 4, 4.5, 5, 5.5, 6, 6.5, 7, 7.5, or 8;

The present disclosure further provides a method for preparing an antibody-drug conjugate as shown in general formula (Pc-Lₐ-Y-Dr), or a pharmaceutically acceptable salt or solvate thereof, comprising the following steps: 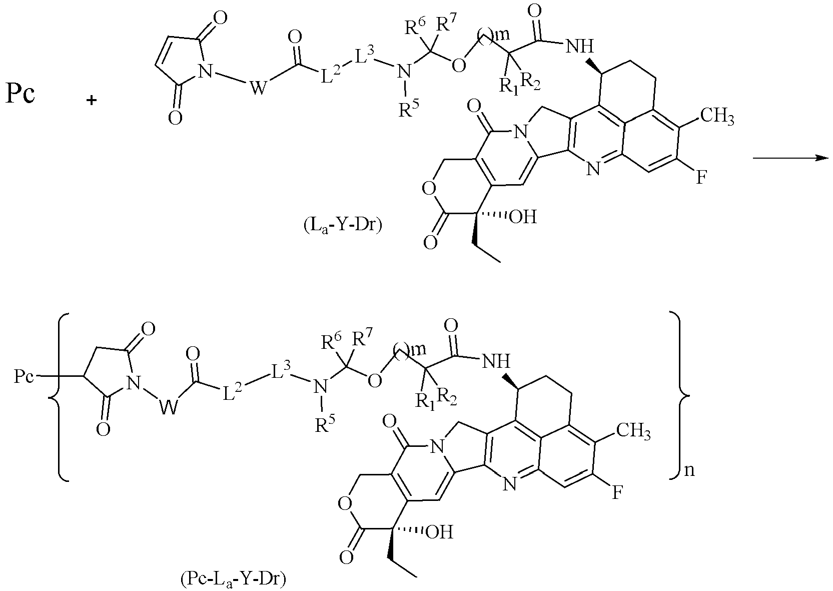 after reduction of Pc, performing a coupling reaction with general formula (Lₐ-Y-Dr) to obtain a compound as shown in general formula (Pc-Lₐ-Y-Dr); wherein Pc is the anti-SEZ6 antibody or the antigen-binding fragment thereof according to any one of the foregoing. W, L², L³, R¹, R², R⁵-R⁷, m, and n are as defined in general formula (Pc-Lₐ-Y-Dr).

In another embodiment, another method is provided, wherein general formula Lₐ-Y-Dr is a compound as shown in general formula L_{b}-Y-Dr, 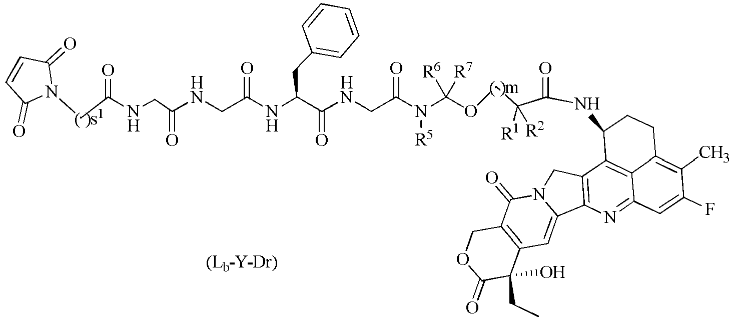 or a tautomer, mesomer, racemate, enantiomer, or diastereomer thereof, or a mixture thereof, or a pharmaceutically acceptable salt thereof, wherein R¹, R², R⁵-R⁷, s¹, and m are as defined in general formula Pc-L_{b}-Y-Dr.

In a preferred embodiment of the present disclosure, in the method for the antibody-drug conjugate as shown in general formula (Pc-Lₐ-Y-Dr) or general formula (Pc-L_{b}-Y-Dr) or pharmaceutically acceptable salt or solvate thereof according to the present disclosure, the compound as shown in general formula (Lₐ-Y-Dr) or general formula (L_{b}-Y-Dr) is selected from: 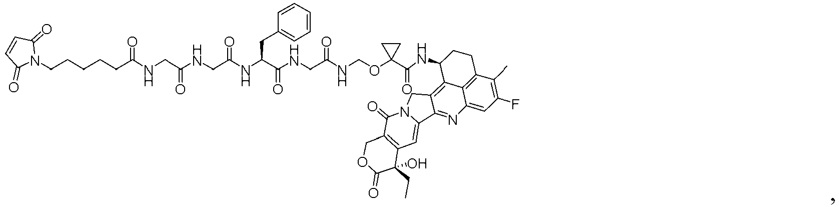 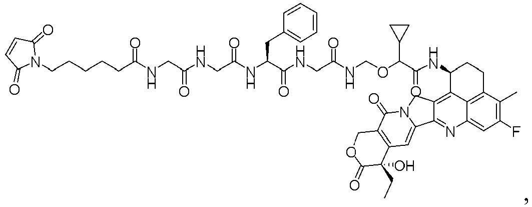 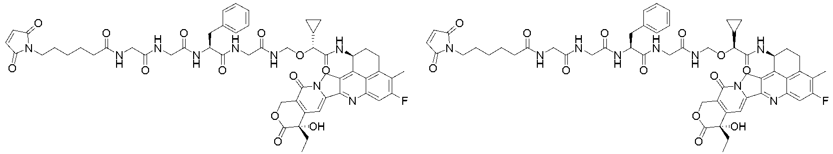 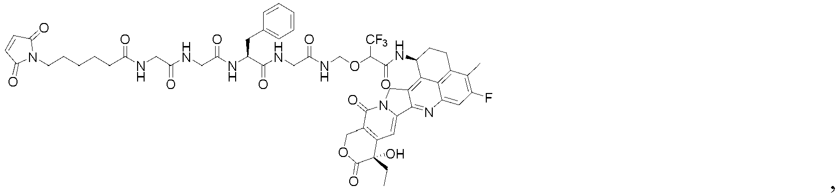 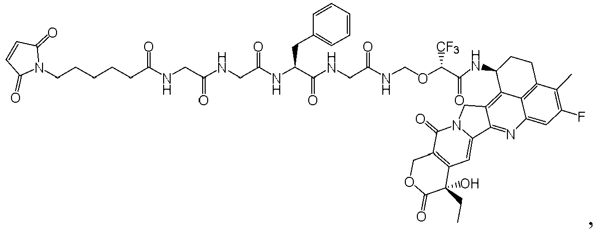 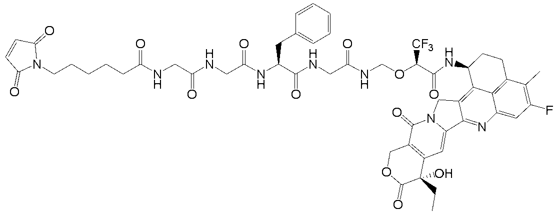 and

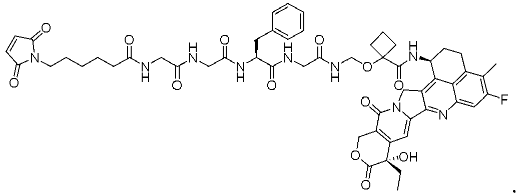

In another aspect, the present disclosure provides a kit comprising the anti-SEZ6 antibody or the antigen-binding fragment thereof according to the present disclosure, or the antibody-drug conjugate or the pharmaceutically acceptable salt or solvate thereof, or the tautomer, mesomer, racemate, enantiomer, or diastereomer thereof, or the mixture thereof according to the present disclosure.

In another aspect, the present disclosure provides a pharmaceutical composition comprising the anti-SEZ6 antibody or the antigen-binding fragment thereof according to the present disclosure, or the antibody-drug conjugate or the pharmaceutically acceptable salt or solvate thereof, or the tautomer, mesomer, racemate, enantiomer, or diastereomer thereof, or the mixture thereof according to the present disclosure, and one or more pharmaceutically acceptable carriers.

In another aspect, the present disclosure provides the use of the anti-SEZ6 antibody or the antigen-binding fragment thereof according to the present disclosure, the antibody-drug conjugate or the pharmaceutically acceptable salt or solvate thereof, or the tautomer, mesomer, racemate, enantiomer, or diastereomer thereof, or the mixture thereof according to the present disclosure, or a pharmaceutical composition comprising same in the manufacture of a medicament for the treatment of a SEZ6-mediated disease or condition, wherein the SEZ6-mediated disease or condition is cancer with high expression of SEZ6.

In another aspect, the present disclosure provides the use of the antibody-drug conjugate or the pharmaceutically acceptable salt or solvate thereof, or the tautomer, mesomer, racemate, enantiomer, or diastereomer thereof, or the mixture thereof according to the present disclosure, or a pharmaceutical composition comprising same in the manufacture of a medicament for the treatment or prevention of a tumour or cancer, wherein the cancer is preferably: lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukaemia, lymphoma, and bone cancer.

The active compound may be formulated into a form suitable for administration by any suitable route, wherein the active compound is preferably in unit dose form, or in a form that allows the patient to self-administer a single dose. The unit dose of the compound or composition of the present disclosure may be expressed as tablets, capsules, cachets, vials, powders, granules, lozenges, suppositories, reconstitutable powders, or liquid preparations.

The dosage of the compound or composition used in the treatment method of the present disclosure will generally vary with the severity of the disease, the body weight of the patient, and the relative efficacy of the compound. However, as a general guide, a suitable unit dose may be 0.1 to 1000 mg.

The pharmaceutical composition of the present disclosure may contain, in addition to the active compound, one or more auxiliary materials selected from the following ingredients: a filler (diluent), a binder, a wetting agent, a disintegrant, or an excipient, etc. Depending on the method of administration, the composition may contain 0.1 to 99 wt% of the active compound.

The anti-SEZ6 antibody or the antigen-binding fragment thereof of the present disclosure has no non-specific binding, exhibits excellent antigen-binding levels, and has a unique binding epitope. The antibody-drug conjugate or the pharmaceutically acceptable salt or solvate thereof, or the tautomer, mesomer, racemate, enantiomer, or diastereomer thereof, or the mixture thereof of the present disclosure has excellent *in vitro* cytotoxicity and *in vivo* tumour suppressive effects, and has excellent *in vivo* pharmacokinetic parameters.

### Detailed Description of the Invention

### I. Terminology

Unless defined otherwise, all technical and scientific terms used herein have the same meanings as commonly understood by those of ordinary skill in the art to which the present disclosure belongs. Although any methods and materials similar or equivalent to those described herein can also be used in the practice or testing of the present disclosure, preferred methods and materials are described herein. In describing and claiming the present disclosure, the following terms are used in accordance with the definitions below.

When a trade name is used in the present disclosure, the applicants intend to include the preparation of the trade name product, and the generic drug and active pharmaceutical ingredient of the trade name product.

Unless stated to the contrary, the terms used in the description and claims have the following meanings.

The term "drug" refers to a cytotoxic drug, denoted as Dr, which is a chemical molecule capable of strongly disrupting normal growth within tumour cells. In principle, cytotoxic drugs can kill tumour cells at a sufficiently high concentration; however, due to a lack of specificity, the cytotoxic drugs can also cause apoptosis of normal cells while killing tumour cells, resulting in serious side effects. The term includes toxins, such as small molecule toxins or enzymatically active toxins of bacterial, fungal, plant or animal origin; radioisotopes (e.g., At²¹¹, I¹³¹, I¹²⁵, Y⁹⁰, Re¹⁸⁶, Re¹⁸⁸, Sm¹⁵³ , Bi²¹², P³², and radioactive isotopes of Lu); toxic drugs; chemotherapeutic drugs; antibiotics; and nucleolysins. Toxic drugs are preferred.

The term "linker unit" (or linker fragment) refers to a chemical structural fragment or bond that is linked to an antibody at one end and to a drug at the other end, and it may also be linked to other linkers before being linked to the drug. Preferred embodiments of the present disclosure are denoted as L and L¹ to L⁴, wherein the L¹ terminus is linked to the antibody, and the L⁴ terminus is linked to the drug (Dr) before being linked to structure unit Y.

The linker, including extenders, spacers, and amino acid units, may be synthesized by methods known in the art, such as those described in US 2005-0238649 A1. The linker may be a "cleavable linker" facilitating the release of the drug in cells. For example, an acid-labile linker (e.g., hydrazone), a protease-sensitive (e.g., peptidase-sensitive) linker, a photolabile linker, a dimethyl linker or a disulfide-containing linker may be used (Chari et al., Cancer Research, 52:127-131 (1992); U.S. Patent No. 5,208,020).

The term "ligand-drug conjugate" means that a ligand is linked to a biologically active drug by a stable linker unit. In the present disclosure, the "ligand-drug conjugate" is preferably an antibody-drug conjugate (ADC), which means that a monoclonal antibody or an antibody fragment is linked to a biologically active toxic drug by a stable linker unit.

The three-letter and single-letter codes for amino acids used in the present disclosure are as described in J. biol. chem, 243, p3558 (1968).

The term "antibody" refers to an immunoglobulin, which is a tetrapeptide chain structure composed of two identical heavy chains and two identical light chains linked by inter-chain disulfide bonds. Since the amino acid composition and arrangement sequence of the heavy chain constant region of an immunoglobulin are different, the antigenicity of the immunoglobulin is also different. Accordingly, immunoglobulin can be divided into five classes, or isotypes of immunoglobulins, namely IgM, IgD, IgG, IgA and IgE, and the corresponding heavy chains thereof are a µ chain, a δ chain, a γ chain, an α chain and an ε chain, respectively. The same class of Ig can also be divided into different subclasses according to the differences in the amino acid composition of the hinge region thereof and the number and position of heavy chain disulfide bonds. For example, IgG can be divided into IgG1, IgG2, IgG3, and IgG4. The light chains are divided into a κ chain or a λ chain by the difference in the constant region. Each Ig class of the five Ig classes can have either a κ chain or a λ chain.

The sequences of about 110 amino acids near the N-terminus of the antibody heavy chain and light chain vary greatly and are the variable regions (Fv regions); the sequences of other amino acids near the C-terminus are relatively stable and are the constant regions. The variable region includes three hypervariable regions (HVR) and four framework regions (FR) with relatively conserved sequences. Three hypervariable regions, also known as complementarity determining regions (CDRs), determine the specificity of the antibody. Each light chain variable region (LCVR) and heavy chain variable region (HCVR) consists of three CDRs and four FRs, which are arranged in the following order from the amino terminus to the carboxyl terminus: FR1, CDR1, FR2, CDR2, FR3, CDR3, FR4. The three CDRs of the light chain refer to LCDR1, LCDR2, and LCDR3; and the three CDRs of the heavy chain refer to HCDR1, HCDR2, and HCDR3. The number and position of the CDR amino acid residues in the LCVR and HCVR of the antibody or the antigen-binding fragment of the present disclosure conform to the known Kabat numbering rules (LCDR1-3, HCDR2-3), or conform to the kabat and chothia numbering rules (HCDR1).

The term "fully human antibody" or "whole human antibody", also known as "fully human monoclonal antibody", refers to an antibody whose variable and constant regions are both of human origin, thereby eliminating immunogenicity and toxic side effects. The development of monoclonal antibodies has undergone four stages: murine monoclonal antibodies, chimeric monoclonal antibodies, humanized monoclonal antibodies, and fully human monoclonal antibodies. Major relevant technologies for the preparation of fully human antibodies include: human hybridoma technology, EBV-transformed B-lymphocyte technology, phage display technology, transgenic mouse technology for antibody preparation, single B-cell antibody preparation technology, etc. The "fully human antibody" in the present disclosure is obtained using phage display technology. Phage display technology involves isolating B cells from human PBMCs, spleen, or lymph node tissue and constructing a native single-chain phage human antibody library, or screening antibodies obtained by immunizing transgenic mice capable of expressing human antibody light and heavy chains.

The term "antigen-binding fragment" refers to one or more fragments of an antibody that retain the ability to specifically bind to an antigen. Fragments of a full-length antibody have been shown to perform the antigen-binding function of the antibody. Examples of binding fragments included within the "antigen-binding fragment" include: (i) an Fab fragment, i.e., a monovalent fragment consisting of VL, VH, CL and CH1 domains; (ii) an F(ab')2 fragment, i.e., a bivalent fragment comprising two Fab fragments linked by a disulfide bridge in the hinge region; (iii) a Fd fragment consisting of VH and CH1 domains; (iv) a Fv fragment consisting of VH and VL domains of a single arm of an antibody; (v) a single domain or dAb fragment (Ward et al., (1989) Nature 341: 544-546) consisting of a VHH domain; and (vi) an isolated complementarity determining region (CDR), or (vii) a combination of two or more isolated CDRs that can be optionally linked by a synthetic linker. Furthermore, although the two domains of the Fv fragment, VL and VH, are encoded by separate genes, they can be linked using a recombinant method by a synthetic linker, thus producing a single protein chain in which the VL and VH regions pair to form a monovalent molecule (referred to as single-chain Fv (scFv); see, e.g., Bird et al. (1988) Science, 242:423-426; and Huston et al. (1988) Proc. Natl. Acad. Sci. USA, 85:5879-5883). Such single-chain antibodies are also intended to be included within the term "antigen-binding fragment" of an antibody. Such antibody fragments are obtained using conventional techniques known to those skilled in the art, and screened for utility in the same manner as for intact antibodies. An antigen-binding moiety may be produced by recombinant DNA technology or by enzymatic or chemical cleavage of an intact immunoglobulin. The antibody can be antibodies of different isotypes, for example, an IgG (e.g., an IgG1, IgG2, IgG3 or IgG4 subtype), IgA1, IgA2, IgD, IgE or IgM antibody.

Fab is an antibody fragment having a molecular weight of about 50,000 and having antigen-binding activity, which is obtained by treating an IgG antibody molecule with a protease papain (cleaving the amino acid residue at position 224 of H chain), wherein about half of the N-terminal side of the H chain and the entire L chain are linked together by a disulfide bond.

F(ab')2 is an antibody fragment having a molecular weight of about 100,000 and having antigen-binding activity and comprising two Fab regions linked at the hinge position, which is obtained by digesting a portion below two disulfide bonds in the hinge region of IgG with the enzyme pepsin.

Fab' is an antibody fragment having a molecular weight of about 50,000 and having antigen-binding activity, which is obtained by cleaving the disulfide bond in the hinge region of the aforementioned F(ab')2.

Furthermore, the Fab' can be produced by inserting DNA encoding the Fab' fragment of an antibody into a prokaryotic expression vector or a eukaryotic expression vector and introducing the vector into a prokaryote or a eukaryote to express the Fab'.

The term "single-chain antibody", "single-chain Fv" or "scFv" refers to a molecule comprising an antibody heavy chain variable domain (or region; VH) and an antibody light chain variable domain (or region; VL) linked by a linker. Such scFv molecules may have the general structure: NH2-VL-linker-VH-COOH or NH2-VH-linker-VL-COOH. Suitable linkers in the prior art consist of repeated GGGGS amino acid sequences or variants thereof, for example, variants using 1-4 repeats (Holliger et al. (1993), Proc. Natl. Acad. Sci. USA 90:6444-6448). Other linkers that can be used in the present disclosure are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol.

The term "single-domain antibody", "single domain antibody", or "sdAb" means a novel type of antibody that does not comprise the CH1 region of the heavy chain and the light chain, but comprises only a single heavy chain variable region, while retaining the complete antigen binding activity characteristics, also known as a nanobody. Like intact antibodies, it can selectively bind to specific antigens. Compared to the mass of 150-160 kDa of intact antibodies, single-domain antibodies appear much smaller, with a mass of about 12-15 kDa. The first single-domain antibody was artificially engineered from the heavy chain antibody of camels, called the "VHH segment".

The terms "single-domain antibody", "heavy chain variable region domain of a heavy chain antibody", "VHH segment", and "nanobody" are used interchangeably. Single-domain antibodies are the variable regions of heavy chain antibodies. Typically, a single-domain antibody contains three CDRs and four FRs.

Single-domain antibodies can be derived from any species, including mouse, human, camel, llama, goat, rabbit, and cattle. For example, naturally occurring VHH molecules can be derived from antibodies provided by camelid species (such as camels, dromedaries, llamas, and Lama guanicoe). Like intact antibodies, single-domain antibodies are capable of selectively binding to specific antigens. A single-domain antibody can contain only the variable domain of an immunoglobulin chain, and the domain has CDR1, CDR2, and CDR3, as well as framework regions.

The term "CDR" refers to one of six hypervariable regions within the variable domain of an antibody which primarily contribute to antigen binding. One of the most common definitions of the six CDRs is provided by Kabat E.A. et al., (1991) Sequences of proteins of immunological interest. NIH Publication, 91-3242). As used herein, the Kabat definition of CDRs applies only to CDR1, CDR2, and CDR3 of the light chain variable domain (CDR L1, CDR L2, and CDR L3 or L1, L2, and L3), and to CDR2 and CDR3 of the heavy chain variable domain (CDR H2 and CDR H3 or H2 and H3).

The term "antibody framework" refers to a portion of a variable domain VL or VH, which serves as a scaffold for the antigen-binding loops (CDRs) of the variable domain. Essentially, it is a variable domain without CDRs.

The term "epitope" or "antigenic determinant" refers to a site on an antigen to which an immunoglobulin or antibody specifically binds. An epitope typically includes at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively binds" and "specifically binds" refer to the binding of an antibody to an epitope on a predetermined antigen. Typically, the antibody binds with an affinity (KD) of about less than 10-7 M, e.g., about less than 10-8 M, 10-9 M, or 10-10 M, or less.

The term "nucleic acid" refers to DNA molecules and RNA molecules. The nucleic acid molecule may be single-stranded or double-stranded, but is preferably double-stranded DNA. When a nucleic acid is placed in a functional relationship with another nucleic acid sequence, it is "operably linked". For example, a promoter or enhancer is operably linked to a coding sequence if it affects the transcription of the coding sequence.

The term "expression vector" refers to a nucleic acid molecule capable of transporting another nucleic acid to which it has been linked. In one embodiment, the vector is a "plasmid", which refers to a circular double-stranded DNA loop into which additional DNA segments may be ligated. In another embodiment, the vector is a viral vector, wherein additional DNA segments may be ligated into the viral genome. The vectors disclosed herein are capable of autonomously replicating in a host cell into which they have been introduced (e.g., bacterial vectors having a bacterial origin of replication and episomal mammalian vectors) or can be integrated into the genome of a host cell upon introduction into the host cell, thereby replicating along with the host genome (e.g., non-episomal mammalian vectors).

Methods for producing and purifying antibodies and antigen-binding fragments are well known in the prior art, for example, those described in Cold Spring Harbor's Antibody Experimentation Technical Guide, Chapters 5-8 and 15. Antigen-binding fragments can also be prepared using conventional methods. In the antibody or the antigen-binding fragment of the present invention, one or more human FRs are added to the CDR of non-human origin by genetic engineering method. Human FR germline sequences can be obtained by alignment using the MOE software and the IMGT human antibody variable region germline gene database (from the website of ImMunoGeneTics(IMGT) at http://imgt.cines.fr), or can be obtained from The Immunoglobulin FactsBook, 2001, ISBN 012441351.

The term "host cell" refers to a cell into which an expression vector has been introduced. Host cells may include bacterial, microbial, plant, or animal cells. Bacteria susceptible to transformation include members of the *Enterobacteriaceae*, such as strains of *Escherichia coli* or *Salmonella*; the *Bacillaceae*, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibodies or antigen-binding fragments of the present disclosure can be prepared and purified using conventional methods. For example, cDNA sequences encoding the heavy and light chains can be cloned and recombined into a GS expression vector. The recombinant immunoglobulin expression vector can be stably transfected into CHO cells. As a more recommended prior art, mammalian expression systems result in glycosylation of antibodies, particularly at the highly conserved N-terminal site of the Fc region. Positive clones are subjected to expanded culture in a serum-free medium in a bioreactor to produce antibodies. The culture medium with the secreted antibodies can be purified using conventional techniques. For example, purification is performed using a Protein A or Protein G Sepharose FF column containing an adjusted buffer. Non-specifically bound fractions are washed away. The bound antibody is then eluted by the PH gradient method, and the antibody fragments are detected by SDS-PAGE and collected. The antibodies can be filtered and concentrated using conventional methods. Soluble mixtures and polymers can also be removed using conventional methods, such as molecular sieves and ion exchange. The resulting products need to be immediately frozen, such as at -70°C, or lyophilized.

The term "peptide" refers to a compound fragment between an amino acid and a protein, formed by the interconnection of two or more amino acid molecules by peptide bonds. It is a structural and functional fragment of the protein, such as hormones and enzymes, which are essentially peptides in nature.

The term "sugar" refers to a biological macromolecule composed of three elements, C, H, and O, and can be classified into monosaccharides, disaccharides, and polysaccharides, among others.

The term "fluorescent probe" refers to a class of fluorescent molecules that have characteristic fluorescence in the ultraviolet-visible-near infrared region and whose fluorescent properties (such as excitation and emission wavelengths, intensity, lifetime, and polarization) can sensitively change in response to alterations in the properties of its surrounding environment, such as polarity, refractive index, and viscosity. These fluorescent molecules non-covalently interact with nucleic acids (DNA or RNA), proteins, or other macromolecular structures to alter one or several fluorescent properties, and therefore can be used to study the properties and behaviours of macromolecular substances.

The term "toxic drug" refers to a substance that inhibits or prevents cell functions and/or causes cell death or destruction, including toxins and other compounds that can be used in the treatment of tumours.

The term "alkyl" refers to a saturated aliphatic hydrocarbon group, which is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms (containing 1, 2, 3, 4, 5, or 6 carbon atoms). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl can be substituted or unsubstituted. When the alkyl is substituted, the substitution may occur at any available point of attachment, and the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo.

The term "heteroalkyl" refers to alkyl containing one or more heteroatoms selected from N, O, or S, wherein the alkyl is as defined above.

The term "alkylene" refers to a saturated linear or branched aliphatic hydrocarbon group having two residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, more preferably alkylene containing 1 to 6 carbon atoms (containing 1, 2, 3, 4, 5 or 6 carbon atoms). Non-limiting examples of alkylene include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylidene (-CH₂CH₂CH₂CH₂-), 1,5-butylidene (-CH₂CH₂CH₂CH₂CH₂-), etc. The alkylene may be substituted or unsubstituted. When the alkylene is substituted, the substitution may occur at any available point of attachment, and the substituent is preferably one or more substituents independently and optionally selected from alkyl, alkenyl, alkynyl, alkoxy, alkylthio, alkylamino, halogen, mercapto, hydroxyl, nitro, cyano, cycloalkyl, heterocyclyl, aryl, heteroaryl, cycloalkoxy, heterocycloalkoxy, cycloalkylthio, heterocycloalkylthio, and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentoxy, and cyclohexoxy. The alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic cyclic hydrocarbon substituent, and the cycloalkyl comprises 3 to 20 carbon atoms, preferably 3 to 12 carbon atoms, more preferably 3 to 10 carbon atoms, and most preferably 3 to 8 carbon atoms (3, 4, 5, 6, 7, or 8 carbon atoms). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and non-limiting examples of polycyclic cycloalkyl include spiro, fused and bridged cycloalkyl.

The term "heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or S(O)ₘ (wherein m is an integer of 0, 1, or 2), but excluding ring moieties of -O-O-, -O-S- or -S-S-, and the remaining ring atoms are carbon. Preferably, the group comprises 3 to 12 ring atoms, of which 1-4 are heteroatoms (1, 2, 3, or 4 heteroatoms); more preferably, the cycloalkyl ring comprises 3 to 10 ring atoms (3, 4, 5, 6, 7, 8, 9, or 10 ring atoms). Non-limiting examples of monocyclic heterocyclyl include pyrrolidyl, piperidyl, piperazinyl, morpholinyl, thiomorpholinyl, homopiperazinyl, etc. Polycyclic heterocyclyl includes spiro, fused and bridged heterocyclyl.

The term "spiroheterocyclyl" refers to 5- to 20-membered polycyclic heterocyclyl with monocyclic rings sharing one atom (called a spiro atom), wherein one or more ring atoms are heteroatoms selected from nitrogen, oxygen, or S(O)ₘ (wherein m is an integer of 0 to 2), and the remaining ring atoms are carbon. The term may contain one or more double bonds, and none of the rings has a fully conjugated π electron system. Preferably, the group is 6- to 14-membered, and more preferably 7- to 10-membered. According to the number of shared spiro atoms between the rings, the spiroheterocyclyl is divided into monospiroheterocyclyl, bispiroheterocyclyl, or polyspiroheterocyclyl, preferably monospiroheterocyclyl and bispiroheterocyclyl. More preferably, it is a 4-membered/4-membered, 4-membered/5-membered, 4-membered/6-membered, 5-membered/5-membered, or 5-membered/6-membered monospiroheterocyclyl. Non-limiting examples of spiroheterocyclyl include:

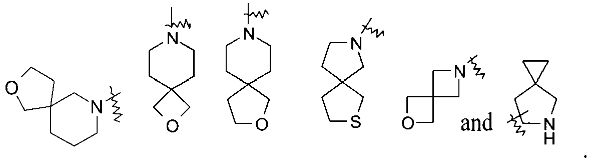

The term "fused heterocyclyl" refers to 5- to 20-membered polycyclic heterocyclyl with each ring in the system sharing a pair of atoms neighbouring other rings in the system, and one or more rings may contain one or more double bonds, and none of the rings has a fully conjugated π electron system, wherein one or more ring atoms are heteroatoms selected from nitrogen, oxygen, or S(O)ₘ (wherein m is an integer of 0, 1, or 2), and the remaining ring atoms are carbon. Preferably, the group is 6- to 14-membered, and more preferably 7- to 10-membered (7-, 8-, 9-, or 10-membered ring). According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include:

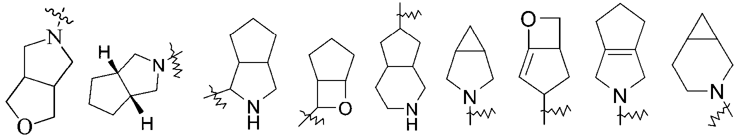

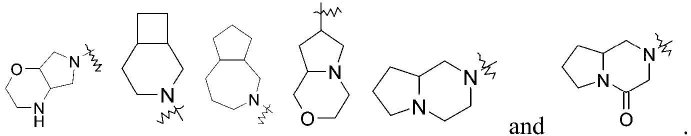

The term "bridged heterocyclyl" refers to a 5- to 14-membered polycyclic heterocyclyl with any two rings sharing two atoms that are not directly connected. It may contain one or more double bonds, and none of the rings has a fully conjugated π electron system, wherein one or more ring atoms are heteroatoms selected from nitrogen, oxygen, or S(O)ₘ (wherein m is an integer of 0, 1, or 2), and the remaining ring atoms are carbon. Preferably, the group is 6- to 14-membered, and more preferably 7- to 10-membered (7-, 8-, 9-, or 10-membered ring). According to the number of constituent rings, it can be divided into bicyclic, tricyclic, tetracyclic or polycyclic bridged heterocyclyl, preferably bicyclic, tricyclic or tetracyclic, and more preferably bicyclic or tricyclic. Non-limiting examples of bridged heterocyclyl include:

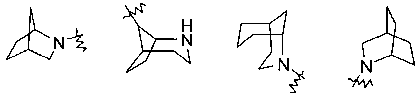

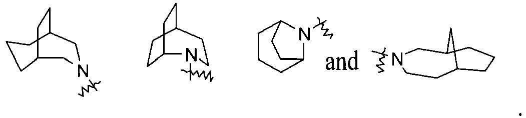

The heterocyclyl ring can be fused to an aryl, heteroaryl or cycloalkyl ring, wherein the ring connected to the parent structure is heterocyclyl, and the non-limiting examples thereof include: 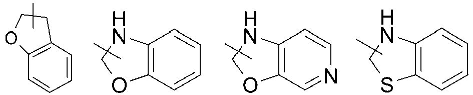 etc.

The heterocyclyl can be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo.

The term "aryl" refers to a 6- to 14-membered all-carbon monocyclic or fused polycyclic (*i.e*., rings sharing adjacent pairs of carbon atoms) group having a conjugated π electron system, preferably 6- to 10-membered (6-membered, 7-membered, 8-membered, 9-membered, or 10-membered), such as phenyl and naphthyl, preferably phenyl. The aryl ring can be fused to a heteroaryl, heterocyclyl or cycloalkyl ring, wherein the ring connected to the parent structure is an aryl ring, and the non-limiting examples thereof include: 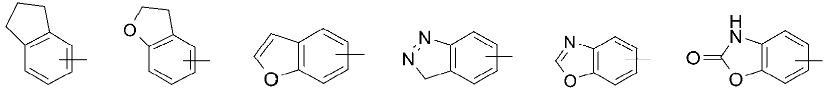 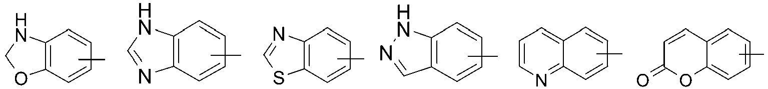 and

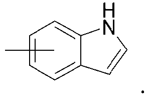

The aryl can be substituted or unsubstituted. When the aryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "heteroaryl" refers to a heteroaromatic system containing 1 to 4 heteroatoms (1, 2, 3, or 4 heteroatoms) and 5 to 14 ring atoms, wherein the heteroatoms are selected from oxygen, sulfur, and nitrogen. The heteroaryl is preferably 5- to 10-membered (5-, 6-, 7-, 8-, 9-, or 10-membered heteroaryl), more preferably 5- or 6-membered, such as furyl, thienyl, pyridyl, pyrrolyl, N-alkylpyrrolyl, pyrimidinyl, pyrazinyl, imidazolyl, tetrazolyl, etc. The heteroaryl ring can be fused to an aryl, heterocyclyl, or cycloalkyl ring, wherein the ring connected to the parent structure is a heteroaryl ring, and the non-limiting examples thereof include:

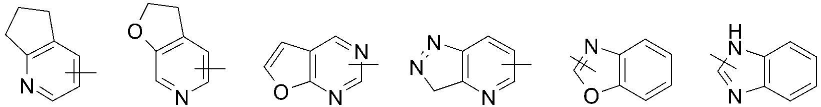

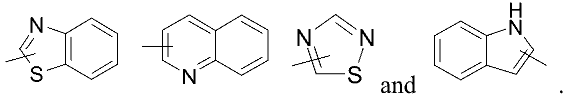

The heteroaryl can be optionally substituted or unsubstituted. When the heteroaryl is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "amino-protecting group" refers to a group that is used to protect an amino group to remain unchanged when other parts of the molecule undergo reactions, wherein the protecting group is easily removable. Non-limiting examples of amino protecting groups include 9-fluorenylmethoxycarbonyl, tert-butoxycarbonyl, acetyl, benzyl, allyl, p-methoxybenzyl, etc. These groups may be optionally substituted with 1-3 substituents (1, 2, or 3 substituents) selected from halogen, alkoxy, or nitro. The amino protecting group is preferably 9-fluorenylmethoxycarbonyl.

The term "haloalkyl" refers to alkyl substituted with one or more halogen, wherein the alkyl is defined as above.

The term "deuterated alkyl" refers to alkyl substituted with one or more deuterium atoms, wherein the alkyl is defined as above.

The term "hydroxyalkyl" refers to alkyl substituted with one or more hydroxyl, where the alkyl is defined as above.

The term "hydroxyl" refers to -OH group.

The term "halogen" refers to fluorine, chlorine, bromine or iodine.

The term "amino" refers to -NH₂.

The term "nitro" refers to -NO₂.

The term "cyano" refers to -CN.

The term "acylamino" refers to -C(O)N(alkyl) or (cycloalkyl), wherein the alkyl and cycloalkyl are as defined above.

The term "carboxylate group" refers to -C(O)O(alkyl) or (cycloalkyl), wherein the alkyl and cycloalkyl are as defined above.

The present disclosure further includes various deuterated forms of the compound of formula (I). Each available hydrogen atom attached to a carbon atom can be independently replaced with a deuterium atom. Those skilled in the art can synthesize the deuterated forms of the compound of formula (I) by referring to relevant literatures. The deuterated forms of the compound of formula (I) can be prepared using commercially available deuterated starting materials, or can be synthesized using deuterated reagents using conventional techniques. The deuterated reagents include, but are not limited to, deuterated borane, solution of trideuterated borane in tetrahydrofuran, deuterated lithium aluminium hydride, deuterated iodoethane, deuterated iodomethane, etc.

"Optional" or "optionally" means that the event or circumstance subsequently described may but not necessarily occur, and the description includes the occasion where the event or circumstance occurs or does not occur. For example, "heterocyclyl optionally substituted with alkyl" means the alkyl may be present but be not necessarily present, and the description includes the case where the heterocyclyl is substituted with alkyl and the case where the heterocyclyl is not substituted with alkyl.

"Substituted" means that one or more hydrogen atoms, preferably at most 5, more preferably 1, 2, or 3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, an amino or hydroxyl group having a free hydrogen may be unstable when bound to a carbon atom having an unsaturated (e.g., ethylenic) bond.

The term "pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

The term "pharmaceutically acceptable salt" refers to a salt of the antibody-drug conjugate of the present disclosure, or a salt of the compound described in the present disclosure. Such salts are safe and effective when used in mammals and possess the required biological activity. The antibody-drug conjugate of the present disclosure comprises at least one amino group and thus may form a salt with an acid. Non-limiting examples of pharmaceutically acceptable salts include: hydrochloride, hydrobromide, hydroiodide, sulphate, hydrogen sulphate, citrate, acetate, succinate, ascorbate, oxalate, nitrate, phosphate, hydrogen phosphate, dihydrogen phosphate, salicylate, hydrogen citrate, tartrate, maleate, fumarate, formate, benzoate, mesylate, ethanesulfonate, benzenesulphonate, and p-toluenesulfonate.

The term "solvate" refers to a pharmaceutically acceptable solvate formed from the antibody-drug conjugate of the present disclosure with one or more solvent molecules. Non-limiting examples of solvent molecules include water, ethanol, acetonitrile, isopropanol, DMSO, ethyl acetate.

The term "drug loading" refers to the average number of cytotoxic drug molecules loaded per antibody in the molecule of formula (I), and may also be represented as the ratio of the amount of drug to the amount of antibody. The drug loading may range from 0 to 12, preferably from 1 to 10, cytotoxic drugs (D) conjugated per antibody (Pc). In embodiments of the present disclosure, the drug loading is denoted as n, and may also be referred to as a DAR value. Exemplary values are averages of 1, 2, 3, 4, 5, 6, 7, 8, 9, and 10. The mean number of drug molecules per ADC molecule after a conjugation reaction can be characterized by conventional methods such as UV/visible spectroscopy, mass spectrometry, ELISA assays, and HPLC.

In one embodiment of the present disclosure, the cytotoxic drug is conjugated to the N-terminal amino and/or the ε-amino of the lysine residue of the antibody by a linker unit. Generally, the number of drug molecules that can be conjugating to the antibody in the conjugation reaction will be less than the theoretical maximum.

The loading of the antibody-cytotoxic drug conjugate can be controlled by the following non-limiting methods, including:
(1) controlling a molar ratio of a linking reagent to a monoclonal antibody,
(2) controlling reaction time and temperature, and
(3) selecting different reaction reagents.

For the preparation of conventional pharmaceutical compositions, reference is made to Chinese Pharmacopoeia.

The term "carrier" used for the drug disclosed herein refers to a system that can alter the manner in which the drug enters the human body and the distribution of the drug within the human body, control the release rate of the drug, and deliver the drug to a target organ. Drug carrier release and targeting systems can reduce drug degradation and loss, reduce side effects, and increase bioavailability. For example, polymeric surfactants that can be used as carriers, due to their unique amphiphilic structures, can self-assemble to form various forms of aggregates. Preferred examples include micelles, microemulsions, gels, liquid crystals, vesicles, etc. These aggregates possess the ability to encapsulate drug molecules while also exhibiting good membrane permeability, and therefore can be used as excellent drug carriers.

The term "excipient" is an additive other than a main drug in a pharmaceutical preparation, and may also be referred to as an auxiliary material. The excipient can include: a binder, a filler, a disintegrating agent, and a lubricant in tablets; a matrix portion of semi-solid preparations such as an ointment and a cream; a preservative, an antioxidant, a flavouring agent, a fragrance, a co-solvent, an emulsifier, a solubilizer, an osmotic pressure regulator, a colorant, etc. in liquid preparations.

The term "diluent", also referred to as a filler, is used primarily to increase the weight and volume of tablets. The addition of a diluent not only ensures a certain volume, but also reduces the dose deviation of the main ingredient, improves the compression moldability of a drug, and the like. When the drug in a tablet contains oily components, an absorbent is necessarily added to absorb the oily components so as to maintain a "dry" state and thus facilitate the preparation of the tablet. Examples include starch, lactose, inorganic salts of calcium, microcrystalline cellulose, and the like.

The pharmaceutical composition may be in the form of a sterile injectable aqueous solution. Available and acceptable vehicles and solvents include water, Ringer's solution, and isotonic sodium chloride solution. The sterile injectable preparation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be injected into the bloodstream of a patient by localized bulk injection. Alternatively, the solution and microemulsion are preferably administered in a manner that maintains a constant circulating concentration of the compound of the present disclosure. To maintain such a constant concentration, a continuous intravenous drug delivery device may be used. An example of such a device is the Deltec CADD-PLUS. TM. 5400 intravenous injection pump.

The pharmaceutical composition may be in the form of a sterile injectable aqueous or oily suspension for intramuscular and subcutaneous administration. The suspension may be formulated according to a known technique using those suitable dispersing agents or wetting agents and suspending agents described above. The sterile injectable preparation may also be a sterile injectable solution or suspension prepared in a non-toxic parenterally acceptable diluent or solvent, e.g., a solution prepared in 1,3-butanediol. In addition, a sterile fixed oil may be conveniently used as a solvent or a suspending medium. For this purpose, any blend fixed oil including synthetic mono- or di-glycerides can be used. In addition, fatty acids such as oleic acid may also be used in the preparation of injections.

The present disclosure relates to a class of cleavable linker arms having a specific structure, active substances having a specific structure, and antibody-drug conjugates (ADCs) consisting of the linker arms, the active substances and an antibody. Such ADCs are complexes formed by linking a toxic substance to an antibody via a spacer. The antibody drug conjugate (ADC) is degraded *in vivo* to release active molecules, thereby playing an anti-tumour role.

### Brief Description of the Drawings

FIG. 1: Activity detection results of recombinant human SEZ6 protein.
FIG. 2A: Detection results of human SEZ6 expression in stably transfected cell line; FIG. 2B: Detection results of monkey SEZ6 expression in stably transfected cell line.
FIG. 3A: Binding activity detection results of murine chimeric antibodies to human SEZ6 on the cell membrane; FIG. 3B: Binding activity detection results of murine chimeric antibodies to monkey SEZ6 on the cell membrane.
FIG. 4: Internalization and immunotoxin killing activity detection results of murine chimeric antibodies.
FIG. 5: Binding activity detection results of humanized antibodies to human cell membrane protein.
FIG. 6A: Internalization and immunotoxin killing activity detection results of humanized antibodies on H69 cells; FIG. 6B: Internalization and immunotoxin killing activity detection results of humanized antibodies on H82 cells.
FIG. 7A: Binding activity detection results of chimeric nanobodies to human SEZ6 on the cell membrane; FIG. 7B: Binding activity detection results of chimeric nanobodies to monkey SEZ6 on the cell membrane.
FIG. 8: Internalization and immunotoxin killing activity detection results of chimeric nanobodies on H69 cells.
FIG. 9: Binding activity detection results of humanized nanobodies to human SEZ6 protein on the cell membrane.
FIG. 10: Internalization and immunotoxin killing activity detection results of humanized nanobodies on H69 cells.
FIG. 11: Binding activity detection results of PR3178 and PR0071 to SEZ6 homologous family proteins.
FIG. 12: SEZ6-affinity epitope identification results of PR3178 and PR0071.
FIG. 13A: *In vitro* inhibitory activity of PR3178-9106 and PR0071-9106 against H69 cells; FIG. 13B: *In vitro* inhibitory activity of PR3178-9106 and PR0071-9106 against H82 cells.
FIG. 14: Anti-tumour activity of PR3178-9106 and PR0071-9106 in H69 xenograft model.
FIG. 15: Single-dose pharmacokinetic evaluation of PR3178-9106 and PR0071-9106 in SCID mice.

### Detailed Description of Embodiments

The following examples are used to further describe the present invention, but these examples do not limit the scope of the present invention.

Experimental methods without specific conditions indicated in the examples or test examples are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Associates, Wiley Interscience, NY. The reagents for which the specific source is not specified, are conventional commercially available reagents.

### Example 1. Detection of activity of recombinant SEZ6 protein

Recombinant human SEZ6-Fc fusion protein was prepared by fusing a human IgG1 Fc tag protein to the C-terminus of the ECD region of human SEZ6 protein (see GenBank database, accession number: NP_ 849191, SEQ ID NO: 1). Recombinant human SEZ6-His6 protein was prepared by fusing a polyhistidine tag His6 to the C-terminus of the extracellular domain (ECD) of human SEZ6 (see GenBank database, accession number: NP_849191). Recombinant monkey SEZ6-His6 protein was prepared by fusing a His tag to the C-terminus of the ECD region of monkey SEZ6 (see GenBank database, accession number: XP_005583333.2, SEQ ID NO: 2). Recombinant mouse SEZ6-His6 protein was prepared by fusing a His tag to the C-terminus of the ECD region of mouse SEZ6 (position 1-922) (see GenBank database, accession number: NP_067261, SEQ ID NO: 3). Extracellular domain sequences are underlined.

**Table 1. Amino acid sequences of proteins used for immunization or screening**

| Protein | Amino acid sequence |
|---|---|
| Human SEZ6 | MRPVALLLLPSLLALLAHGLSLEAPTVGKGQAPGIEETDGELTAAP TPEQPERGVHFVTTAPTLKLLNHHPLLEEFLQEGLEKGDEELRPALP FQPDPPAPFTPSPLPRLANQDSRPVFTSPTPAMAAVPTQPQSKEGPW SPESESPMLRITAPLPPGPSMAVPTLGPGEIASTTPPSRAWTPTQEGP GDMGRPWVAEVVSQGAGIGIQGTITSSTASGDDEETTTTTTIITTTIT TVQTPGPCSWNFSGPEGSLDSPTDLSSPTDVGLDCFFYISVYPGYGV EIKVQNISLREGETVTVEGLGGPDPLPLANQSFLLRGQVIRSPTHQA ALRFQSLPPPAGPGTFHFHYQAYLLSCHFPRRPAYGDVTVTSLHPG GSARFHCATGYQLKGARHLTCLNATQPFWDSKEPVCIAACGGVIR NATTGRIVSPGFPGNYSNNLTCHWLLEAPEGQRLHLHFEKVSLAED DDRLIIRNGDNVEAPPVYDSYEVEYLPIEGLLSSGKHFFVELSTDSS GAAAGMALRYEAFQQGHCYEPFVKYGNFSSSTPTYPVGTTVEFSC DPGYTLEQGSIIIECVDPHDPQWNETEPACRAVCSGEITDSAGVVLS PNWPEPYGRGQDCIWGVHVEEDKRIMLDIRVLRIGPGDVLTFYDG DDLTARVLGQYSGPRSHFKLFTSMADVTIQFQSDPGTSVLGYQQGF VIHFFEVPRNDTCPELPEIPNGWKSPSQPELVHGTVVTYQCYPGYQ VVGSSVLMCQWDLTWSEDLPSCQRVTSCHDPGDVEHSRRLISSPKF PVGATVQYICDQGFVLMGSSILTCHDRQAGSPKWSDRAPKCLLEQ LKPCHGLSAPENGARSPEKQLHPAGATIHFSCAPGYVLKGQASIKC VPGHPSHWSDPPPICRAASLDGFYNSRSLDVAKAPAASSTLDAAHI AAAIFLPLVAMVLLVGGVYFYFSRLQGKSSLQLPRPRPRPYNRITIE SAFDNPTYETGSLSFAGDERI (SEQ ID NO: 1) |
| Monkey SEZ6 | MRPAALLLLPSLLALLAHGLSSEAPTMGKGQAPGIEETDGELTAAP TPEQPERGVHFVTTAPTLKLLNHHPLLEEFLQEGLEKGDEELRPALP FQPDPPTPFTPSPLPRLANQDSRPVFTSPTPATAAVPTQPQSKEGPW SLESEPPVLRITAPLPPGPSMAVPTLRPGERPSTTPPSRAWTPTQEGP GDMGRPWVPEVVSQGAGIGIQGTIASSTASGDDEETTTTTTTIITTT ITTVQTPGPCSWNFSGPEGSLDSPTDLSSPPDVGLDCFFYISVYPGYG VEIKVQNISLREGETVTVEGLGGPDPLPLANQSFLLRGQVIRSPTHQ AALRFQSLPPPAGPGTFHFHYQAYLLSCHFPHRPAYGDVTVTSLHP GGSARFHCATGYQLKGARHLTCLNATQPFWDSKEPVCIAACGGVI RNATTGRIVSPGFPGNYSNNLTCHWLLEAPEGQRLHLHFEKVSLAE DDDRLIIRNGDNVEAPPVYDSYEVEYLPIEGLLSSGKHFFVELSTDS SGAAAGMALRYEAFQQGHCYEPFVKYGNFSSSAPTYPVGTTVEFS CDPGYTLEQGSIIIECVDPHDPQWNETEPACRAVCSGEITDSAGVVL SPNWPEPYGRGQDCIWGVHVEEDKRIMLDVRVLRIGPGDVLTFYD GDDLTARVLGQYSGPHSHFKLFTSMADVTIQFQSDPGTSVLGYQQ GFVIHFFEVPRNDTCPELPEIPNGWKSPSQPDLVHGTVVTYQCYPG YQVVGSSVLMCQWDLTWSEDLPSCQRVTSCHDPGDVEHSRRLISS PKFPVGATVQYICDQGFVLTGTSILTCHDRQAGSPKWSDRAPKCLL EQLKPCHGLSAPENGARSPEKRLHPAGATIHFSCAPGYVLKGQASI KCVPGHPSHWSDPPPICKAASLDGFYNSRSLDVAKAPAASSTLDAA HIAAAIFLPLVAMVLLVGGVYFYFSRLQGKSSLQLPRTRPRPYNRIT VESAFDNPTYETGSLSFAGDERI (SEQ ID NO: 2) |
| Mouse SEZ6 | MRPAALLLLPSLLALLAHGLSSEAPITGEGHATGIRETDGELTAAPT PEQSDRGVHFVTTAPTLKLLNHHPLLEEFLQEGLEREEAPQPALPFQ PDSPTHFTPSPLPRLTNQDNRPVFTSPTPAVAAAPTQPHSREKPWNL ESKPPELSITSSLPPGPSMAVPTLLPEDRPSTTPPSQAWTPTQEGPGD MDRPWVPEVMSKTTGLGVEGTIATSTASGDDEETTTTIITTTVTTV QPPGPCSWNFSGPEGSLDSPTAPSSPSDVGLDCFYYISVYPGYGVEI KVENISLQEGETITVEGLGGPDPLPLANQSFLLRGQVIRSPTHQAAL RFQSLPLPAGPGTFHFRYQAYLLSCHFPRRPAYGDVTVTSLHPGGS AHFHCATGYQLKGARFLTCLNATQPFWDSQEPVCIAACGGVIRNA TTGRIVSPGFPGNYSNNLTCHWLLEAPESQRLHLHFEKVSLAEDDD RLIIRNGNNVEAPPVYDSYEVEYLPIEGLLSSGRHFFVEFSTDSSGAA AGMALRYEAFQQGHCYEPFVKYGNFSSSAPSYPVGTTVEFSCDPG YTLEQGSIIIECVDLHDPQWNETEPACRAVCSGEITDSAGVVLSPNW PEPYGRGQDCIWGVHVEEDKRIMLDIRVLRIGSGDVLTFYDGDDLT ARVLGQYSGPRGHFKLFTSMADVTIQFQSDPGTSALGYQQGFVIHF FEVPRNDTCPELPEIPNGWKNPSQPELVHGTVVTYQCYPGYQVVGS SILMCQWDLSWSEDLPSCQRVTSCHDPGDVEHSRRLISSPKFPVGA TVQYVCDQGFVLTGSAILTCHDRQAGSPKWSDRAPKCLLEQFKPC HGLSAPENGARSPEKRLHPAGATIHFSCAPGYVLKGQASIKCVPGH PSHWSDPPPICRAASLDGFYNGRSLDVAKAPAASSALDAAHLAAAI FLPLVAMVLLVGGVYLYFSRFQGKSPLQLPRTHPRPYNRITVESAF DNPTYETGSLSFAGDERI (SEQ ID NO: 3) |
| Human SEZ6L1 | MPAARPPAAGLRGISLFLALLLGSPAAALERDALPEGDASPLGPYLL PSGAPERGSPGKEHPEERVVTAPPSSSQSAEVLGELVLDGTAPSAHH DIPALSPLLPEEARPKHALPPKKKLPSLKQVNSARKQLRPKATSAAT VQRAGSQPASQGLDLLSSSTEKPGPPGDPDPIVASEEASEVPLWLDR KESAVPTTPAPLQISPFTSQPYVAHTLPQRPEPGEPGPDMAQEAPQE DTSPMALMDKGENELTGSASEESQETTTSTIITTTVITTEQAPALCSV SFSNPEGYIDSSDYPLLPLNNFLECTYNVTVYTGYGVELQVKSVNL SDGELLSIRGVDGPTLTVLANQTLLVEGQVIRSPTNTISVYFRTFQD DGLGTFQLHYQAFMLSCNFPRRPDSGDVTVMDLHSGGVAHFHCH LGYELQGAKMLTCINASKPHWSSQEPICSAPCGGAVHNATIGRVLS PSYPENTNGSQFCIWTIEAPEGQKLHLHFERLLLHDKDRMTVHSGQ TNKSALLYDSLQTESVPFEGLLSEGNTIRIEFTSDQARAASTFNIRFE AFEKGHCYEPYIQNGNFTTSDPTYNIGTIVEFTCDPGHSLEQGPAIIE CINVRDPYWNDTEPLCRAMCGGELSAVAGVVLSPNWPEPYVEGED CIWKIHVGEEKRIFLDIQFLNLSNSDILTIYDGDEVMPHILGQYLGNS GPQKLYSSTPDLTIQFHSDPAGLIFGKGQGFIMNYIEVSRNDSCSDLP EIQNGWKTTSHTELVRGARITYQCDPGYDIVGSDTLTCQWDLSWSS DPPFCEKIMYCTDPGEVDHSTRLISDPVLLVGTTIQYTCNPGFVLEG SSLLTCYSRETGTPIWTSRLPHCVSEESLACDNPGLPENGYQILYKR LYLPGESLTFMCYEGFELMGEVTIRCILGQPSHWNGPLPVCKVNQD SFEHALEVAEAAAETSLEGGNMALAIFIPVLIISLLLGGAYIYITRCR YYSNLRLPLMYSHPYSQITVETEFDNPIYETGETREYEVSI (SEQ ID NO: 4) |
| Human SEZ6L2 | MGTPRAQHPPPPQLLFLILLSCPWIQGLPLKEEEILPEPGSETPTVASE ALAELLHGALLRRGPEMGYLPGSDRDPTLATPPAGQTLAVPSLPRA TEPGTGPLTTAVTPNGVRGAGPTAPELLTPPPGTTAPPPPSPASPGPP LGPEGGEEETTTTIITTTTVTTTVTSPVLCNNNISEGEGYVESPDLGS PVSRTLGLLDCTYSIHVYPGYGIEIQVQTLNLSQEEELLVLAGGGSP GLAPRLLANSSMLGEGQVLRSPTNRLLLHFQSPRVPRGGGFRIHYQ AYLLSCGFPPRPAHGDVSVTDLHPGGTATFHCDSGYQLQGEETLIC LNGTRPSWNGETPSCMASCGGTIHNATLGRIVSPEPGGAVGPNLTC RWVIEAAEGRRLHLHFERVSLDEDNDRLMVRSGGSPLSPVIYDSD MDDVPERGLISDAQSLYVELLSETPANPLLLSLRFEAFEEDRCFAPF LAHGNVTTTDPEYRPGALATFSCLPGYALEPPGPPNAIECVDPTEPH WNDTEPACKAMCGGELSEPAGVVLSPDWPQSYSPGQDCVWGVHV QEEKRILLQVEILNVREGDMLTLFDGDGPSARVLAQLRGPQPRRRL LSSGPDLTLQFQAPPGPPNPGLGQGFVLHFKEVPRNDTCPELPPPEW GWRTASHGDLIRGTVLTYQCEPGYELLGSDILTCQWDLSWSAAPP ACQKIMTCADPGEIANGHRTASDAGFPVGSHVQYRCLPGYSLEGA AMLTCYSRDTGTPKWSDRVPKCALKYEPCLNPGVPENGYQTLYK HHYQAGESLRFFCYEGFELIGEVTITCVPGHPSQWTSQPPLCKVTQT TDPSRQLEGGNLALAILLPLGLVIVLGSGVYIYYTKLQGKSLFGFSG SHSYSPITVESDFSNPLYEAGDTREYEVSI (SEQ ID NO: 5) |

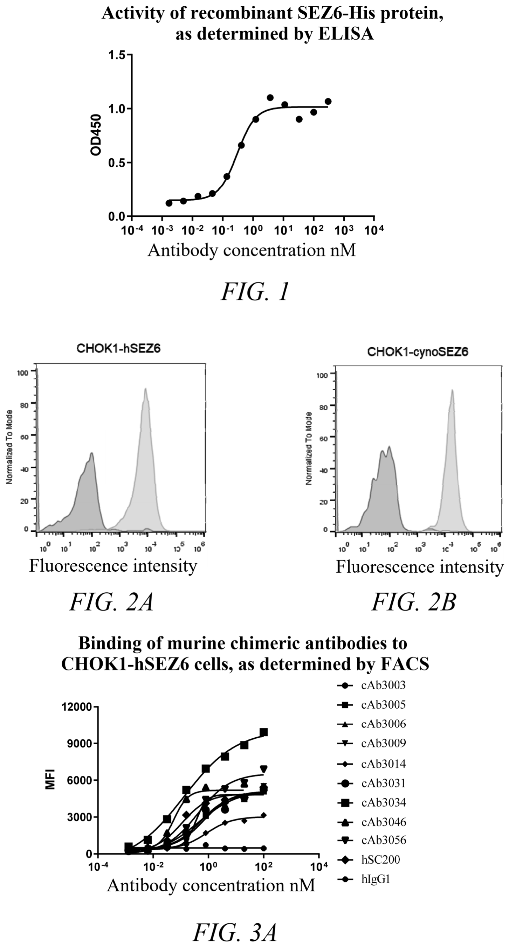

To detect the good immunological activity of the recombinant protein, verification was performed by ELISA (enzyme-linked immunosorbent assay). Specifically: The recombinant human SEZ6-His6 protein was diluted to 1 µg/mL with PBS, added to an ELISA microplate at 100 µL per well, and incubated overnight at 4°C; an ELISA blocking solution (PBS buffer containing 1% BSA (w/v), pH 7.4) was added, the plate was blocked at 37°C for 2 hours, serially diluted anti-SEZ6 detection antibodies hSC200 (hSC17.200, heavy chain SEQ ID NO: 6, and light chain SEQ ID NO: 7) were then sequentially added, and the plates were incubated at 37°C for 1 hour, and washed 2-3 times with a washing solution; and a horseradish peroxidase (HRP)-labelled secondary antibody was added, and the plates were incubated at 37°C for 1 hour, and washed 2-3 times with a washing solution. 100 µL of TMB substrate was added into each well, and incubated for 15 minutes at room temperature, and 50 µL of stop solution (2 M HCl) was then added to each well. OD450nm values were read using an ELISA plate reader (SpectraMax M5e). The results are shown in FIG. 1, indicating that the recombinant human SEZ6-His6 protein can bind to the SEZ6 antibody hSC200 with high affinity and can be used for subsequent *in vivo* protein immunization.

WO 2019232241 (AbbVie Stemcentrx LLC) discloses the hSC200 sequence as follows:

EVQLVQSGAEVKKPGESLKISCKGSGYSFTSSWINWVRQMPGKGLEW
MGRIYPGEGDTNYNGNFEGQVTISADKSISTAYLQWSSLKASDTAMYYCT
RGLVMDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLM
ISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ
ID NO: 6)

EIVLTQSPATLSLSPGERATLSCRASQSVDYNGISYMHWYQQKPGQAP
RLLIYAASNVQSGIPARFSGSGSGTDFTLTISSLEPEDFAVYYCQQSIEDPPTF
GGGTKVEIKRTVAAPSVFIFPPSDEQLKSGTASVVCLLNNFYPREAKVQWK
VDNALQSGNSQESVTEQDSKDSTYSLSSTLTLSKADYEKHKVYACEVTHQ
GLSSPVTKSFNRGEC (SEQ ID NO: 7)

### Example 2. Establishment of stable cell line expressing recombinant human SEZ6

To screen candidate antibody molecules with affinity for membrane proteins, overexpressing cell lines were constructed for the screening process. Specifically, CHO-K1 cells were transfected with a plasmid encoding the recombinant human SEZ6 protein or a plasmid encoding the cynomolgus monkey SEZ6 protein, and then cell lines stably expressing recombinant human or cynomolgus monkey SEZ6 protein were obtained by screening. Nucleotide sequences encoding the human or cynomolgus monkey SEZ6 protein were cloned into lentiviral expression vectors, and plasmids were prepared. The CHO-K1 cell line was transfected using lentiviral transfection methods well known in the industry, selectively cultured for two weeks using F-12 medium (purchased from Gibco) containing Puromycin, subcloned in 96-well culture plates by a limiting dilution method, and cultured in a cell culture incubator. After two weeks, the selected monoclonal colonies were detected and screened by flow cytometry analysis. The results in FIG. 2 show that the cell lines can stably express human SEZ6 or monkey SEZ6 at high levels and can be used for subsequent antibody screening processes.

### Example 3. Obtaining of murine anti-SEZ6 monoclonal antibodies by immunizing mice with recombinant SEZ6 proteins

To screen antibodies that can specifically bind to human and monkey SEZ6 membrane proteins, the full-length human SEZ6 expression plasmid, recombinant human SEZ6-Fc, and human SEZ6-His6 protein were used as immunogens to immunize 6-8-week-old Balb/c and SJL/J mice, and recombinant human SEZ6-His6 was used as immunogen to immunize Balb/c and SJL/J mice in this experiment. Among these, the primary immunization dose of the protein was 50 µg per mouse. 2 weeks after the primary immunization, booster immunizations were administered at a dose of 25 µg per mouse, with intervals of 2 weeks between booster immunizations.

One week after each booster immunization, serum samples were collected, and the antibody titre in the mouse serum was detected by ELISA. The plate was coated with 1 µg/mL recombinant human SEZ6-His6, incubated at 4°C overnight, blocked with a PBST buffer containing 1% BSA for 1 hour, and washed 3 times. Starting at 1:100 in a blocking buffer, the mouse serum was serially diluted 10-fold, and incubated at 37°C for 1 hour. The plate was washed 3 times, and incubated with an anti-mouse IgG-Fc secondary antibody (HRP-labelled) for 1 hour. The plate was washed 3 times with PBST, and 100 µL of TMB substrate was added to each well. After 15 minutes, the reaction was terminated with 2 M HCl, and the absorbance at 450 nm was determined. The sera of immunized mice showed varying degrees of binding to the immunogens, and the maximum dilution factor of the sera was 1:10⁵, at which the antigen-antibody reaction could still be exhibited.

For the final immunization, 25 µg of recombinant human SEZ6-His6 protein was administered via intraperitoneal injection. After 5 days, the mice were sacrificed, and their spleens were harvested and ground to collect splenocytes. The contaminating red blood cells in the splenocytes were lysed by adding NH4OH at a final concentration of 1% (w/w) to obtain a splenocyte suspension. The cells were washed 3 times by centrifugation at 1000 rpm. The mouse splenocytes were mixed with mouse myeloma cells SP2/0 at a ratio of 5:1 based on viable cell count, and cell fusion was performed using an efficient electrofusion method. The fused cells were diluted with DMEM medium containing 20% foetal bovine serum and 1×HAT (w/w), and added into a 96-well cell culture plate at 200 µL/1 × 10⁵ cells/well, and the plate was placed in an incubator at 37°C and 5% (v/v) CO2. After 14 days, the fused cells were screened by ELISA and FACS. Positive clones with OD450 nm > 1.0 and a FACS-positive cell ratio greater than 20% were expanded into a 24-well cell plate. The cells were further expanded at 37°C and 5% (v/v) CO2 in DMEM medium containing 10% (w/w) HT foetal bovine serum. After 3 days, the culture medium in the 24-well cell plate was centrifuged to collect the supernatant, and the binding activity to SEZ6 protein and SEZ6 positive cells was determined by ELISA and FACS. After two rounds of monoclonalization, positive clones were selected for sequencing. The variable region sequences of the murine antibodies are shown in Table 2.

**Table 2. Sequences of murine anti-SEZ6 antibodies**

| Antibody number | Heavy chain variable region (VH) and light chain variable region (VL) amino acid sequences | |
|---|---|---|
| cAb3003 | VH | QVQLQQSGAELVRPGASVTLSCKASGYTFTGYEMHWVKQTPVH GLEWIGGIDPETDNTVYNQKFKGKAILTEDKSSSTAYMELRRLTS EDSAVYYCTRGDWYFDIWGTGTTVTVSS (SEQ ID NO: 8) |
| | VL | DIVLSQSPSSQAVSVGEKVTMSCKSSQSLLNSRTRENYLAWYQQ KPGQSPKLLIYWASTRESGVPDRFTGSGSGTDFTLTISSVQAEDL AVYYCQQSYNLFTFGSGTILEIK (SEQ ID NO: 9) |
| cAb3005 | VH | QVQLQQSGAELARPGASVTLSCKASGYKFTDYEMHWVKETPVN GLEWIGGIDPETENTVYNQKFKVKAILTEDKSSRTAYMELRSLTS EDSAVYYCTRGDWYFDVWGTGTTVTVSS (SEQ ID NO: 10) |
| | VL | DIVMSQSPSSQAVSVGEKVTMSCKSSQSLLNSRTRENYLAWYQQ KAGQSPKLLIYWASTRDSGVPDRFTGSGSGTAFTLTINSVQAEDL AVYYCKQSYNLFTFGSGTKLEIK (SEQ ID NO: 11) |
| cAb3006 | VH | QVQLQQSGAELARPGASVTLSCKASGYTFTDYEMHWVKETPVD GLEWIGGIDPETDNTVYNQKFKVKAILTEDKSSRIAYMELRSLTS EDSAVYYCTRGDWYFDVWGTGTTVTVSS (SEQ ID NO: 12) |
| | VL | DIVMSQSPSSQAVSVGEKVTMSCKSSQSLLNSRTRENYLAWYQQ KAGQSPKLLIYWASTRDSGVPDRFTGSGSGTDFTLTINSVQAEDL AVYYCKQSYNLFTFGSGTKLEIK (SEQ ID NO: 13) |
| cAb3009 | VH | QVQLQQSGAELVRPGASVTLSCKASGYTFTDYEMHWVRQTPVH GLEWIGGIDPESDNTVYNQKFKGKVTLTEDKSSSTAYMEFRSLTS EDSAVYYCTRGDWYFDVWGTGTTVTVSS (SEQ ID NO: 14) |
| | VL | DIVMSQSPSSQAVSVGEKVTMTCKSSQSLLNSRTRENYLAWYQQ KPGQSPKLLIYWASTRESGVPDRFTGSGSGADFTLTISSVQAEDL AVYYCGQSYNLFTFGSGTKLEIK (SEQ ID NO: 15) |
| cAb3014 | VH | QVQLQQPGAELVKPGASVKLSCKASGYTFSIYWMHWVKQRPG QGLEWIGMIHPNSGSTNYNEKFKSKATLTVDKSSSTAYMQLSSL TSEDSAVYYCARGGNYVAMDYWGQGTSVTVSS (SEQ ID NO: 16) |
| | VL | QIVLTQSPAIMSASLGERVTMTCTASSSVSSSYLHWYQQKPGSSP KLWIYSTSNLASGVPARFSGSGSGTSYSLTISSMEAEDAATYYCH QYHRSLTFGSGTKLEIK (SEQ ID NO: 17) |
| cAb3031 | VH | DVQLQESGPGLVKPSQSLSLTCSVTGQSITSGYFWNWIRQLPGNK LEWMGHIRYDGNNNYNPSLKNRVSITRDTSKNQFFLKLNSVTTE DTATYYCARWLLRYYAMDSWGQGTSVTVSS (SEQ ID NO: 18) |
| | VL | DVVMTQTPLTLSVIIGQPASISCKSSQSLLDSDGKTYLNWLLQRP GHSPKRLIYLVSKLDSGVPDRFTGSGSGTDFTLKISRVEAEDLGV YYCWQGTHFPWTFGGGTKLEIK (SEQ ID NO: 19) |
| cAb3034 | VH | QVQLQQSGAELARPGASVKMSCKASGYTFTSYTMHWVKQRPG QGLEWIGYINPRSDYTKYNQKVKDKATLTADKSSSTAYMQLSSL TSEDSAVYYCARGGLGLDYWGQGTTLTVSS (SEQ ID NO: 20) |
| | VL | DIQMTQSPSSLSASLGERVSLTCRASQEISGYLSWLQQTPDGTIKR LIYAASTLHSGVPKRFSGSRSGSDYSLTISSLESEDFAGYYCLQYA SYPYTFGGGTKLEIK (SEQ ID NO: 21) |
| cAb3046 | VH | QVQLQQSGAELARPGASVKMSCKASGYTFTSYTMHWVKQRPG QGLEWIGYINPSSDYTKYNQKFKGKATMTADKSSSTAYMQLSSL TSEDSAVYYCGRGLVPFAYWGQGTLVTVSA (SEQ ID NO: 22) |
| | VL | DIQMTQSPSSLSASLGERVSLTCRASQEISGYLIWLQQKPDGTIKR LIYAASTLDSGVPKRVSGSRSGSDYSLTISSLESEDFADYYCLQY ASYPLTFGGGTKLELK (SEQ ID NO: 23) |
| cAb3056 | VH | QVQLQQSGPELMKPGASVKISCKASGYTFTDYYINWMKQRPGQ GLEWIGWIFPGSDNPYYNEMFKDKATVSVDKSSSAVFMLLTSLT SEDSAVYFCARGRYYGLYTMDYWGQGTSVTVSS (SEQ ID NO: 24) |
| | VL | DIQMTQTTSSLSASLGDRVTISCRASQDIRNYLNWYQQKADGTV KFLIYYTSRLHSGVPSRFSGSGSGTDFSLTISNLEQEDVATYFCQQ GNTLPWTFGGGTKVEIK (SEQ ID NO: 25) |

By replacing the constant regions of the murine monoclonal antibodies, recombinant chimeric antibodies were obtained. Subsequently, the nucleotide sequences encoding the variable regions of the murine monoclonal antibodies were cloned into the pTT5 vector containing the protein sequences encoding the human heavy and light chain constant regions (Human IgG1, kappa). The gene sequences were synthesized, and then transfected into HEK293 cells. After 5 days, the cells were removed by centrifugation, and the cell culture medium was collected and filtered. The harvested cell culture supernatant was loaded onto a protein A column (MabSelect SuRe, GE). The bound antibodies were eluted with glycine, and the eluate was neutralized with 1 M Tris. The buffer was replaced with PBS to obtain the chimeric antibodies.

### Example 4. Obtaining of anti-SEZ6 nanobodies by immunizing camels with recombinant SEZ6 protein

To screen more antibodies that specifically bind to human and monkey SEZ6 membrane proteins, camels were added as an immunized species in this experiment. The specific method was as follows: 2 healthy camels were immunized with recombinant human SEZ6-Fc. After 4-5 immunizations, 200 ml of peripheral blood was collected from each camel, and PBMCs were isolated. RNA was extracted using TriZol, and reverse-transcribed to prepare cDNA. The antibody gene was then amplified by PCR. After enzyme digestion, ligation, and transformation, a VHH phage display library (with a library capacity of 1.2 × 10⁹) was constructed. The random sequencing results show that the positive rate of antibody gene insertion reaches 96%.

The previously constructed immune antibody library was screened using biotinylated recombinant human SEZ6-His6 protein, and 3-5 rounds of affinity panning were performed. Monoclonal colonies were randomly picked to prepare phages, followed by phage ELISA detection. Positive clones were selected for sequencing, and the sequences were analysed. Clones with unique sequences were used to construct VHH-hFc expression vectors, which were transiently expressed in 293 cells. Detection was performed by ELISA and FACS. The amino acid sequences of some VHH antibodies are shown in Table 3.

**Table 3. Sequences of camel-derived anti-SEZ6 antibodies**

| Antibody number | Amino acid sequence of camel-derived VHH antibody | |
|---|---|---|
| cAb025 | VHH | EVQLVESGGGSVQPGGSLRLSCEASGLTYNKDCMAWFRQAPG KEREGVAIINGVGRTRYADSVKGRFTISKDNAKNTLYLQMNNL KPEDTAMYYCAAQRASSSCGSLSRAWYDYWGRGTQVTVSS (SEQ ID NO: 26) |
| cAb314 | VHH | HVQLVESGGGSVQAGGSLRLSCAVSGFTSSRGWMAWFRQAPG KEREGVAVIGRRSGDIYYADSLKGRFTISHDSTENTLYLQMNNL KPEDTATYYCAVGDQAGHVLTSSPYRYWGQGTQVTVSS (SEQ ID NO: 27) |
| cAb272 | VHH | DVQLVESGGGSVQAGGSLRVSCAVSGYTKSSGWMGWFRQAP GKEREGVAAIGRRSGDRYYVDSVKGRFTISLDSTKNTLYLQMN DLKPEDTGTYYCAAGGRAGNVLTSQPYRYWGQGTQVTVSS (SEQ ID NO: 28) |

### Example 5. Humanization of antibodies

To reduce immune responses in humans caused by non-human species, murine and camelid antibodies needed to be humanized. The specific implementation method was as follows: For humanized antibodies, after the structure of murine monoclonal antibodies was predicted by homology modelling, the CDRs of the murine antibodies were chimerized onto appropriate human GermLine frameworks (Bioinformation. 2014; 10(4): 180-186; Methods Mol Biol. 2019; 1904: 213-230), then back mutations were introduced at sites that potentially affect antibody-antigen binding, and finally the nucleotide sequences encoding the variable regions of the humanized monoclonal antibodies were cloned into the pTT5 vector containing the protein sequences encoding the human heavy and light chain constant regions (Human IgG1, kappa). Following a method similar to that described above, HEK293 cells were transfected to produce the humanized antibodies.

The human IgG1 constant region sequence is as follows: (SEQ ID NO: 33)

ASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTC
LVKGFYPSDIAVEWESNGQPENNYKTTPPVLDSDGSFFLYSKLTVDKSRW
QQGNVFSCSVMHEALHNHYTQKSLSLSPGK

The Kappa constant region sequence is as follows: (SEQ ID NO: 59)

The framework selection for the variable regions used in humanization of the murine antibodies is shown in Table 4, the variable region sequences of the humanized antibodies are shown in Table 5, the heavy chain CDRs (HCDR1/HCDR2/HCDR3) and light chain CDRs (LCDR1/LCDR2/LCDR3) are shown in Table 6, and the full-length amino acid sequences of the humanized antibodies are shown in Table 7. PR3178 was a humanized antibody of cAb3009, PR3121 and PR3123 were humanized antibodies of cAb3014, and PR3132 was a humanized antibody of cAb3056.

**Table 4. Framework selection for variable regions in humanization of murine antibodies**

| Murine antibody number | Humanized antibody number | Variable region | Framework 1 (FR1) | Framework 2 (FR2) | Framework 3 (FR3) | Framework 4 (FR4) |
|---|---|---|---|---|---|---|
| cAb3009 | PR3178 | VH | IGHV1-46*01 | IGHV1-46*01 | IGHV1-46*01 | IGHJ6*01 |
| | | VL | IGKV4-1*01 | IGKV4-1*01 | IGKV4-1*01 | IGKJ2*01 |
| cAb3014 | PR3121 | VH | IGHV1-2*02 | IGHV1-2*02 | IGHV1-2*02 | IGHJ4*01 |
| | | VL | IGKV1-39*01 | IGKV1-39*01 | IGKV1-39*01 | IGKJ4*01 |
| | PR3123 | VH | IGHV1-2*02 | IGHV1-2*02 | IGHV1-2*02 | IGHJ4*01 |
| | | VL | IGKV1-39*01 | IGKV1-39*01 | IGKV1-39*01 | IGKJ4*01 |
| cAb3056 | PR3132 | VH | IGHV1-46*01 | IGHV1-46*01 | IGHV1-46*01 | IGHJ4*01 |
| | | VL | IGKV1-39*01 | IGKV1-39*01 | IGKV1-39*01 | IGKJ4*01 |

**Table 5. Variable region sequences of humanized anti-SEZ6 antibodies**

| Antibody number | Amino acid sequences of heavy chain variable region (VH) and light chain variable region (VL) | |
|---|---|---|
| PR3178 | VH | QVQLVQSGAEVKKPGASVKVSCKASGYTFTDYEMHWVRQAP GQGLEWMGGIDPESDNTVYNQKFKGRVTLTEDTSTSTAYMEL RSLRSEDTAVYYCTRGDWYFDVWGQGTTVTVSS (SEQ ID NO: 29) |
| | VL | DIVMTQSPDSLAVSLGERATINCKSSQSLLNSRTRENYLAWYQ QKPGQPPKLLIYWASTRESGVPDRFSGSGSGTDFTLTISSLQAED VAVYYCGQSYNLFTFGQGTKLEIK (SEQ ID NO: 30) |
| PR3121 | VH | EVQLVQSGAEVKKPGSSVKVSCKASGYTFSIYWMHWVRQAPG QGLEWIGMIHPNSGSTNYNEKFKSRATLTVDKSTSTAYMELSS LRSEDTAVYYCARGGNYVAMDYWGQGTLVTVSS (SEQ ID NO: 31) |
| | VL | DIQMTQSPSSLSASVGDRVTITCTASSSVSSSYLHWYQQKPGKA PKLLIYSTSNLASGVPSRFSGSGSGTDFTLTISSLQPEDFATYYCH QYHRSLTFGGGTKVEIK (SEQ ID NO: 32) |
| PR3123 | VH | EVQLVQSGAEVKKPGSSVKVSCKASGYTFSIYWMHWVRQAPG QGLEWIGMIHPNSGSTNYNEKFKSRATLTVDKSTSTAYMELSS LRSEDTAVYYCARGGNYVAMDYWGQGTLVTVSS (SEQ ID NO: 31) |
| | VL | DIQLTQSPSSLSASVGDRVTMTCTASSSVSSSYLHWYQQKPGKS PKLWIYSTSNLASGVPSRFSGSGSGTDYTLTISSLQPEDFATYYC HQYHRSLTFGGGTKVEIK (SEQ ID NO: 34) |
| PR3132 | VH | EVQLVQSGAEVKKPGASVKISCKASGYTFTDYYINWMRQAPG QGLEWIGWIFPGSDNPYYNEMFKDRVTVTVDKSTSTVYMELSS LRSEDTAVYFCARGRYYGLYTMDYWGQGTLVTVSS (SEQ ID NO: 35) |
| | VL | DIQMTQSPSSLSASVGDRVTITCRASQDIRNYLNWYQQKPGKA PKFLIYYTSRLHSGVPSRFSGSGSGTDFTLTISSLQPEDFATYYC QQGNTLPWTFGGGTKVEIK (SEQ ID NO: 36) |

**Table 6. CDR sequences of humanized anti-SEZ6 antibodies (kabat numbering system)**

| Antibody number | Heavy chain variable region CDR | | Light chain variable region CDR | |
|---|---|---|---|---|
| PR3178 | HCDR1 | DYEMH (SEQ ID NO: 37) | LCDR1 | KSSQSLLNSRTRENYLA (SEQ ID NO: 40) |
| | HCDR2 | GIDPESDNTVYNQKFKG (SEQ ID NO: 38) | LCDR2 | WASTRES (SEQ ID NO: 41) |
| | HCDR3 | GDWYFDV (SEQ ID NO: 39) | LCDR3 | GQSYNLFT (SEQ ID NO: 42) |
| PR3121/ PR3123 | HCDR1 | IYWMH (SEQ ID NO: 43) | LCDR1 | TASSSVSSSYLH (SEQ ID NO: 46) |
| | HCDR2 | MIHPNSGSTNYNEKFKS (SEQ ID NO: 44) | LCDR2 | STSNLAS (SEQ ID NO: 47) |
| | HCDR3 | GGNYVAMDY (SEQ ID NO: 45) | LCDR3 | HQYHRSLT (SEQ ID NO: 48) |
| PR3132 | HCDR1 | DYYIN (SEQ ID NO: 49) | LCDR1 | RASQDIRNYLN (SEQ ID NO: 52) |
| | HCDR2 | WIFPGSDNPYYNEMFKD (SEQ ID NO: 50) | LCDR2 | YTSRLHS (SEQ ID NO: 53) |
| | HCDR3 | GRYYGLYTMDY (SEQ ID NO: 51) | LCDR3 | QQGNTLPWT (SEQ ID NO: 54) |

**Table 7. Full-length amino acid sequences of heavy and light chains of humanized antibodies**

| Antibody number | Full-length amino acid sequences of heavy chain and light chain | |
|---|---|---|
| PR3178 | Heavy chain | QVQLVQSGAEVKKPGASVKVSCKASGYTFTDYEMHWVRQAP GQGLEWMGGIDPESDNTVYNQKFKGRVTLTEDTSTSTAYMEL RSLRSEDTAVYYCTRGDWYFDVWGQGTTVTVSSASTKGPSVF PLAPSSKSTSGGTAALGCLVKDYFPEPVTVSWNSGALTSGVHT FPAVLQSSGLYSLSSVVTVPSSSLGTQTYICNVNHKPSNTKVD KKVEPKSCDKTHTCPPCPAPELLGGPSVFLFPPKPKDTLMISRT PEVTCVVVDVSHEDPEVKFNWYVDGVEVHNAKTKPREEQYN STYRVVSVLTVLHQDWLNGKEYKCKVSNKALPAPIEKTISKA KGQPREPQVYTLPPSREEMTKNQVSLTCLVKGFYPSDIAVEWE SNGQPENNYKTTPPVLDSDGSFFLYSKLTVDKSRWQQGNVFS CSVMHEALHNHYTQKSLSLSPGK (SEQ ID NO: 55) |
| | Light chain | DIVMTQSPDSLAVSLGERATINCKSSQSLLNSRTRENYLAWYQ QKPGQPPKLLIYWASTRESGVPDRFSGSGSGTDFTLTISSLQAE DVAVYYCGQSYNLFTFGQGTKLEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC (SEQ ID NO: 56) |
| PR3121 | Heavy chain | EVQLVQSGAEVKKPGSSVKVSCKASGYTFSIYWMHWVRQAP GQGLEWIGMIHPNSGSTNYNEKFKSRATLTVDKSTSTAYMEL SSLRSEDTAVYYCARGGNYVAMDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAVE WESNGQPENNYKTTPPVLDSDGSFFLYSKLTVDKSRWQQGNV FSCSVMHEALHNHYTQKSLSLSPGK (SEQ ID NO: 57) |
| | Light chain | DIQMTQSPSSLSASVGDRVTITCTASSSVSSSYLHWYQQKPGK APKLLIYSTSNLASGVPSRFSGSGSGTDFTLTISSLQPEDFATYY CHQYHRSLTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC (SEQ ID NO: 58) |
| PR3123 | Heavy chain | EVQLVQSGAEVKKPGSSVKVSCKASGYTFSIYWMHWVRQAP GQGLEWIGMIHPNSGSTNYNEKFKSRATLTVDKSTSTAYMEL |
| | | SSLRSEDTAVYYCARGGNYVAMDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNTK VDKKVEPKSCDKTHTCPPCPAPELLGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAVE WESNGQPENNYKTTPPVLDSDGSFFLYSKLTVDKSRWQQGNV FSCSVMHEALHNHYTQKSLSLSPGK (SEQ ID NO: 57) |
| | Light chain | DIQLTQSPSSLSASVGDRVTMTCTASSSVSSSYLHWYQQKPGK SPKLWIYSTSNLASGVPSRFSGSGSGTDYTLTISSLQPEDFATY YCHQYHRSLTFGGGTKVEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC (SEQ ID NO: 60) |
| PR3132 | Heavy chain | EVQLVQSGAEVKKPGASVKISCKASGYTFTDYYINWMRQAPG QGLEWIGWIFPGSDNPYYNEMFKDRVTVTVDKSTSTVYMELS SLRSEDTAVYFCARGRYYGLYTMDYWGQGTLVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSNT KVDKKVEPKSCDKTHTCPPCPAPELLGGPSVFLFPPKPKDTLM ISRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIAV EWESNGQPENNYKTTPPVLDSDGSFFLYSKLTVDKSRWQQGN VFSCSVMHEALHNHYTQKSLSLSPGK (SEQ ID NO: 61) |
| | Light chain | DIQMTQSPSSLSASVGDRVTITCRASQDIRNYLNWYQQKPGKA PKFLIYYTSRLHSGVPSRFSGSGSGTDFTLTISSLQPEDFATYYC QQGNTLPWTFGGGTKVEIKRTVAAPSVFIFPPSDEQLKSGTAS VVCLLNNFYPREAKVQWKVDNALQSGNSQESVTEQDSKDST YSLSSTLTLSKADYEKHKVYACEVTHQGLSSPVTKSFNRGEC (SEQ ID NO: 62) |

The variable region frameworks used for humanization of the nanobodies are shown in Table 8, the variable region sequences of the humanized antibodies are shown in Table 9, the heavy chain CDRs (HCDR1/HCDR2/HCDR3) are shown in Table 10, and the amino acid sequences of the humanized antibodies fused to the human IgG1-Fc fragment are shown in Table 11. PR0069 was a humanized antibody of cAb025, and PR0071 was a humanized antibody of cAb314.

**Table 8. Framework selection for variable regions in humanization of anti-SEZ6 nanobodies**

| Camel antibody number | Humanized antibody number | Variable region | Framework 1 (FR1) | Framework 2 (FR2) | Framework 3 (FR3) | Framework 4 (FR4) |
|---|---|---|---|---|---|---|
| cAb025 | PR0069 | VHH | IGHV3-7*01 | IGHV3-7*01 | IGHV3-7*01 | IGHJ2*01 |
| cAb314 | PR0071 | VHH | IGHV3-7*01 | IGHV3-7*01 | IGHV3-7*01 | IGHJ3*01 |

**Table 9. Variable region sequences of humanized anti-SEZ6 nanobodies**

| Antibody number | Amino acid sequence of heavy chain variable region (VHH) | |
|---|---|---|
| PR0069 | VHH | EVQLVESGGGLVQPGGSLRLSCAASGFTFSKDCMAWFRQAPG KGREGVAIISGVGRTRYADSVKGRFTISRDNAKNSLYLQMNSL RAEDTAVYYCAAQRASSSCGSLSRAWYDYWGRGTLVTVSS (SEQ ID NO: 63) |
| PR0071 | VHH | HVQLVESGGGLVQPGGSLRLSCAVSGFTSSRGWMAWFRQAPG KEREGVAVIGRRSGDIYYADSLKGRFTISHDNAKNSLYLQMNS LRAEDTATYYCAVGDQAGHVLTSSPYRYWGQGTMVTVSS (SEQ ID NO: 64) |

**Table 10. CDR sequences of humanized anti-SEZ6 nanobodies (kabat numbering system)**

| Antibody number | VHH variable region CDR | |
|---|---|---|
| PR0069 | HCDR1 | KDCMA (SEQ ID NO: 65) |
| | HCDR2 | IISGVGRTRYADSVKG (SEQ ID NO: 66) |
| | HCDR3 | QRASSSCGSLSRAWYDY (SEQ ID NO: 67) |
| PR0071 | HCDR1 | RGWMA (SEQ ID NO: 68) |
| | HCDR2 | VIGRRSGDIYYADSLKG |
| | | (SEQ ID NO: 69) |
| | HCDR3 | GDQAGHVLTSSPYRY (SEQ ID NO: 70) |

**Table 11. Amino acid sequences of humanized anti-SEZ6 nanobody-Fc proteins**

| Antibody number | Full-length amino acid sequence | |
|---|---|---|
| PR0069 | VHH -Fc | EVQLVESGGGLVQPGGSLRLSCAASGFTFSKDCMAWFRQAPG KGREGVAIISGVGRTRYADSVKGRFTISRDNAKNSLYLQMNSL RAEDTAVYYCAAQRASSSCGSLSRAWYDYWGRGTLVTVSSEP KSSDKTHTCPPCPAPELLGGPSVFLFPPKPKDTLMISRTPEVTCV VVDVSHEDPEVKFNWYVDGVEVHNAKTKPREEQYNSTYRVV SVLTVLHQDWLNGKEYKCKVSNKALPAPIEKTISKAKGQPREP QVYTLPPSREEMTKNQVSLTCLVKGFYPSDIAVEWESNGQPEN NYKTTPPVLDSDGSFFLYSKLTVDKSRWQQGNVFSCSVMHEAL HNHYTQKSLSLSPGK (SEQ ID NO: 71) |
| PR0071 | VHH -Fc | HVQLVESGGGLVQPGGSLRLSCAVSGFTSSRGWMAWFRQAPG KEREGVAVIGRRSGDIYYADSLKGRFTISHDNAKNSLYLQMNS LRAEDTATYYCAVGDQAGHVLTSSPYRYWGQGTMVTVSSEPK SSDKTHTCPPCPAPELLGGPSVFLFPPKPKDTLMISRTPEVTCVV VDVSHEDPEVKFNWYVDGVEVHNAKTKPREEQYNSTYRVVS VLTVLHQDWLNGKEYKCKVSNKALPAPIEKTISKAKGQPREPQ VYTLPPSREEMTKNQVSLTCLVKGFYPSDIAVEWESNGQPENN YKTTPPVLDSDGSFFLYSKLTVDKSRWQQGNVFSCSVMHEALH NHYTQKSLSLSPGK (SEQ ID NO: 72) |

### Example 6 SEZ6 binding activity of anti-SEZ6 antibodies on cells

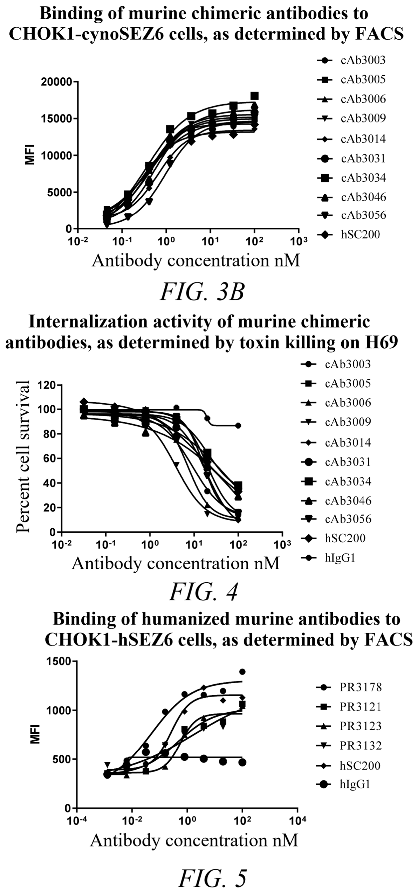

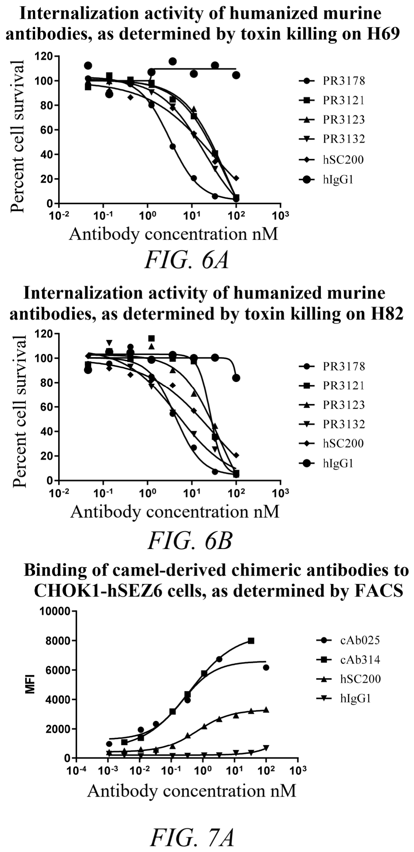

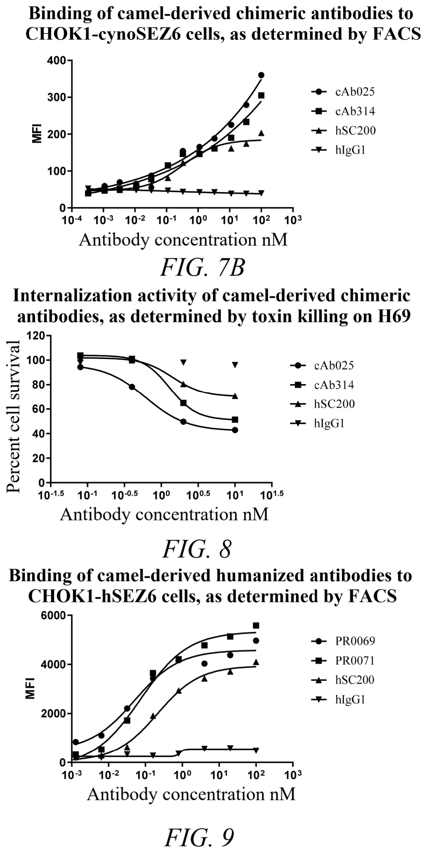

To screen chimeric or humanized antibodies that can bind to human and monkey SEZ6 membrane proteins with high affinity, the fluorescence signal of antibodies bound to the cell surface was detected, and the binding strength of the antibodies was evaluated on the basis of the intensity of the fluorescence signal. Specifically: Serially diluted antibody molecules and control molecules were incubated with 1 × 10⁵ cells at 4°C for 1 hour. Excess antibodies were washed away. An Alexa Flour 647-labeled murine anti-human Fc antibody was added, followed by incubation at 4°C for 30 minutes. After washing away excess antibodies, the cells were resuspended in 200 µL of 1% BSA/PBS buffer. The fluorescence signal on the cell surface was read using a Thermo Attune NxT flow cytometer. Table 12 shows the binding characteristics of murine chimeric antibodies (FIG. 3), Table 13 shows the binding characteristics of humanized murine antibodies (FIG. 5), Table 14 shows the binding characteristics of chimeric nanobodies (FIG. 7), and Table 15 shows the affinity characteristics of humanized nanobodies (FIG. 9). The results show that: the candidate antibodies have SEZ6 membrane protein binding activity close to that of hSC200.

**Table 12. In vitro characterization of murine SEZ6 chimeric antibodies**

| **Chimeric antibody name or number** | **Binding to CHO-SEZ6, as determined by FACS** | | **Binding to CHO-cynoSEZ6, as determined by FACS** | |
|---|---|---|---|---|
| | **EC50 (nM)** | **MFIₘₐₓ** | **EC50 (nM)** | **MFIₘₐₓ** |
| cAb3003 | 0.68 | 5112 | 0.42 | 14254 |
| cAb3005 | 0.52 | 4918 | 0.41 | 14703 |
| cAb3006 | 0.35 | 4827 | 0.41 | 14995 |
| cAb3009 | 0.81 | 5415 | 0.43 | 15567 |
| cAb3014 | 0.85 | 3031 | 0.65 | 13421 |
| cAb3031 | 0.42 | 5175 | 0.54 | 15209 |
| cAb3034 | 0.13 | 10099 | 0.37 | 17323 |
| cAb3046 | 0.06 | 5183 | 0.48 | 16231 |
| cAb3056 | 0.42 | 6556 | 0.95 | 14566 |
| hSC200 | 0.19 | 4030 | 0.26 | 13188 |

**Table 13. In vitro characterization of humanized murine anti-SEZ6 antibodies**

| **Humanized antibody name or number** | **Binding to CHO-SEZ6, as determined by FACS** | |
|---|---|---|
| | **EC50 (nM)** | **MFIₘₐₓ** |
| PR3178 | 0.05 | 1305 |
| PR3121 | 0.45 | 1015 |
| PR3123 | 0.56 | 966 |
| PR3132 | 1.72 | 1206 |
| hSC200 | 0.26 | 1155 |

**Table 14. In vitro characterization of camel-derived SEZ6 chimeric antibodies**

| **Chimeric antibody** | **Binding to CHO-SEZ6, as determined by FACS** | |
|---|---|---|
| **name or number** | **EC50 (nM)** | **MF_{Ima}x** |
| cAb025 | 0.23 | 6603 |
| cAb314 | 0.38 | 8476 |
| hSC200 | 0.66 | 3305 |

**Table 15. In vitro characterization of humanized camel-derived anti-SEZ6 antibodies**

| **Humanized antibody name or number** | **Binding to CHO-SEZ6, as determined by FACS** | |
|---|---|---|
| | **EC50 (nM)** | **MFIₘₐₓ** |
| PR0069 | 0.05 | 4584 |
| PR0071 | 0.07 | 5338 |
| hSC200 | 0.21 | 3943 |

### Example 7 Determination of in vitro cellular internalization activity of anti-SEZ6 antibodies

To evaluate the internalization activity of antibodies, a DT3C antibody internalization activity evaluation system was used. DT3C is a recombinantly expressed fusion protein with a molecular weight of 70 KD, formed by fusing Fragment A of diphtheria toxin (toxin moiety only) and the 3C fragment of group G streptococcus (IgG-binding moiety). This protein has a high affinity for the IgG portion of an antibody, and enters cells along with the antibody upon internalization. Within the cell, under the action of furin protease, the toxic DT is released. The DT can inhibit the activity of EF2-ADP ribosylation, block the protein translation process, and finally cause cell death. By using this system, the cell-killing effect resulting from both antibody internalization and immunotoxins can be observed simultaneously (Yamaguchi, M., Hama, H., et al., Biochemical and Biophysical Research Communications 454 (2014) 600-603).

The evaluation of internalization and immunotoxin activity dependent on DT3C was specifically as follows: Sterile-filtered DT3C and the test chimeric antibody (the molar concentration of DT3C was twice the molar concentration of the antibody) were uniformly mixed in a 1:1 volume ratio, and stood and incubated at 37°C for 30 minutes. The resulting mixture was then serially diluted with complete medium, and added to cells (H69 or H82) (3000 cells/well), followed by incubation in an incubator at 37°C and 5% carbon dioxide for 6 days. CellTiter-Glo was added, followed by incubation at room temperature in the dark for 10 min. Chemiluminescence was read using PerkinElmer. As shown in FIG. 4: the murine chimeric antibodies cAb3009, cAb3014, and cAb3056 exhibit stronger internalization and immunotoxin killing activity than those of the control molecule hSC200. Additionally, the series of sequence-similar chimeric antibodies cAb3003, cAb3005, cAb3006, and cAb3009, all exhibit significantly superior internalization activity compared to the control molecule hSC200. For this series of sequences, the general formulas of the CDRH1, CHDRH2, and CDRH3 are XYEMH, GIDPEXXNTVYNQKFKX, and GDWYFDX; and the general formulas of the CDRL1, CDRL2, and CDRL3 are KSSQSLLNSRTRENYLA, WASTRXX, and XQSYNLFT.

**Table 16. In vitro characterization of humanized murine anti-SEZ6 antibodies**

| **Chimeric antibody name or number** | **Toxin killing on H82** | |
|---|---|---|
| | **EC50 (nM)** | **Maximum killing (%)** |
| PR3178 | 4.25 | 95.4 |
| PR3121 | 26.20 | 94.0 |
| PR3123 | 40.78 | 95.0 |
| PR3132 | 4.74 | 94.0 |
| Hsc200 | 27.92 | 79.4 |

**Table 17. In vitro characterization of camel-derived SEZ6 chimeric antibodies**

| **Chimeric antibody name or number** | **Toxin killing on H69** | |
|---|---|---|
| | EC50 (nM) | Maximum killing (%) |
| cAb025 | 0.62 | 57.2 |
| cAb314 | 1.30 | 48.6 |
| hSC200 | 1.42 | 29.3 |

**Table 18. In vitro characterization of humanized camel-derived anti-SEZ6 antibodies**

| **Chimeric antibody name or number** | **Toxin killing on H69** | |
|---|---|---|
| | **EC50 (nM)** | **Maximum killing (%)** |
| PR0069 | 1.49 | 99.0 |
| PR0071 | 4.21 | 92.0 |
| hSC200 | 15.83 | 67.0 |

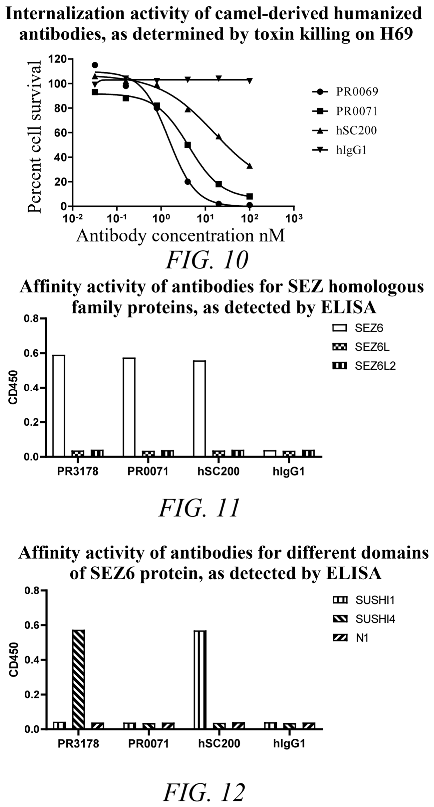

As shown in Table 16 and FIG. 6: the humanized murine anti-SEZ6 antibodies PR3178, PR3121, PR3123, and PR3132 exhibit internalization and immunotoxin killing activity comparable to or slightly stronger than those of the control molecule hSC200. As shown in Table 17 and FIG. 8: the camel-derived chimeric antibodies cAb025 and cAb314 exhibit stronger internalization and immunotoxin killing activity than those of the control molecule hSC200. As shown in Table 18 and FIG. 10: the humanized camel-derived anti-SEZ6 antibodies PR0069 and PR0071 exhibit stronger internalization and immunotoxin killing activity than those of the control molecule hSC200.

### Example 8 Detection of affinity of anti-SEZ6 antibodies for SEZ6 homologous family proteins SEZ6L and SEZ6L2

To screen antibodies that can specifically bind to SEZ6, the binding of the antibodies to SEZ6 homologous family proteins was detected by ELISA. Specifically: Recombinant human SEZ6-Fc fusion protein, SEZ6L-Fc protein, and SEZ6L2-Fc were diluted to 1 µg/mL with PBS, added to ELISA microplates at 100 µL per well, and incubated overnight at 4°C; an ELISA blocking solution (PBS buffer containing 1% BSA (w/v), pH 7.4) was added, the plates were blocked at 37°C for 2 hours, 10 nM detection antibodies were then added separately, and the plates were incubated at 37°C for 1 hour, and washed 2-3 times with a washing solution; and a horseradish peroxidase (HRP)-labelled secondary antibody was added, and the plates were incubated at 37°C for 1 hour, and washed 2-3 times with a washing solution. 100 µL of TMB substrate was added into each well, and incubated for 15 minutes at room temperature, and 50 µL of stop solution (2 M HCl) was then added to each well. OD450nm values were read using an ELISA plate reader (SpectraMax M5e).

From the results as shown in FIG. 11, both PR3178 and PR0071 bind to SEZ6 protein and do not bind to SEZ6L protein and SEZ6L2 protein, indicating that PR3178 and PR0071 have good binding specificity.

### Example 9 Identification of SEZ6-affinity epitopes of anti-SEZ6 antibodies

The internalization activity of an antibody correlates with antigen-affinity epitopes. The antigen-affinity epitopes of the antibodies were identified by ELISA in this experiment. Specifically: Recombinant human SEZ6-SUSHI1 protein, SEZ6L-SUSHI2 protein, and SEZ6-N1 protein were diluted to 1 µg/mL with PBS, respectively, added to ELISA microplates at 100 µL per well, and incubated overnight at 4°C; an ELISA blocking solution (PBS buffer containing 1% BSA (w/v), pH 7.4) was added, the plates were blocked at 37°C for 2 hours, 10 nM detection antibodies were then added separately, and the plates were incubated at 37°C for 1 hour, and washed 2-3 times with a washing solution; and a horseradish peroxidase (HRP)-labelled secondary antibody was added, and the plates were incubated at 37°C for 1 hour, and washed 2-3 times with a washing solution. 100 µL of TMB substrate was added into each well, and incubated for 15 minutes at room temperature, and 50 µL of stop solution (2 M HCl) was then added to each well. OD450nm values were read using an ELISA plate reader (SpectraMax M5e).

From the results as shown in FIG. 12, PR3178 binds to the SUSHI4 domain of the SEZ6 protein, while PR0071 binds to an individual domain of SEZ6 other than SUSHI1, SUSHI4, and N1. The screened antibody molecules all have unique affinity epitopes and exhibit superior internalization activity.

### Example 10 Evaluation of kinetics of antibody-SEZ6 interaction by surface plasmon resonance SPR technology

The affinity activity of antibodies PR3178 and PR0071 was detected by SPR using a BIAcore 8K (Cityva) system. The sensor chip protein A and related reagents for detection were purchased from Cytiva. Antibodies PR3178 and PR0071 and the control antibody were diluted to 1 µg/ml with HBS-EP+ buffer, respectively. The flow rate was set to 10 µl/min. The antibodies were captured to a level of 200 RU. His-tagged SEZ6 antigen was diluted with HBS-EP+ buffer in a certain ratio to achieve a concentration gradient of 0 nM, 3.125 nM, 6.25 nM, 12.5 nM (two replicates), 25 nM, 50 nM, 100 nM, and 200 nM. The flow rate was set to 30 µl/min during sample analysis. The association time was 120 s, and the dissociation time was 900 s. Regeneration was then performed using pH 1.5 Gly-HCl buffer as the regeneration buffer, with the regeneration flow rate set to 30 µl/min and the regeneration time set to 30 s. The response signal was plotted with analysis time as the abscissa and response value as the ordinate. The obtained data were fitted using BIAcore 8K analysis software, employing a 1: 1 Langmuir binding model to determine kinetic constants such as association rate constant (Ka), dissociation rate constant (Kd), and equilibrium dissociation constant (KD). The results show that PR3178 and PR0071 molecules bind to human and cynomolgus monkey antigens, and their affinity was stronger than that of hSC200.

**Table 19. Binding kinetic parameters of anti-SEZ6 antibodies**

| Kinetic determination | Human SEZ6-His6 | | | Cynomolgus monkey SEZ6-His6 | | |
|---|---|---|---|---|---|---|
| Antibody name | Ka (1/Ms) | Kd (1/s) | KD (M) | Ka (1/Ms) | Kd (1/s) | KD (M) |
| PR3178 | 3.96E+05 | 6.40E-04 | 1.61E-09 | 3.98E+05 | 6.16E-04 | 1.55E-09 |
| PR0071 | 2.57E+05 | 5.13E-04 | 2.00E-09 | 1.65E+05 | 6.65E-04 | 4.03E-09 |
| hSC200 | 7.82E+04 | 4.68E-03 | 5.99E-08 | 8.20E+04 | 4.75E-03 | 5.79E-08 |

### Example 11 Conjugation of PR3178 and PR0071 with small molecule toxin

To target the small molecule toxin 9106 to tumour sites, antibodies PR3178 and PR0071 needed to be conjugated with the small molecule toxin 9106 in this experiment. The specific experiment was as follows: At 37°C, a prepared aqueous solution of tris(2-carboxyethyl)phosphine hydrochloride (TCEP-HCl) (10 mM, 75.4 µL, 753.5 nmol) was added to a solution of antibody in aqueous PBS buffer (0.05 M aqueous PBS buffer with pH = 6.5; 10.0 mg/mL, 5.0 mL, 342.5 nmol). The resulting mixture was placed in a shaker under water bath, and reacted with shaking at 37°C for 3 hours, and then the reaction was stopped. The reaction solution was cooled to 25°C in a water bath.

Compound 9106 (3.7 mg, 3424.6 nmol), prepared according to WO 2020063676 A1, was dissolved in 250 µL of dimethyl sulfoxide, and the resulting solution was added to the above reaction solution. The resulting mixture was placed in a shaker under water bath, and reacted with shaking at 25°C for 3 hours, and then the reaction was stopped. The reaction solution was desalted using HiPrep 26/10 (buffer: PBS pH 7.2-7.4, flow rate 10 mL/min), and concentrated using an ultrafiltration tube, yielding PBS buffer of PR3178-9106 and PR0071-9106 (PR3178-9106 concentration was 1.0 mg/mL, 26 mg; PR0071-9106 concentration was 2.0 mg/mL, 20 mg), which were refrigerated at 4°C. The anti-SEZ6 detection antibody hSC200 (hSC17.200, heavy chain SEQ ID NO: 6, light chain SEQ ID NO: 7) was conjugated with 9106 using the same method described above.

The structures of the obtained antibody-drug conjugates PR3178-9106 and PR0071-9106 are as follows. The drug loading was calculated by RP-HPLC, and the DAR values for PR3178-9106 and PR0071-9106 were: n = 4.1 and n = 3.6, respectively.

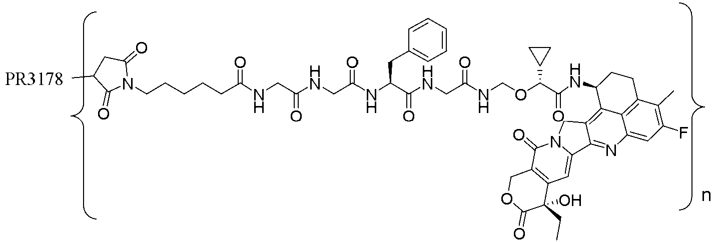

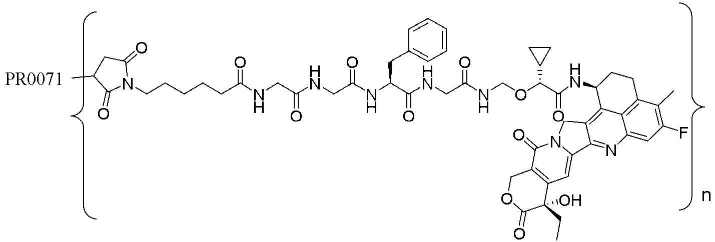

### Example 12 Determination of activity of ADC in inhibiting proliferation of tumour cell lines in vitro

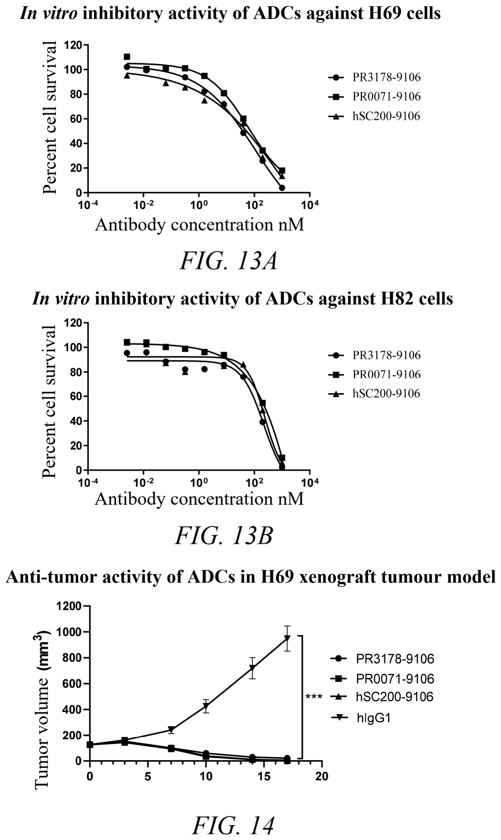

To detect the *in vitro* anti-tumour activity of ADC molecules after conjugation, the *in vitro* activity of the ADCs was reflected by the inhibition of the growth of tumour cell lines. Specifically: Detection cells H69 and H82 were grown overnight in 96-well culture plates, with a density of 3000 cells per well, respectively. On the next day, equal volumes of serially diluted toxin-drug conjugates were added. After 5 days, cell viability was determined using the CellTiter-Glo luminescent cell viability assay kit (Promega), as described in the manufacturer's protocol. The cell viability was evaluated as a percentage of that of the untreated control cells. As shown in FIG. 13, the ADC drugs have good killing activity against H69 and H82 tumour cell lines.

**Table 20. Killing activity of ADCs against cell lines with different expression levels**

| Cell line | Concentration for 50% of maximal killing (nM) | | |
|---|---|---|---|
| | PR3178-9106 | PR0071-9106 | hSC200-9106 |
| H69 | 38.51 | 73.36 | 52.18 |
| H82 | 136.54 | 252.27 | 194.01 |

### Example 13 Evaluation of in vivo anti-tumour activity of ADCs

To detect the *in vivo* anti-tumour activity of ADC molecules, an H69 xenograft tumour model was used to detect changes in tumour growth size to reflect the anti-tumour activity of the ADCs. Specifically: To generate xenografts, SCID mice were subcutaneously inoculated on the right rear back with 5 × 10^6 H69 cells/0.2 ml (PBS+gel). When the mean tumour volume reached 128 mm³, the mice were divided into 5 groups based on tumour volume and body weight, with 6 mice per group. Administration was initiated on the day of grouping, with two intraperitoneal administrations on day 0 and day 7. The tumour volume and body weight were measured twice weekly, and the data were recorded.

Tumour volume V = 1/2×a×b², where a and b represent the length and width, respectively. Relative tumour proliferation rate T/C (%) = (T-T0)/(C-C0) × 100, where T and C are the tumour volumes of the treatment group and the control group at the end of the experiment; and T0 and C0 are the tumour volumes of the treatment group and the control group at the beginning of the experiment. Tumour growth inhibition TGI (%) = 1-T/C (%). As described above, a human IgG control antibody was used as a negative control. TGI represents the maxim tumour growth inhibition during the experiment.

From the study results as shown in FIG. 14 and Table 21, at equimolar dose levels, the ADCs exhibit significant anti-tumour activity against the growth of H69 xenograft tumours.

**Table 21. In vivo anti-tumour activity of anti-SEZ6 ADC**

| ADC | DAR | Dose/Route/Regimen | Number of mice | TGImax (%) |
|---|---|---|---|---|
| PR3178-9106 | 4 | 3 mg/kg/ip/QW*3 | 6 | 97.77%*** |
| PR0071-9106 | 4 | 1.5 mg/kg/ip/QW*3 | 6 | 99.83%*** |
| hSC200-9106 | 4 | 3 mg/kg/ip/QW*3 | 6 | 99.41%*** |
| hIgG1 | 4 | 3 mg/kg/ip/QW*3 | 6 | - |

| | | | | |
|---|---|---|---|---|
| Note: vs control hIgG1: *P < 0.05, **p < 0.001, ***p < 0.0001 | | | | |

### Example 14 Determination of serum concentration of toxin-drug conjugates

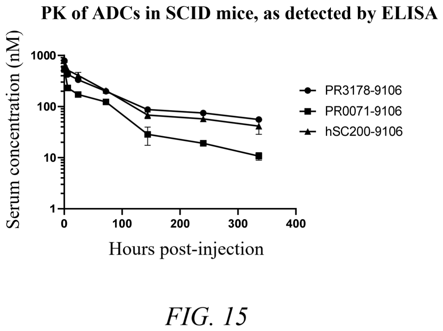

To detect the pharmacokinetic characteristics of ADCs in mice, the concentration of the ADCs in mouse serum after a single administration was measured by ELISA. Specifically: SCID mice were used. An equimolar dose of the ADC drug was administered via tail vein injection as a single administration. Blood was collected at the following time points: 15 min, 6 h, 24 h, 72 h, 144 h, 240 h, and 336 h after the first administration. The serum concentration was detected by ELISA. Plates were coated with anti-hFc and incubated overnight at 4°C. After the plates were washed once with 300 µL of PBST buffer, diluted test serum was added and incubated for 1 hour at room temperature. Each tube of serum was detected in two plates. For one serum plate, after the plate was washed three times with 300 µL of PBST buffer, 100 µL of HRP-labelled goat anti-human IgG Fc solution was added to each well and incubated for one hour at room temperature. After the plate was washed five times with PBST buffer, a chromogenic solution was added. After the colour development was completed, a stop solution was added, and the OD450 was read using a microplate reader. PK parameters were calculated using the non-compartmental model in Phoenix software. The results are shown in FIG. 15 and Table 22, indicating that PR3178-9106 has pharmacokinetic characteristics superior to those of hSC200-9106.

**Table 22. Pharmacokinetic parameters of ADC drugs in mice**

| PK parameter | Dose (mpk) | T1/2 (days) | AUC 0-t (h*nmol/ml) | Cmax (nmol/ml) | CL (L/h/kg) |
|---|---|---|---|---|---|
| PR3178-9106 | 10 | 4.71±0.11 | 56605±2555 | 785.94±46 | 0.0012 |
| PR0071-9106 | 5 | 3.01±0.10 | 23357±870 | 547.39±67 | 0.0029 |
| hSC200-9106 | 10 | 3.71±0.26 | 53007±2981 | 831.27+85 | 0.0013 |

The use and welfare of experimental animals in the present disclosure comply with the regulations of Association for Assessment and Accreditation of Laboratory Animal Care (AAALAC International). The health status and mortality of the animals were monitored daily, and routine examinations included observing the effects of test substances and drugs on the daily behavioural performance of the animals, such as behavioural activities, body weight changes, and external signs.

Although the specific embodiments of the present disclosure have been described above, it will be understood by those skilled in the art that these are merely illustrative, and that various changes or modifications can be made to these embodiments without departing from the principles and spirit of the present disclosure. Therefore, the scope of protection of the present disclosure is defined by the appended claims.

## Claims

1. An anti-SEZ6 antibody or an antigen-binding fragment thereof, **characterized by** comprising a heavy chain variable region (VH) or a heavy chain single domain (VHH), wherein the heavy chain variable region or the heavy chain single domain comprises:
(1) heavy chain HCDR1, HCDR2, and HCDR3 as set forth in amino acid sequences of SEQ ID NOs: 37, 38, and 39; or
(2) heavy chain HCDR1, HCDR2, and HCDR3 as set forth in amino acid sequences of SEQ ID NOs: 43, 44, and 45; or
(3) heavy chain HCDR1, HCDR2, and HCDR3 as set forth in amino acid sequences of SEQ ID NOs: 49, 50, and 51; or
(4) heavy chain HCDR1, HCDR2, and HCDR3 as set forth in amino acid sequences of SEQ ID NOs: 65, 66, and 67; or
(5) heavy chain HCDR1, HCDR2, and HCDR3 as set forth in amino acid sequences of SEQ ID NOs: 68, 69, and 70.

2. The anti-SEZ6 antibody or the antigen-binding fragment thereof according to claim 1, **characterized by** comprising a heavy chain variable region (VH) and a light chain variable region (VL), wherein the heavy chain variable region and the light chain variable region comprise:
(1) heavy chain HCDR1, HCDR2, and HCDR3 as set forth in the amino acid sequences of SEQ ID NOs: 37, 38, and 39, and light chain LCDR1, LCDR2, and LCDR3 as set forth in the amino acid sequences of SEQ ID NOs: 40, 41, and 42; or
(2) heavy chain HCDR1, HCDR2, and HCDR3 as set forth in the amino acid sequences of SEQ ID NOs: 43, 44, and 45, and light chain LCDR1, LCDR2, and LCDR3 as set forth in the amino acid sequences of SEQ ID NOs: 46, 47, and 48; or
(3) heavy chain HCDR1, HCDR2, and HCDR3 as set forth in the amino acid sequences of SEQ ID NOs: 49, 50, and 51, and light chain LCDR1, LCDR2, and LCDR3 as set forth in the amino acid sequences of SEQ ID NOs: 52, 53, and 54.

3. The anti-SEZ6 antibody or the antigen-binding fragment thereof according to claim 1, **characterized in that** the heavy chain single domain comprises an amino acid sequence as set forth in SEQ ID NO: 63, or a sequence having at least 85%, 90%, 95%, or 99% homology thereto; or an amino acid sequence as set forth in SEQ ID NO: 64, or a sequence having at least 85%, 90%, 95%, or 99% homology thereto;
preferably, the heavy chain single domain comprises the amino acid sequence as set forth in SEQ ID NO: 63 or the amino acid sequence as set forth in SEQ ID NO: 64.

4. The anti-SEZ6 antibody or the antigen-binding fragment thereof according to claim 1 or 2, **characterized by** comprising a heavy chain variable region and a light chain variable region, wherein
the heavy chain variable region comprises an amino acid sequence as set forth in SEQ ID NO: 29, SEQ ID NO: 31, or SEQ ID NO: 35, or a sequence having at least 85%, 90%, 95%, or 99% homology thereto;
the light chain variable region comprises an amino acid sequence as set forth in SEQ ID NO: 30, SEQ ID NO: 32, SEQ ID NO: 34, or SEQ ID NO: 36, or a sequence having at least 85%, 90%, 95%, or 99% homology thereto.

5. The anti-SEZ6 antibody or the antigen-binding fragment thereof according to claim 4, **characterized by** comprising a heavy chain variable region and a light chain variable region, wherein
(1) the heavy chain variable region comprises an amino acid sequence as set forth in SEQ ID NO: 29, or a sequence having at least 85%, 90%, 95%, or 99% homology thereto, and the light chain variable region comprises an amino acid sequence as set forth in SEQ ID NO: 30, or a sequence having at least 85%, 90%, 95%, or 99% homology thereto; or
(2) the heavy chain variable region comprises an amino acid sequence as set forth in SEQ ID NO: 31, or a sequence having at least 85%, 90%, 95%, or 99% homology thereto, and the light chain variable region comprises an amino acid sequence as set forth in SEQ ID NO: 32, or a sequence having at least 85%, 90%, 95%, or 99% homology thereto; or
(3) the heavy chain variable region comprises an amino acid sequence as set forth in SEQ ID NO: 31, or a sequence having at least 85%, 90%, 95%, or 99% homology thereto, and the light chain variable region comprises an amino acid sequence as set forth in SEQ ID NO: 34, or a sequence having at least 85%, 90%, 95%, or 99% homology thereto; or
(4) the heavy chain variable region comprises an amino acid sequence as set forth in SEQ ID NO: 35, or a sequence having at least 85%, 90%, 95%, or 99% homology thereto, and the light chain variable region comprises an amino acid sequence as set forth in SEQ ID NO: 36, or a sequence having at least 85%, 90%, 95%, or 99% homology thereto;
preferably, (1) the heavy chain variable region comprises the amino acid sequence as set forth in SEQ ID NO: 29, and the light chain variable region comprises the amino acid sequence as set forth in SEQ ID NO: 30; or
(2) the heavy chain variable region comprises the amino acid sequence as set forth in SEQ ID NO: 31, and the light chain variable region comprises the amino acid sequence as set forth in SEQ ID NO: 32; or
(3) the heavy chain variable region comprises the amino acid sequence as set forth in SEQ ID NO: 31, and the light chain variable region comprises the amino acid sequence as set forth in SEQ ID NO: 34; or
(4) the heavy chain variable region comprises the amino acid sequence as set forth in SEQ ID NO: 35, and the light chain variable region comprises the amino acid sequence as set forth in SEQ ID NO: 36.

6. The anti-SEZ6 antibody or the antigen-binding fragment thereof according to any one of claims 1 to 5, **characterized in that** the antigen-binding fragment is selected from Fab, Fab', F(ab')2, a single-chain antibody (scFv), a single-domain antibody (sdAb), a dimerized V region (diabody), a disulfide-stabilized V region (dsFv), and an antigen-binding fragment of a peptide comprising a CDR.

7. The anti-SEZ6 antibody or the antigen-binding fragment thereof according to any one of claims 1 to 6, **characterized by** comprising an antibody constant region,
wherein the heavy chain constant region is derived from human IgG1, IgG2, IgG3, IgG4, or a variant thereof; and/or the light chain constant region is derived from a human antibody κ or λ chain, or a variant thereof;
preferably, the amino acid sequence of the heavy chain constant region is derived from human IgG1 or a variant thereof; and/or the light chain constant region is derived from a human antibody κ chain or a variant thereof;
more preferably, the heavy chain constant region comprises the amino acid sequence as set forth in SEQ ID NO: 33, and/or the light chain constant region comprises the amino acid sequence as set forth in SEQ ID NO: 59.

8. The anti-SEZ6 antibody or the antigen-binding fragment thereof according to claim 7, **characterized by** comprising a heavy chain and a light chain, wherein
(1) the heavy chain comprises an amino acid sequence as set forth in SEQ ID NO: 55, or a sequence having at least 85%, 90%, 95%, or 99% homology thereto, and the light chain comprises an amino acid sequence as set forth in SEQ ID NO: 56, or a sequence having at least 85%, 90%, 95%, or 99% homology thereto; or
(2) the heavy chain comprises an amino acid sequence as set forth in SEQ ID NO: 57, or a sequence having at least 85%, 90%, 95%, or 99% homology thereto, and the light chain comprises an amino acid sequence as set forth in SEQ ID NO: 58, or a sequence having at least 85%, 90%, 95%, or 99% homology thereto; or
(3) the heavy chain comprises an amino acid sequence as set forth in SEQ ID NO: 57, or a sequence having at least 85%, 90%, 95%, or 99% homology thereto, and the light chain comprises an amino acid sequence as set forth in SEQ ID NO: 60, or a sequence having at least 85%, 90%, 95%, or 99% homology thereto; or
(4) the heavy chain comprises an amino acid sequence as set forth in SEQ ID NO: 61, or a sequence having at least 85%, 90%, 95%, or 99% homology thereto, and the light chain comprises an amino acid sequence as set forth in SEQ ID NO: 62, or a sequence having at least 85%, 90%, 95%, or 99% homology thereto;
preferably, (1) the heavy chain comprises the amino acid sequence as set forth in SEQ ID NO: 55, and the light chain comprises the amino acid sequence as set forth in SEQ ID NO: 56; or
(2) the heavy chain comprises the amino acid sequence as set forth in SEQ ID NO: 57, and the light chain comprises the amino acid sequence as set forth in SEQ ID NO: 58; or
(3) the heavy chain comprises the amino acid sequence as set forth in SEQ ID NO: 57, and the light chain comprises the amino acid sequence as set forth in SEQ ID NO: 60; or
(4) the heavy chain comprises the amino acid sequence as set forth in SEQ ID NO: 61, and the light chain comprises the amino acid sequence as set forth in SEQ ID NO: 62.

9. The anti-SEZ6 antibody or the antigen-binding fragment thereof according to claim 3, **characterized by** consisting of a heavy chain, wherein
(1) the heavy chain comprises an amino acid sequence as set forth in SEQ ID NO: 71, or a sequence having at least 85%, 90%, 95%, or 99% homology thereto; or
(2) the heavy chain comprises an amino acid sequence as set forth in SEQ ID NO: 72, or a sequence having at least 85%, 90%, 95%, or 99% homology thereto;
preferably, (1) the heavy chain comprises the amino acid sequence as set forth in SEQ ID NO: 71; or
(2) the heavy chain comprises the amino acid sequence as set forth in SEQ ID NO: 72.

10. An isolated nucleic acid encoding the anti-SEZ6 antibody or the antigen-binding fragment thereof according to any one of claims 1 to 9.

11. A vector comprising the isolated nucleic acid according to claim 10.

12. A host cell comprising the vector according to claim 11.

13. A method for preparing an anti-SEZ6 antibody or an antigen-binding fragment thereof, **characterized by** comprising culturing the host cell according to claim 12 under conditions suitable for expression of the anti-SEZ6 antibody or the antigen-binding fragment thereof.

14. An antibody-drug conjugate or a pharmaceutically acceptable salt or solvate thereof, or a tautomer, mesomer, racemate, enantiomer, or diastereomer thereof, or a mixture thereof, **characterized by** comprising the anti-SEZ6 antibody or the antigen-binding fragment thereof according to any one of claims 1 to 9 and a drug, wherein the drug is selected from: a cytotoxic agent, a radiolabel, a fluorophore, a chromophore, an imaging agent, an immunomodulator, an angiogenesis inhibitor, a cell proliferation inhibitor, a pro-apoptotic agent, a cytolytic enzyme, and any combination thereof;
preferably, the drug is selected from: a DNA damaging agent, a topoisomerase inhibitor, a microtubule inhibitor, a protein degrader, a STING agonist, and any combination thereof.

15. The antibody-drug conjugate or the pharmaceutically acceptable salt or solvate thereof, or the tautomer, mesomer, racemate, enantiomer, or diastereomer thereof, or the mixture thereof according to claim 14, **characterized by** having a structure as shown in general formula (I):
**Pc-(L-Y-Dr)ₙ** **(I)**
wherein Dr is the drug according to claim 14;
preferably, Dr is selected from: camptothecin, auristatin, maytansinoid, vinca alkaloid, pyrrolobenzodiazepine (PBD), calicheamicin, duocarmycin, daunorubicin, doxorubicin, calicheamicin, anthramycin, neomycin, amatoxin, hemiasterlin, eribulin, tubulysin, and analogs or derivatives thereof;
more preferably, Dr is selected from: exatecan, MMAE, MMAF, MMAD, SN-38, DM1, DM4, pyrrolobenzodiazepine (PBD) dimer, and analogs or derivatives thereof;
n is 1 to 10, and n is a decimal or an integer, preferably, n is 2 to 8, more preferably, n is 3 to 5 or 4 to 8, further preferably, n is 4 to 6, further preferably, n is 5 to 6, still further preferably, n is 2, 2.5, 3, 3.5, 4, 4.5, *5, 5.5,* 6, 6.5, 7, 7.5, or 8;
L and Y are linker units;
Pc is the anti-SEZ6 antibody or the antigen-binding fragment thereof according to any one of claims 1 to 9.

16. The antibody-drug conjugate or the pharmaceutically acceptable salt or solvate thereof, or the tautomer, mesomer, racemate, enantiomer, or diastereomer thereof, or the mixture thereof according to claim 14 or 15, **characterized by** having a structure as shown in general formula (Pc-L-Y-Dr), general formula (II), general formula (III), or general formula (IV): 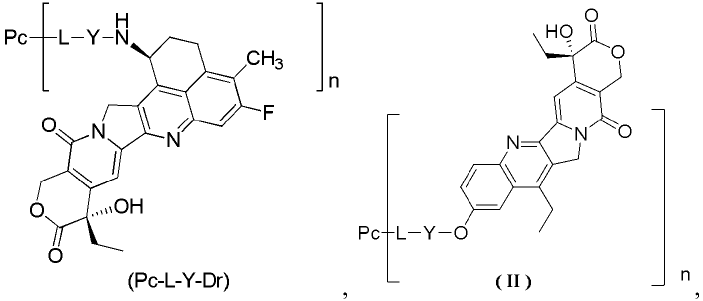 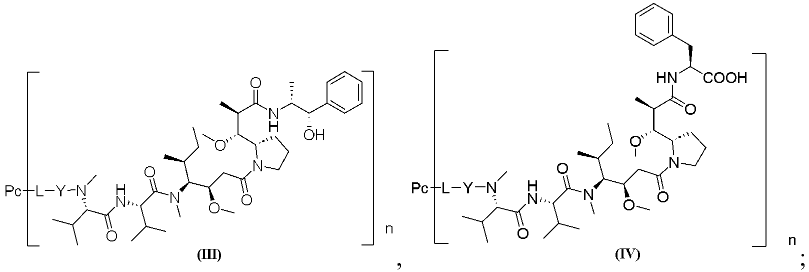 wherein
Y is selected from -O-(CR^{a}R^{b})ₘ-CR¹R²-C(O)-, -O-CR¹R²-(CR^{a}R^{b})ₘ-, -O-CR¹R²-, -NH-(CR^{a}R^{b})ₘ-CR¹R²-C(O)-, or -S-(CR^{a}R^{b})ₘ-CR¹R²-C(O)-;
R^{a} and R^{b} are identical or different, and are each independently selected from a hydrogen atom, a deuterium atom, halogen, alkyl, haloalkyl, deuterated alkyl, alkoxy, hydroxyl, amino, cyano, nitro, hydroxyalkyl, cycloalkyl, or heterocyclyl;
or R^{a} and R^{b} together with the carbon atom to which they are attached form cycloalkyl or heterocyclyl;
R¹ is selected from halogen, haloalkyl, deuterated alkyl, cycloalkyl, cycloalkylalkyl, alkoxyalkyl, heterocyclyl, aryl, or heteroaryl;
R² is selected from a hydrogen atom, halogen, haloalkyl, deuterated alkyl, cycloalkyl, cycloalkylalkyl, alkoxyalkyl, heterocyclyl, aryl, or heteroaryl;
or R¹ and R² together with the carbon atom to which they are attached form cycloalkyl or heterocyclyl;
or R^{a} and R² together with the carbon atom to which they are attached form cycloalkyl or heterocyclyl;
m is an integer from 0 to 4;
n is 1 to 10, and n is a decimal or an integer, preferably, n is 2 to 8, more preferably, n is 3 to 5 or 4 to 8, further preferably, n is 4 to 6, further preferably, n is 5 to 6, still further preferably, n is 2, 2.5, 3, 3.5, 4, 4.5, 5, 5.5, 6, 6.5, 7, 7.5, or 8;
L is a linker unit;
Pc is the anti-SEZ6 antibody or the antigen-binding fragment thereof according to any one of claims 1 to 9.

17. The antibody-drug conjugate or the pharmaceutically acceptable salt or solvate thereof, or the tautomer, mesomer, racemate, enantiomer, or diastereomer thereof, or the mixture thereof according to claim 16,
**characterized in that**
Y is -O-(CR^{a}R^{b})ₘ-CR¹R²-C(O)-;
R^{a} and R^{b} are identical or different, and are each independently selected from a hydrogen atom, a deuterium atom, halogen, or alkyl;
R¹ is haloalkyl or C₃₋₆ cycloalkyl;
R² is selected from a hydrogen atom, haloalkyl, or C₃₋₆ cycloalkyl;
or R¹ and R² together with the carbon atom to which they are attached form C₃₋₆ cycloalkyl;
m is 0 or 1.

18. The antibody-drug conjugate or the pharmaceutically acceptable salt or solvate thereof, or the tautomer, mesomer, racemate, enantiomer, or diastereomer thereof, or the mixture thereof according to any one of claims 16 or 17, **characterized in that** Y is selected from: 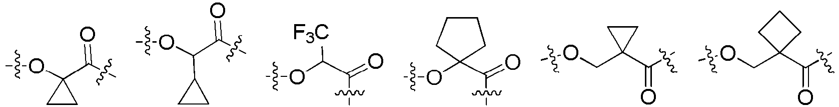 and

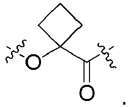

19. The antibody-drug conjugate or the pharmaceutically acceptable salt or solvate thereof, or the tautomer, mesomer, racemate, enantiomer, or diastereomer thereof, or the mixture thereof according to any one of claims 16 to 18, **characterized in that** the O-terminus of Y is linked to the linker unit L.

20. The antibody-drug conjugate or the pharmaceutically acceptable salt or solvate thereof, or the tautomer, mesomer, racemate, enantiomer, or diastereomer thereof, or the mixture thereof according to any one of claims 16 to 19, **characterized in that** the linker unit -L- is -L¹-L²-L³-L⁴-,
L¹ is 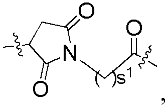 and s¹ is an integer from 2 to 8;
L² is a chemical bond;
L³ is a tetrapeptide residue;
L⁴ is -NR⁵(CR⁶R⁷)t-, R⁵, R⁶, or R⁷ is identical or different, and is each independently a hydrogen atom or alkyl, and t is 1 or 2.

21. The antibody-drug conjugate or the pharmaceutically acceptable salt or solvate thereof, or the tautomer, mesomer, racemate, enantiomer, or diastereomer thereof, or the mixture thereof according to any one of claims 16 to 19, **characterized in that** the linker unit -L- is -L¹-L²-L³-L⁴-,
L¹ is 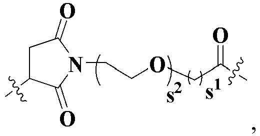 and s¹ and s² are independently integers selected from 0 to 8; preferably, s¹ is 2, and s² is an integer from 2 to 8; more preferably, s¹ is 2, and s² is 2, 3, 4, 5, or 6;
L² is a chemical bond;
L³ is a tetrapeptide residue;
L⁴ is -NR⁵(CR⁶R⁷)t-, R⁵, R⁶, or R⁷ is identical or different, and is each independently a hydrogen atom or alkyl, and t is 1 or 2.

22. The antibody-drug conjugate or the pharmaceutically acceptable salt or solvate thereof, or the tautomer, mesomer, racemate, enantiomer, or diastereomer thereof, or the mixture thereof according to any one of claims 16 to 21, **characterized in that** in the linker unit -L-, the L¹ terminus is linked to Pc, and the L⁴ terminus is linked to Y.

23. The antibody-drug conjugate or the pharmaceutically acceptable salt or solvate thereof, or the tautomer, mesomer, racemate, enantiomer, or diastereomer thereof, or the mixture thereof according to any one of claims 16 to 22, **characterized in that** the tetrapeptide residue of L³ is an amino acid residue formed by two or more amino acids selected from phenylalanine, glycine, valine, lysine, citrulline, serine, glutamic acid, or aspartic acid; preferably, the tetrapeptide residue is GGFG.

24. The antibody-drug conjugate or the pharmaceutically acceptable salt or solvate thereof, or the tautomer, mesomer, racemate, enantiomer, or diastereomer thereof, or the mixture thereof according to any one of claims 16 to 23, the antibody-drug conjugate is selected from: 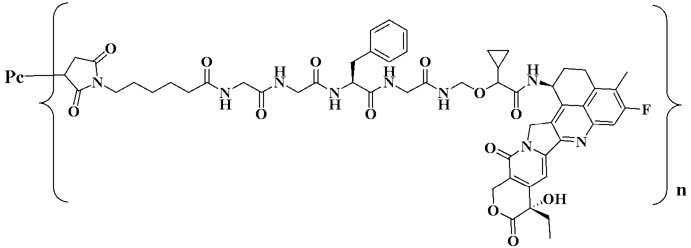 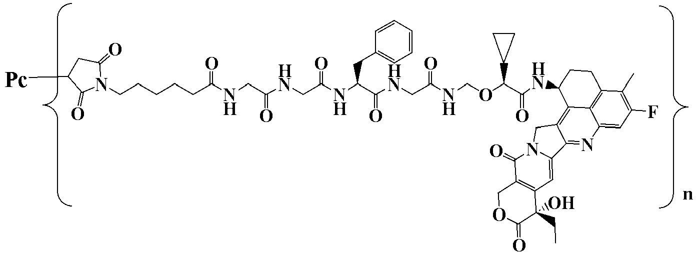 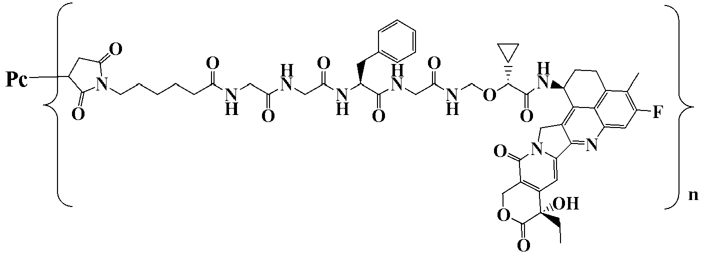 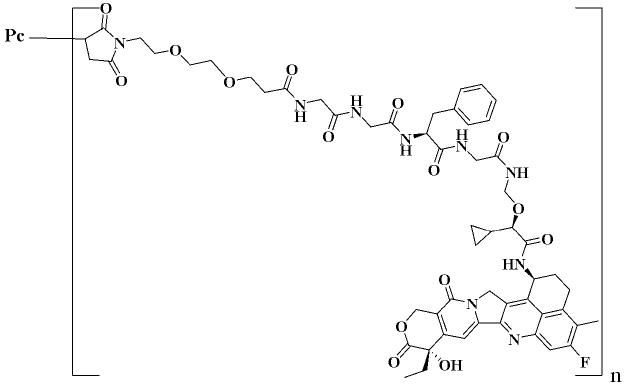 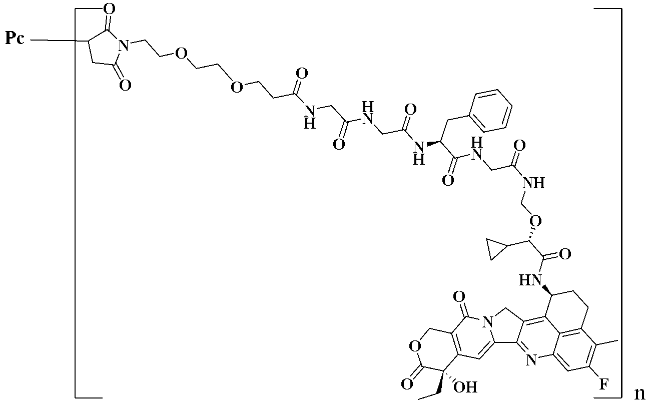 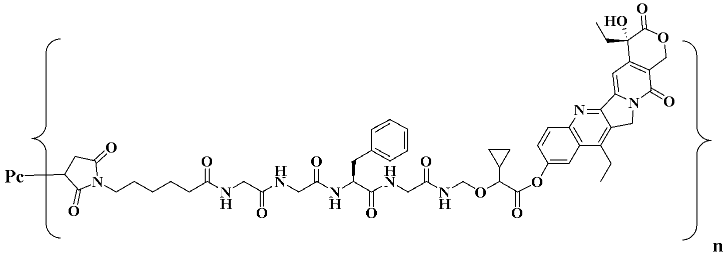 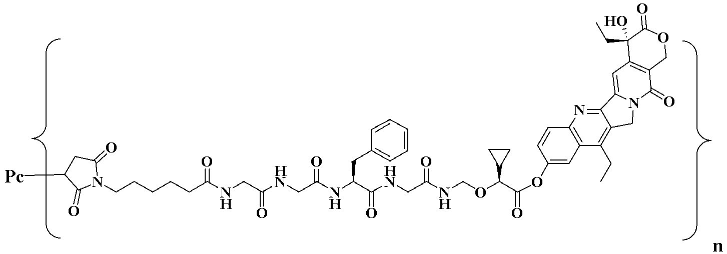 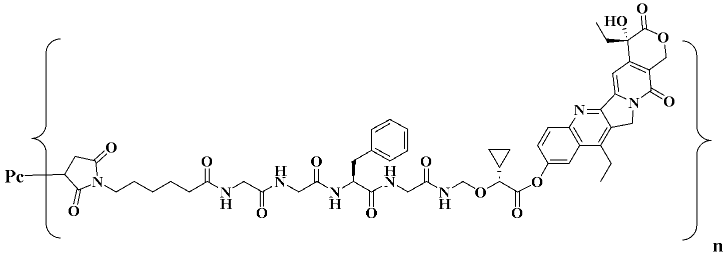 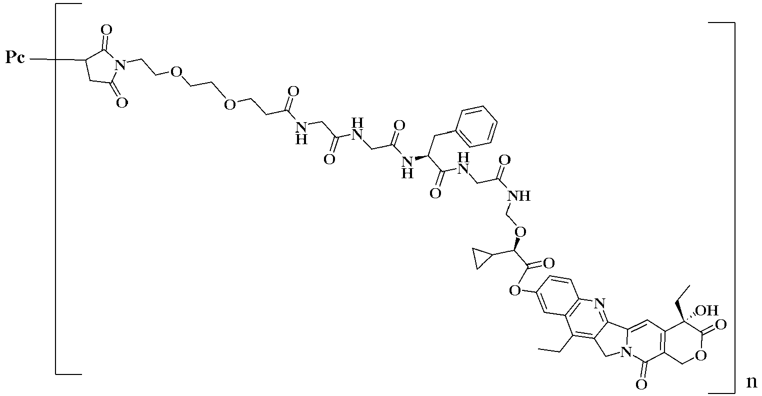 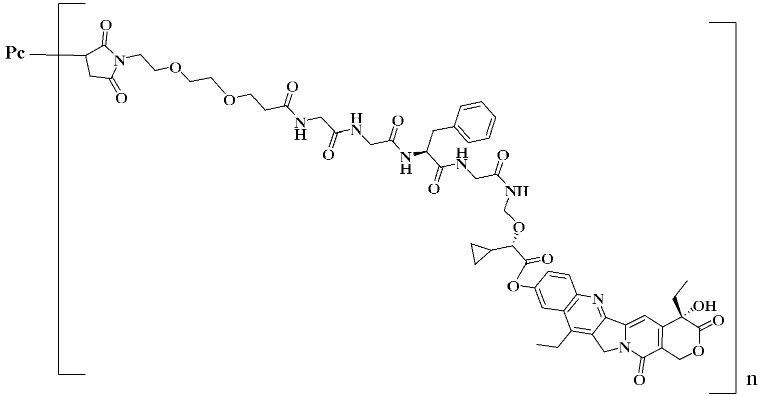 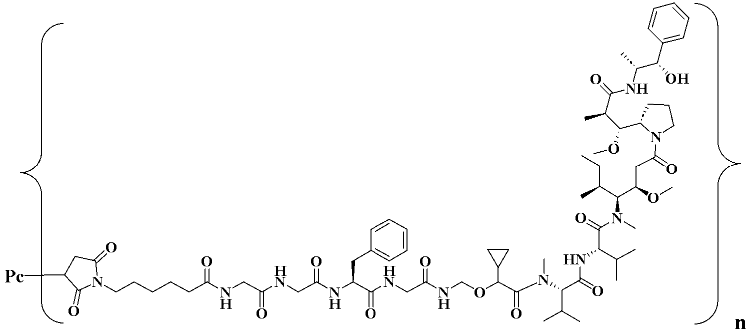 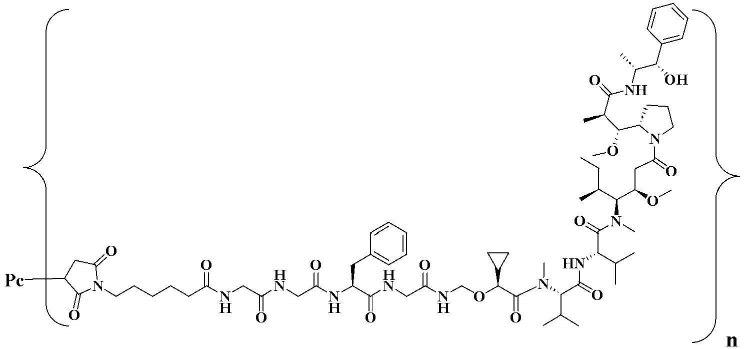 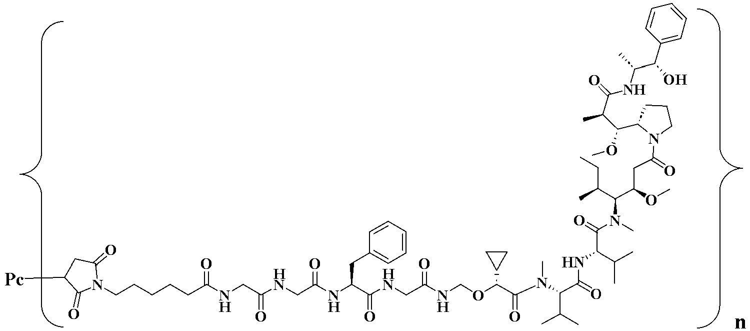 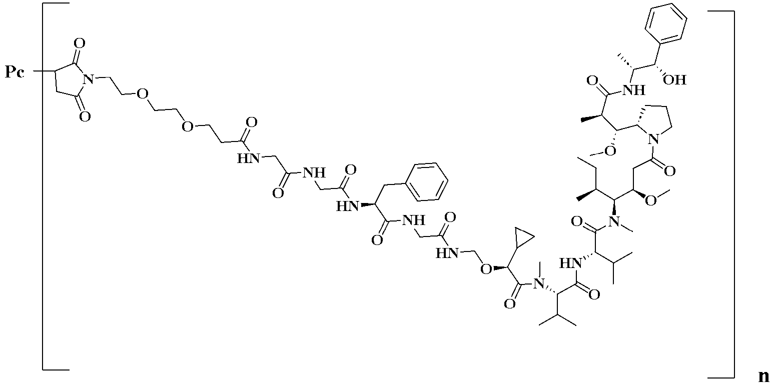 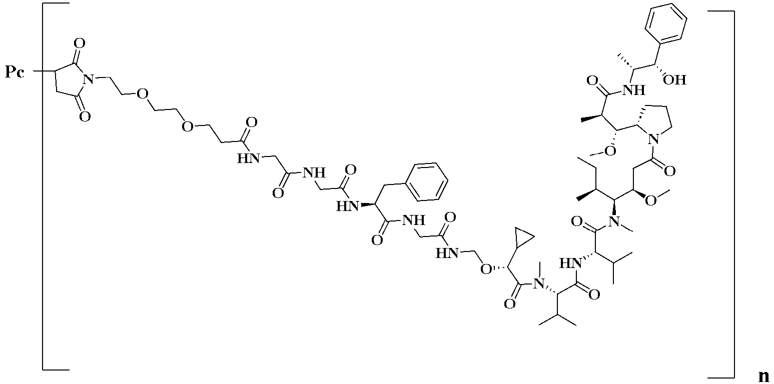 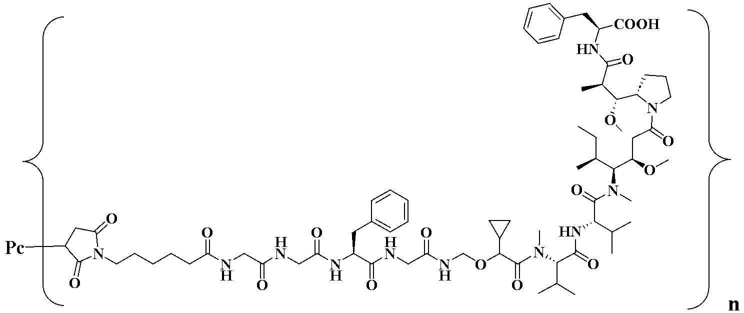 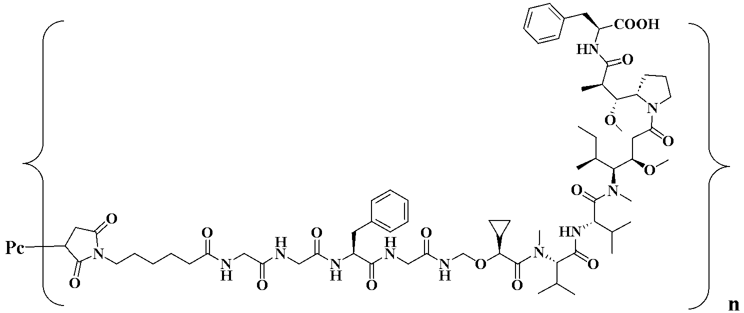 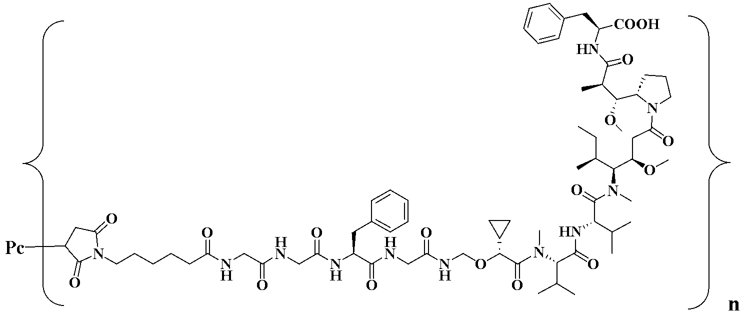 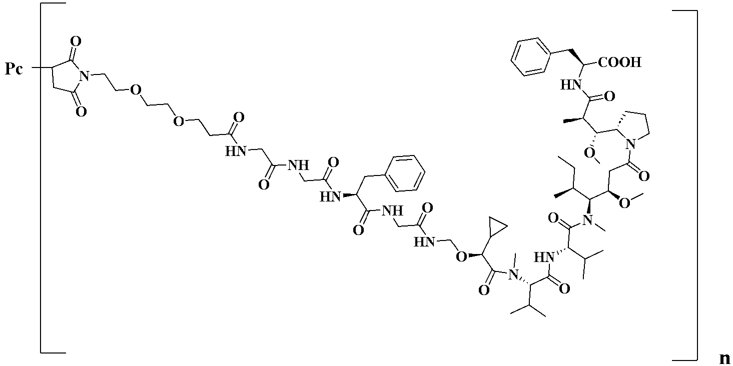 and 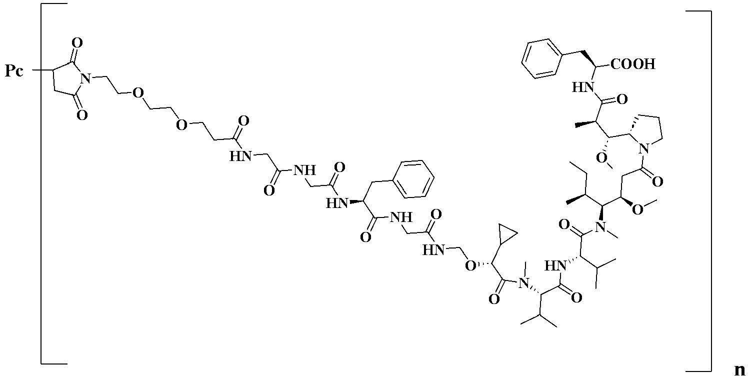 wherein Pc and n are as defined in claim 16.

25. The antibody-drug conjugate or the pharmaceutically acceptable salt or solvate thereof, or the tautomer, mesomer, racemate, enantiomer, or diastereomer thereof, or the mixture thereof according to any one of claims 16 to 24, the antibody-drug conjugate is selected from: 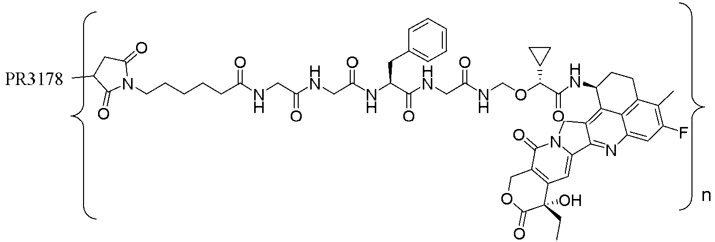 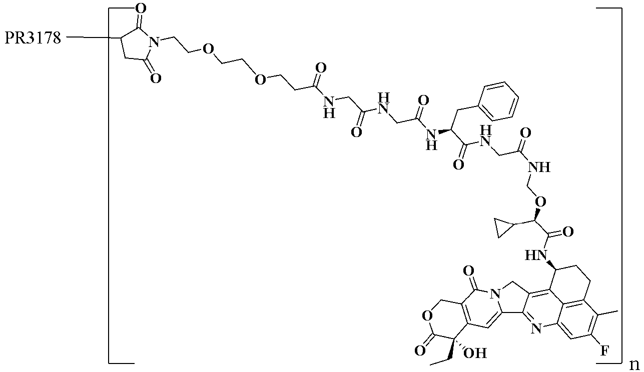 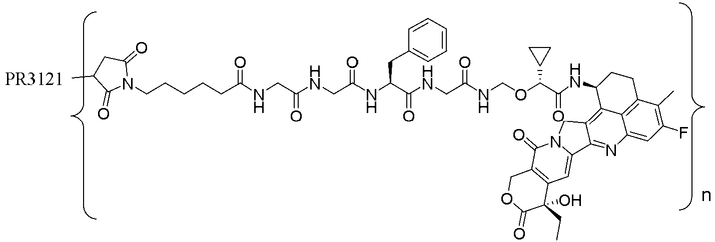 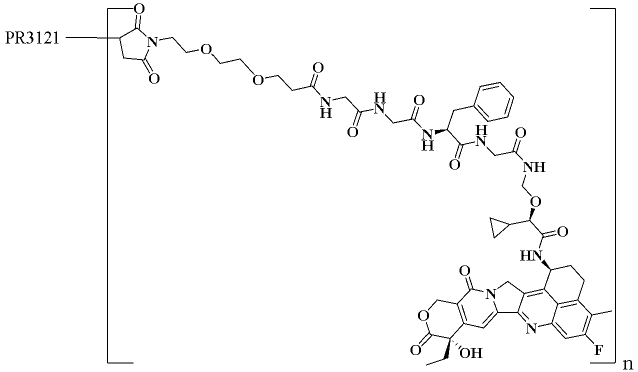 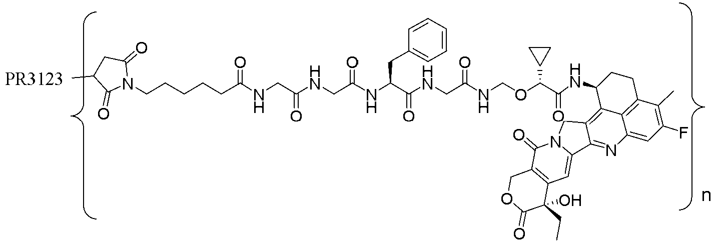 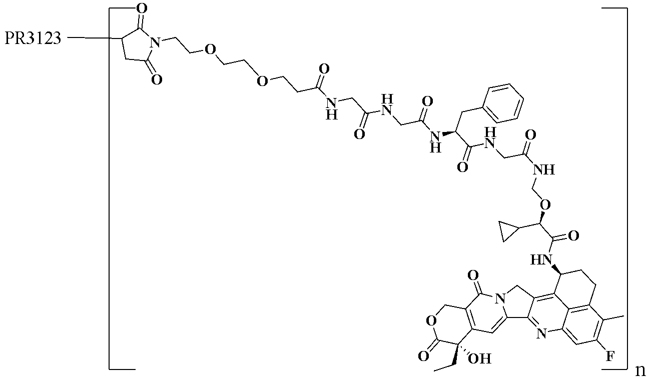 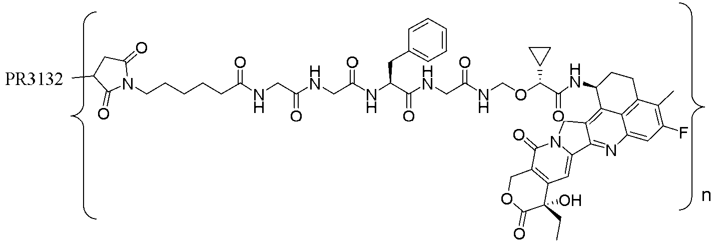 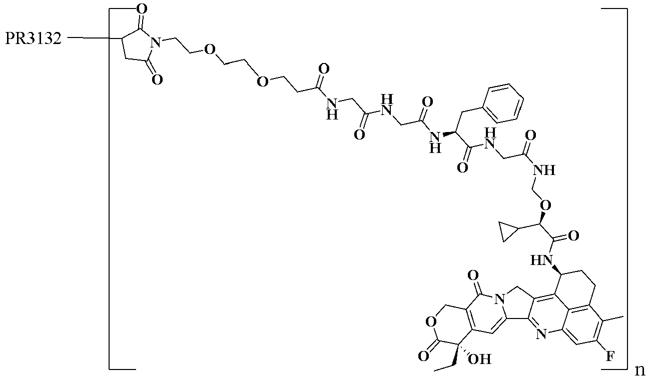 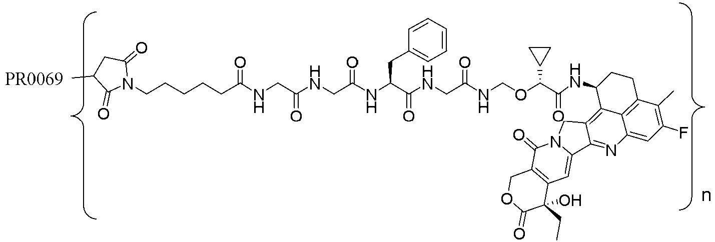 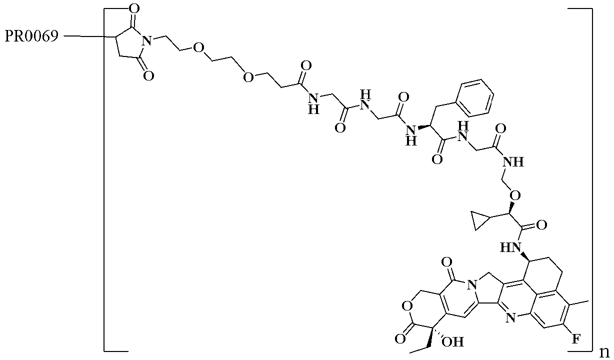 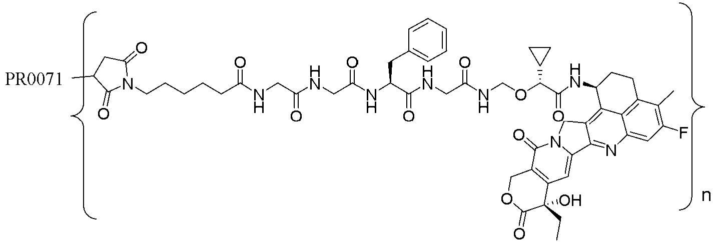 and 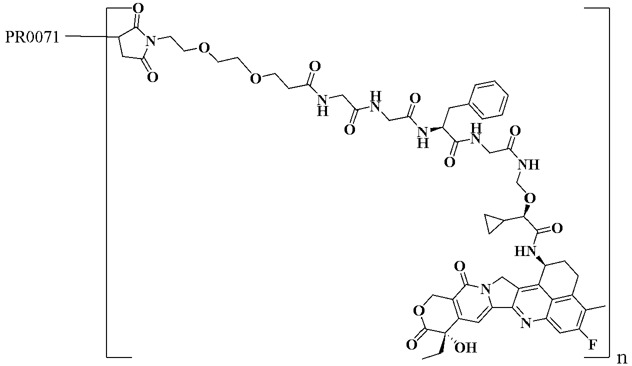 wherein n is as defined in claim 16.

26. A kit comprising the anti-SEZ6 antibody or the antigen-binding fragment thereof according to any one of claims 1 to 9, or the antibody-drug conjugate or the pharmaceutically acceptable salt or solvate thereof, or the tautomer, mesomer, racemate, enantiomer, or diastereomer thereof, or the mixture thereof according to any one of claims 14 to 25.

27. A pharmaceutical composition comprising the anti-SEZ6 antibody or the antigen-binding fragment thereof according to any one of claims 1 to 9, or the antibody-drug conjugate or the pharmaceutically acceptable salt or solvate thereof, or the tautomer, mesomer, racemate, enantiomer, or diastereomer thereof, or the mixture thereof according to any one of claims 14 to 25, and one or more pharmaceutically acceptable carriers.

28. Use of the anti-SEZ6 antibody or the antigen-binding fragment thereof according to any one of claims 1 to 9, or the antibody-drug conjugate or the pharmaceutically acceptable salt or solvate thereof, or the tautomer, mesomer, racemate, enantiomer, or diastereomer thereof, or the mixture thereof according to any one of claims 14 to 25, or the pharmaceutical composition according to claim 27 in the manufacture of a medicament for the treatment of a SEZ6-mediated disease or condition;
preferably, the SEZ6-mediated disease or condition is cancer with high expression of SEZ6.

29. Use of the anti-SEZ6 antibody or the antigen-binding fragment thereof according to any one of claims 1 to 9, or the antibody-drug conjugate or the pharmaceutically acceptable salt or solvate thereof, or the tautomer, mesomer, racemate, enantiomer, or diastereomer thereof, or the mixture thereof according to any one of claims 14 to 25, or the pharmaceutical composition according to claim 27 in the manufacture of a medicament for the treatment or prevention of a tumour or cancer;
preferably, the tumour or cancer is selected from: lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukaemia, lymphoma, and bone cancer.
